# EUROPEAN PATENT SPECIFICATION

(11) **EP 3 066 130 B1**
(45) Date of publication and mention of the grant of the patent: **15.05.2019**
(21) Application number: 14803300.4
(22) Date of filing: 07.11.2014
(51) Int. Cl.: C07K 16/32, C07K 16/28, C07K 16/46, A61K 39/395, A61P 35/00, A61P 35/02

(54) **ANTI-WT1/HLA BI-SPECIFIC ANTIBODY**
BISPEZIFISCHER ANTI-WT1/HLA-ANTIKÖRPER
ANTICORPS BISPÉCIFIQUE ANTI-WT1/HLA

(30) Priority: 07.11.2013 US 201361901310 P; 15.08.2014 US 201462037875 P
(43) Date of publication of application: 14.09.2016
(73) Proprietor: Memorial Sloan-Kettering Cancer Center, New York, NY 10065 (US); Eureka Therapeutics, Inc., Emeryville, CA 94608 (US)
(72) Inventor: SCHEINBERG, David, New York, NY 10025 (US); XIANG, Jingyi, Wainut Creek, CA 94597 (US); DAO, Tao, New York, NY 10128 (US); YAN, Su, State College, PA 16803 (US); LIU, Cheng, Oakland, CA 94618 (US)
(74) Representative: Carpmaels & Ransford LLP
(86) International application number: PCT/US2014/064621
(87) International publication number: WO 2015/070061

(56) References cited:
- WO-A1-94/28027
- WO-A1-2005/040220
- WO-A2-2012/135854
- DAO TAO ET AL: "Targeting the Intracellular WT1 Oncogene Product with a Therapeutic Human Antibody", SCIENCE TRANSLATION MEDICINE, AMERICAN ASSOCIATION FOR THE ADVANCEMENT OF SCIENCE, US, vol. 5, no. 176, 1 March 2013 (2013-03-01) , XP009178626, ISSN: 1946-6234, DOI: 10.1126/SCITRANSLMED.3005661
- DMITRY PANKOV ET AL: "A Bi-Specific T Cell Engaging Monoclonal Antibody (mAb) Derived From a TCR-Like Mab Specific For WT1/HLA-A0201 (ESK-BiTE) Shows a Potent Activity Against Human AML and Ph+ ALL In Mouse Models", BLOOD, vol. 122, no. 21, 15 November 2013 (2013-11-15), page 2521, XP055169088,

## Description

### Technical Field

The present invention relates generally to antibodies against cytosolic proteins. More particularly, the invention relates to antibodies against Wilm's tumor oncogene protein (WT1), specifically bi-specific antibodies that recognize a WT1 peptide in conjunction with a major histocompatibility antigen, as well as an antigen displayed on the surface of an immune effector cell.

### Background of the Invention

The development of therapeutic T cell receptor-like monoclonal antibodies (TCRm mAbs), that recognize peptide fragments of key intracellular proteins in the context of MHC class I molecules, is emerging as a new approach to target intracellular tumor-specific antigens (Ags). Most tumor-specific Ags are intracellular proteins, inaccessible to classical mAb therapy. These proteins are degraded, processed and displayed by MHC class I molecules as peptide/MHC complexes, that are recognized by the TCR of cytotoxic T lymphocytes (CTLs). Consequently, numerous approaches aiming at triggering T cell responses toward the low density of tumor-specific peptide/MHC complexes have been attempted, with limited success. Wilms' tumor protein (WT1) is a well-validated human tumor-specific Ag for T cell immunotherapy. WT1 is over-expressed in a wide range of human hematopoietic malignancies, leukemia stem cells, and diverse solid tumors. In normal adult tissue, the protein has limited and low expression, which makes it an ideal cancer-specific target (Gessler et al. Nature. 1990;346(6280):194-197; Menssen et al. Leukemia. 1995;9(6):1060-1067; Oji et al. Jpn J Cancer Res. 1999;90(2):194-204). Both WT1 epitope-specific T cells and antibodies to WT1 whole protein have been detected in patients with hematopoietic malignancies and solid tumors, indicating that WT1 is a highly immunogenic antigen (Gaiger et al. Clinical cancer research : an official journal of the American Association for Cancer Research. 2001;7(3 Suppl):761s-5s; Gillmore et al. Clinical cancer research : an official journal of the American Association for Cancer Research. 2006;12(1):34-42). Furthermore, a correlation between graft-versus-leukemia and detectable WT1-specific CTLs was observed after allogeneic stem cell transplantation, further demonstrating the therapeutic activity of these T cells.

A WT1-derived peptide fragment, RMFPNAPYL (RMF; SEQ ID NO: 1), is the best studied and most validated epitope for CD8 T cell recognition in the context of HLA-A0201 molecule. The RMF epitope has been widely used in peptide vaccines or as the target of adoptively transferred CD8 T cells expanded ex *vivo* from patients with acute myeloid leukemia (AML), myeloid dysplastic syndrome (MDS) and various solid tumors. These studies demonstrated the immunogenicity of the peptide epitope, which was associated with clinical responses in some patients (Krug et al. Cancer Immunol Immunother. 2010;59(1467-1479); Maslak et al. Blood. 2010;116(2):171-9; Keilholz et al. Blood. 2009;113(26):6541-8; Oka et al. ScientificWorldJournal. 2007;7:649-65; Oka et al. Proceedings of the National Academy of Sciences of the United States of America. 2004;101(38):13885-90; Letsch et al. and Keiholz, U., editor. Associate for Immunotherapy of Cancer: Cancer Immunotherapy - 2nd Annual Meeting; 2004; Mainz, Germany).

Despite the significant progress in T cell immunotherapy, objective clinical responses are still rarely seen. Inefficiency of T cell-based therapies has been attributed to low TCR affinities, limited *in vivo* potent cytotoxic responses against high tumor burdens, the lack of effector cell persistence, tolerance to self-tumor Ags, and the immunosuppression by T-regulatory (T-reg) cells and cytokines (Morris et al. Blood Reviews 2006; 20: 61-69; Konnig R. Curr Opin Immunol 2002: 14 (1) 75-83). To develop a different approach to targeting this important epitope of WT1, a fully human TCRm mAb specific for the RMF/HLA-A0201 complex was generated. The mAb showed potent therapeutic activity against WT1-expressing leukemia and solid tumors, both *in vitro* and *in vivo,* via antibody-dependent cellular cytotoxicity (ADCC) (Dao et al. Sci Transl Med. 2013;5(176):176ra133).

ADCC depends on the presence of natural killer (NK) cells, macrophages, neutrophils and other immune-effector cells, that can be extremely heterogeneous in patients with leukemias or cancers, especially after therapy. An alternative and effective approach to mediate mAb cytolytic therapy is to use T cells as the effector cells. T cells are among the most potent cytotoxic cells and account for the largest number of circulating cytotoxic cells. Recent approaches that add mAb specificity to T cells, such as adoptively transferring T cells engineered with antibody-based chimeric antigen receptors (known as CARs) and bi-specific mAbs with dual specificities for tumor Ags and CD3 T cells, have emerged as efficient strategies to re-direct polyclonal human T cells to well-defined tumor-associated Ags. Bi-specific antibody constructs are designed to cross link the surface Ag on cancer cells to the TCR/CD3 complex on T cells. The molecules can redirect both CD4 and CD8 T cells to kill tumor cells in a serial fashion that is independent of the cells' intrinsic Ag-specific TCR recognition, co-stimulatory molecules, and HLA expression on tumor cells. It also avoids vaccination, cytokine administration or ex *vivo* expansion and infusion of Ag-specific, patient-derived T cells (Frankel and Baeuerle. Current opinion in chemical biology. 2013;17(3):385-92; Brischwein et al. Journal of immunotherapy. 2007;30(8):798-807; Nagorsen et al. Pharmacology & therapeutics. 2012;136(3):334-42; Aigner et al. Leukemia. 2013;27(5):1107-15; Spiess et al. Nature biotechnology. 2013;31(8):753-8; Nagorsen and Baeuerle. Experimental cell research. 2011;317(9):1255-60). The bi-specific t-cell engager (BiTE®) antibody Blinatumomab (Amgen, Thousand Oaks, CA), specific for the pan B-cell Ag CD19 and the CD3e signaling chain of the TCR, is FDA approved for the treatment of non-Hodgkin's lymphoma and acute lymphocytic leukemia (ALL).

WO 2012/135854 identifies and characterizes antigen-binding proteins, such as antibodies, that are able to target cytosolic/intracellular proteins, for example, the WT1 oncoprotein. The disclosed antibodies target a peptide/MHC complex as it would typically appear on the surface of a cell following antigen processing of WT1 protein and presentation by the cell.

WO 2005/040220 teaches a cytotoxically active CD3 specific binding construct comprising a first domain specifically binding to human CD3 and an Ig-derived second binding domain.

### Summary of the Invention

The invention provides a recombinant antibody comprising:
(I) a first antigen-binding portion which is capable of specifically binding to WT1/HLA, comprising one of:
   (A) a single chain variable fragment (scFV) comprising an amino acid sequence selected from the group consisting of SEQ ID NOS: 54, 18, 36, 72, 90, 108 and 132; or
   (B) a heavy chain variable domain (VH) and a light chain variable domain (VL), wherein the VH and VL, respectively, comprise amino acid sequences selected from the group consisting of SEQ ID NOS: (i) 50 and 52; (ii) 14 and 16; (iii) 32 and 34; (iv) 68 and 70; (v) 86 and 88; (vi) 104 and 106; and (vii) 128 and 130; or
   (C)
      (i) a VH CDR1 comprising the amino acid sequence set forth in SEQ ID NO: 38; a VH CDR2 comprising the amino acid sequence set forth in SEQ ID NO: 39; a VH CDR3 comprising the amino acid sequence set forth in SEQ ID NO: 40; a VL CDR1 comprising the amino acid sequence set forth in SEQ ID NO: 44; a VL CDR2 comprising the amino acid sequence set forth in SEQ ID NO: 45; and a VL CDR3 comprising the amino acid sequence set forth in SEQ ID NO: 46;
      (ii) a VH CDR1 comprising the amino acid sequence set forth in SEQ ID NO: 2; a VH CDR2 comprising the amino acid sequence set forth in SEQ ID NO: 3; a VH CDR3 comprising the amino acid sequence set forth in SEQ ID NO: 4; a VL CDR1 comprising the amino acid sequence set forth in SEQ ID NO: 8; a VL CDR2 comprising the amino acid sequence set forth in SEQ ID NO: 9; and a VL CDR3 comprising the amino acid sequence set forth in SEQ ID NO: 10;
      (iii) a VH CDR1 comprising the amino acid sequence set forth in SEQ ID NO: 20; a VH CDR2 comprising the amino acid sequence set forth in SEQ ID NO: 21 ; a VH CDR3 comprising the amino acid sequence set forth in SEQ ID NO: 22; a VL CDR1 comprising the amino acid sequence set forth in SEQ ID NO: 26; a VL CDR2 comprising the amino acid sequence set forth in SEQ ID NO: 27; and a VL CDR3 comprising the amino acid sequence set forth in SEQ ID NO: 28;
      (iv) a VH CDR1 comprising the amino acid sequence set forth in SEQ ID NO: 56; a VH CDR2 comprising the amino acid sequence set forth in SEQ ID NO: 57; a VH CDR3 comprising the amino acid sequence set forth in SEQ ID NO: 58; a VL CDR1 comprising the amino acid sequence set forth in SEQ ID NO: 62; a VL CDR2 comprising the amino acid sequence set forth in SEQ ID NO: 63; and a VL CDR3 comprising the amino acid sequence set forth in SEQ ID NO: 64;
      (v) a VH CDR1 comprising the amino acid sequence set forth in SEQ ID NO: 74; a VH CDR2 comprising the amino acid sequence set forth in SEQ ID NO: 75; a VH CDR3 comprising the amino acid sequence set forth in SEQ ID NO: 76; a VL CDR1 comprising the amino acid sequence set forth in SEQ ID NO: 80; a VL CDR2 comprising the amino acid sequence set forth in SEQ ID NO: 81 ; and a VL CDR3 comprising the amino acid sequence set forth in SEQ ID NO: 82; or
      (vi) a VH CDR1 comprising the amino acid sequence set forth in SEQ ID NO: 92; a VH CDR2 comprising the amino acid sequence set forth in SEQ ID NO: 93; a VH CDR3 comprising the amino acid sequence set forth in SEQ ID NO: 94; a VL CDR1 comprising the amino acid sequence set forth in SEQ ID NO: 98; a VL CDR2 comprising the amino acid sequence set forth in SEQ ID NO: 99; and a VL CDR3 comprising the amino acid sequence set forth in SEQ ID NO: 100; and
(II) a second antigen-binding portion comprising:
   (A) a single chain variable fragment (scFV) comprising the amino acid sequence set forth in SEQ ID NO: 113; or
   (B) a heavy chain variable domain (VH) and a light chain variable domain (VL), wherein the VH and VL, respectively, comprise the amino acid sequences set forth in SEQ ID NOS: 112 and 111 or a heavy chain and a light chain, wherein the heavy chain and light chain, respectively, comprise the amino acid sequences set forth in SEQ ID NOS: 134 and 136.

The invention also provides a nucleic acid that encodes a recombinant antibody of the invention.

The invention also provides a pharmaceutical composition comprising the recombinant antibody or nucleic acid of the invention.

The invention also provides the antibody or pharmaceutical composition of the invention for use in a method for treating a WT1-positive disease in a subject wherein the WT1-positive disease is a chronic leukemia or acute leukemia or WT1+cancer.

The invention also provides a recombinant antibody for use in a method for stimulating a primary T cell response and a secondary T cell response in a subject comprising administering a composition comprising the recombinant antibody according to the invention, wherein the primary T cell response comprises stimulating cytotoxic T cells against the first tumor antigen, and wherein the secondary T cell response comprises stimulating effector T cells and/or memory T cells against the first tumor antigen and/or against a second tumor antigen.

Other features of the invention are set out in the appended claims.

### Summary of the Disclosure

The bi-specific antibodies that have been described previously are all directed to well-known, high density cell surface Ags that are not tumor-specific. The present disclosure relates to bi-specific antibodies derived from a TCRm mAb, designated ESK. The ESK-bi-specific antibody was able to selectively bind WT1/HLA-A0201 positive tumor cells and showed potent therapeutic activity in multiple human cancer models by redirecting human T cell cytotoxicity. Redirection of the T cell population to the cancer was demonstrated by dual target and effector cell tracking and imaging. The TCRm mAb bi-specific antibodies described herein are a potent therapeutic agent targeting a widely-expressed low density intracellular tumor-specific Ag, e.g., WT1. The bi-specific antibodies described herein are also capable of inducing a secondary T cell response specific for antigens other than WT1.

In one aspect, the disclosure relates to a recombinant antibody comprising a first antigen-binding portion that specifically binds to a WT1 peptide complexed with a major histocompatibility complex antigen such as HLA-A2 and can, therefore, bind to a WT1/HLA-A2⁺ cell even when WT1 is present at low density. The antibody further comprises a second antigen-binding portion that specifically binds to a surface antigen on an immune effector cell, for example, CD3, and can, therefore, also bind the immune effector cell.

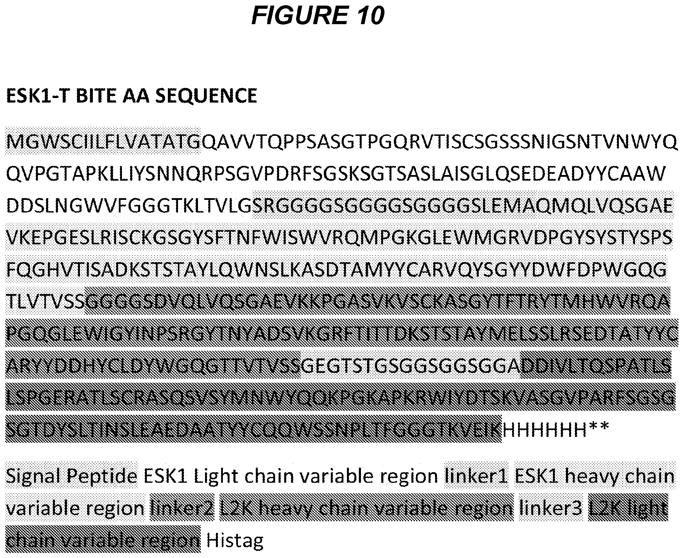

In one aspect, the disclosure relates to a recombinant antibody, wherein said recombinant antibody comprises: (i) a first antigen-binding portion comprising: (A) a heavy chain (HC) variable region comprising HC-CDR1, HC-CDR2 and HC-CDR3; and a light chain (LC) variable region comprising LC-CDR1, LC-CDR2 and LC-CDR3, comprising amino acid sequences as set forth in Tables 1-6; (B) a VH and a VL comprising first and second amino acid sequences as set forth in Tables 1-6; or (C) a scFv comprising an amino acid sequence as set forth in Tables 1-6; and (ii) a second antigen-binding portion comprising an amino acid sequence as set forth in Table 7. In one embodiment, the recombinant antibody comprises the amino acid sequence shown in Figure 10 (SEQ ID NO: 110).

In another aspect, the disclosure relates to a nucleic acid that encodes a recombinant bi-specific antibody disclosed herein.

In a related aspect, the disclosure relates to pharmaceutical compositions comprising the recombinant bi-specific antibody and a pharmaceutically acceptable excipient. In another aspect, the disclosure relates to pharmaceutical compositions comprising a nucleic acid encoding the recombinant bi-specific antibody and a pharmaceutically acceptable excipient.

In yet another aspect, the disclosure relates to a method for killing a WT1⁺ cell, said method comprising contacting the WT1⁺ cell with an antibody having specificity for the amino acid sequence of SEQ ID NO: 1 and a cytotoxic T cell. In some embodiments, the cytotoxic T cell is an autologous cell.

In yet another related aspect, the disclosure relates to a method of treatment of a subject having a WT1-positive disease, the method comprising administering to the subject a therapeutically effective amount of a recombinant bi-specific antibody described herein. In one embodiment, the method further comprises administering to the subject CD3⁺ cytotoxic T cells that are autologous. In one embodiment, the WT1-positive disease is a chronic leukemia or acute leukemia or WT1⁺ cancer, for example, chronic myelocytic leukemia, multiple myeloma (MM), acute lymphoblastic leukemia (ALL), acute myeloid/myelogenous leukemia (AML), myelodysplastic syndrome (MDS), mesothelioma, ovarian cancer, gastrointestinal cancers, breast cancer, prostate cancer or glioblastoma.

In one aspect, the disclosure relates to a recombinant bi-specific antibody comprising a first antigen-binding portion comprising one of: (A) a single chain variable fragment (scFV) comprising an amino acid sequence selected from the group consisting of SEQ ID NOS: 18, 36, 54, 72, 90, 108, and 132; or (B) a heavy chain variable domain (VH) and a light chain variable domain (VL), wherein the VH and VL, respectively, comprise amino acid sequences selected from the group consisting of SEQ ID NOS: (i) 14 and 16; (ii) 32 and 34; (iii) 50 and 52; (iv) 68 and 70; (v) 86 and 88; (vi) 104 and 106; (vii) 128 and 130; or (C) (i) the following three VH complementarity determining regions (CDRs): (a) a VH CDR1 comprising the amino acid sequence selected from the group consisting of SEQ ID NOS: 2, 20, 38, 56, 74, 92 and 116; and (b) a VH CDR2 comprising an amino acid sequence selected from the group consisting of SEQ ID NOS: 3, 21, 39, 57, 75, 93 and 117; and (c) a VH CDR3 comprising an amino acid sequence selected from the group consisting of SEQ ID NOS: 4, 22, 40, 58, 76, 94 and 118; and (ii) the following three VL CDRs: (a) a VL CDR1 comprising an amino acid sequence selected from the group consisting of SEQ ID NOS: 8, 26, 44, 62, 80, 98, and 122; and (b) a VL CDR2 comprising an amino acid sequence selected from the group consisting of SEQ ID NOS: 9, 27, 45, 63, 81, 99, and 123; and (c) a VL CDR3 comprising an amino acid sequence selected from the group consisting of SEQ ID NOS: 10, 28, 46, 64, 82, 100, and 124. The recombinant bi-specific antibody further comprises a second antigen-binding portion comprising (A) a scFV comprising the amino acid sequence set forth in SEQ ID NO: 113; or (B) a heavy chain variable domain (VH) and a light chain variable domain (VL), wherein the VH and VL, respectively, comprise the amino acid sequences set forth in SEQ ID NOS: 112 and 111 or SEQ ID NOS: 134 and 136.

In another aspect, the disclosure relates to a method of stimulating a primary T cell response and a secondary T cell response in a subject comprising administering a composition comprising a recombinant antibody, said recombinant antibody comprising a first antigen-binding portion and second antigen-binding portion, wherein said first antigen-binding portion specifically binds to a first tumor antigen and said second antigen-binding portion specifically binds to an immune effector cell surface antigen, wherein the primary T cell response comprises stimulating cytotoxic T cells against the first tumor antigen, and wherein the secondary T cell response comprises stimulating effector T cells and/or memory T cells against the first tumor antigen and/or against a second tumor antigen. In one aspect, the secondary T cell response does not require antigen presenting cells or co-stimulatory molecules. In another aspect, the secondary T cell response comprises stimulating effector T cells and/or memory T cells against the second tumor antigen. In one aspect, the second tumor antigen is a non-WT1-RMF tumor antigen.

In yet another aspect, the disclosure relates to a method of producing effector T cells and/or memory T cells against a tumor antigen comprising activating a T cell with a recombinant bi-specific antibody disclosed herein. The effector T cells and/or memory T cells may be produced *in vivo* or ex *vivo.* In one aspect, the effector T cells and/or memory T cells are produced against a non-WT1-RMF tumor antigen, for example, HER2-neu.

The present disclosure is intended to be related as a unified document, and it should be understood that all combinations of features described herein are contemplated, even if the combination of features are not found together in the same sentence, paragraph, or section of this disclosure. With respect to aspects of the disclosure described or claimed with "a" or "an," it should be understood that these terms mean "one or more" unless context unambiguously requires a more restricted meaning. With respect to elements described as one or more within a set, it should be understood that all combinations within the set are contemplated. If aspects of the disclosure are described as "comprising" a feature, embodiments also are contemplated "consisting of" or "consisting essentially of" the feature.

### Brief Description of the Drawings

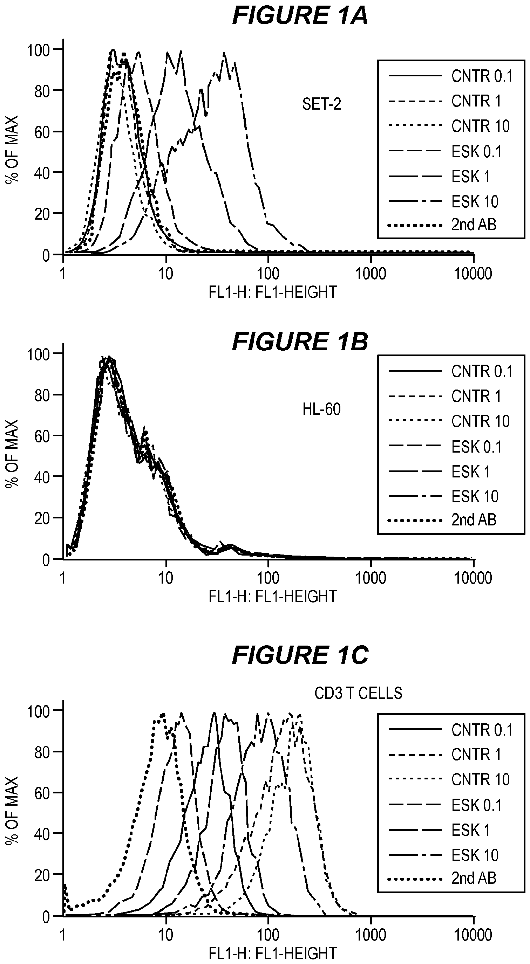

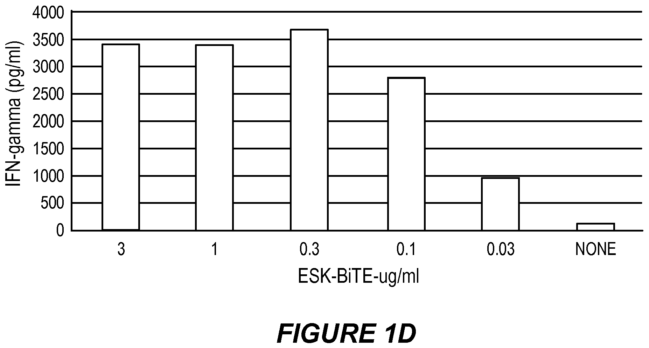

**Figure 1** shows selective binding of one embodiment of an ESK bi-specific antibody to tumor cells and human T cells measured by flow cytometry. SET-2 (**1A**), HL-60 (**1B**) or purified human CD3 T cells (**1C**) were stained with ESK-bi-specific antibody or control at concentrations of 10 µg/ml, 1 µg/ml or 0.1 µg/ml, followed by secondary mAb specific for His tag conjugated to FITC. ESK-bi-specific antibody showed selective binding to SET-2 cells at the concentrations of 10 µg/ml, 1 µg/ml and 0.1 µg/ml. The control secondary mAb alone and control bi-specific antibody at indicated concentrations did not bind to the cells. None of ESK nor control bi-specific antibodies bound to HL-60 at all the concentrations tested. For resting human T cells, ESK-bi-specific antibody showed a weaker binding than the control bi-specific antibody. Median fluorescence intensity (MFI) for ESK-bi-specific antibody: 91.4, 41 and 13.2 at concentration of 10 µg/ml, 1 µg/ml and 0.1 µg/ml, respectively. For control bi-specific antibody: 188, 153 and 26.2 at concentrations of 10 µg/ml, 1 µg/ml and 0.1 µg/ml, respectively. (**1D**) ESK-bi-specific antibody induce IFN-γ secretion in the presence of WT1+/HLA-A0201+ tumor cells. Human T cells and SET-2 cells at a 15:1 ratio were incubated with or without ESK-bi-specific antibody or control bi-specific antibody at a concentration of 3 µg/ml, 1 µg/ml, 0.3 µg/ml, 0.1 µg/ml, or 0.03 µg/ml for overnight. The supernatants were collected and IFN-γ release was measured by ELISA kit. The cultures of T cells alone or T cells with SET-2 cells, plus control bi-specific antibody did not show any detectable IFN-γ. Their values were therefore subtracted from the data shown. The data show the average of duplicate culture and represent one of two similar experiments.

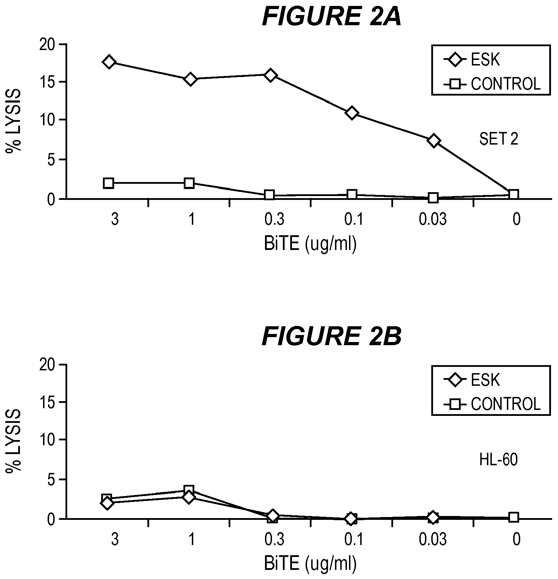

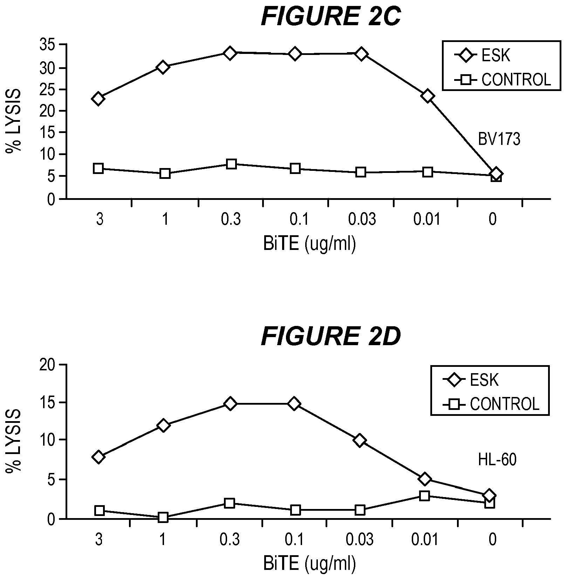

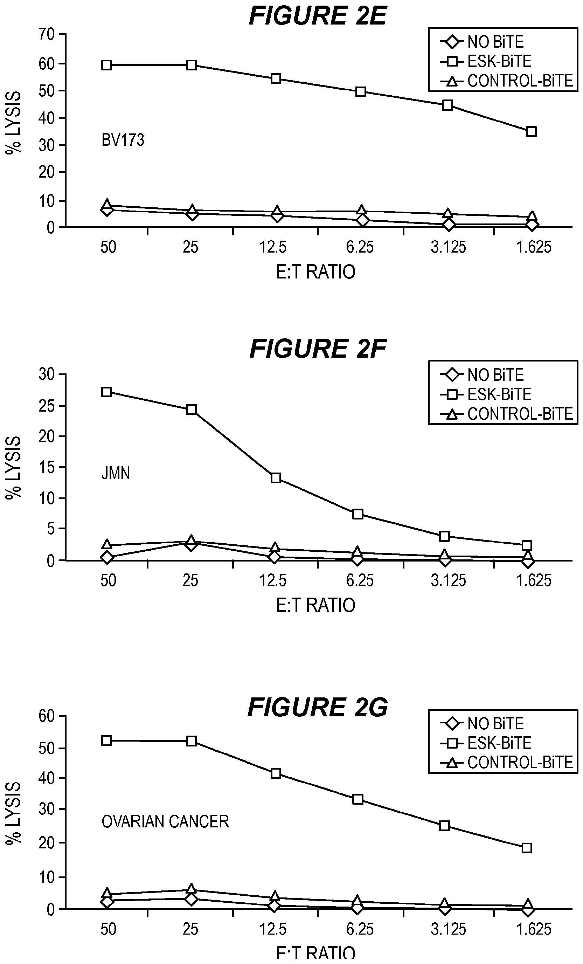

**Figure 2** shows that ESK-bi-specific antibody directs T cell cytotoxicity against WT1+/HLA-A0201+ tumor cells. Purified T cells were incubated with SET-2 (**2A**) or HL-60 cells (**2B**) at E: T ratio of 40:1, in the presence or absence of ESK or control bi-specific antibody at the indicated concentrations. T cell cytotoxicity was measured by ⁵¹Cr-release after 5-hour incubation. Similarly, ESK-directed cytotoxicity of PBMCs against BV173 (**2C**) and CML blasts (**2D**) from patient in a 6-hr -⁵¹Cr release assay at an E:T ratio of 100:1. ESK-bi-specific antibody-mediated cytotoxicity by EBV-primed human T cells was measured by a 5 hour-⁵¹Cr-release assay against BV173 (**2E**), JMN (**2**F) and primary ovarian cancer cells (**2G**) at the indicated E: T ratio. Bi-specific antibodies were used at 0.1 µg/ml. All the data points are average of triplicate cultures and represent one of two to three similar experiments.

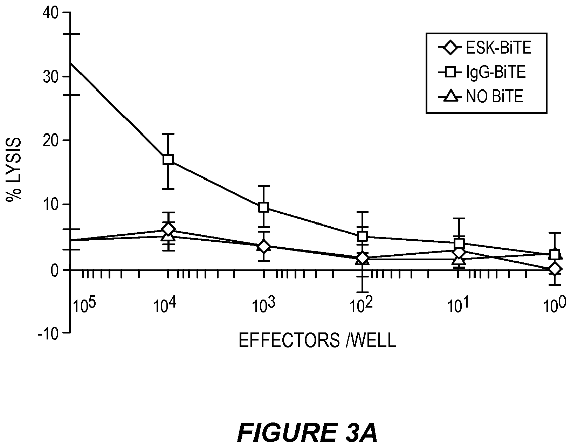

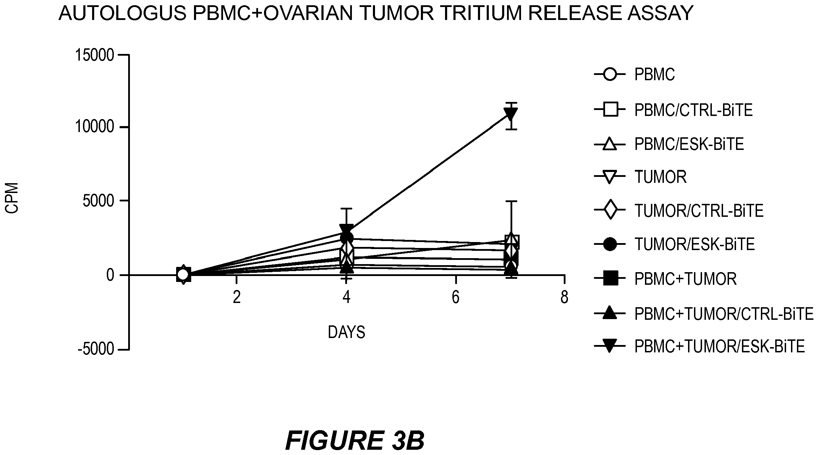

**Figure 3** shows that ESK-bi-specific antibody induces T cell activation in the presence of autologous ovarian cancer cells. PBMCs from a patient at indicated numbers/well were incubated with autologous irradiated ovarian cancer cells, in the presence of 0.1 µg/ml of ESK or control bi-specific antibody for the first 3 days. (**3A**) Cells were cultured for a total of 7 days and on day 8, ⁵¹Cr-labeled autologous tumor cells were added to the effector cells at a thousand cells/well. The ⁵¹Cr-release was measured 6 hrs later. (**3B**) PBMCs, autologous ovarian cancer cells, or PBMCs plus ovarian cancer cells were incubated with or without ESK or control bi-specific antibody at 0.1 µg/ml for 7 days. ³H-Thymidine was added overnight, and the cells were harvested the following day. The data represent triplicate cultures.

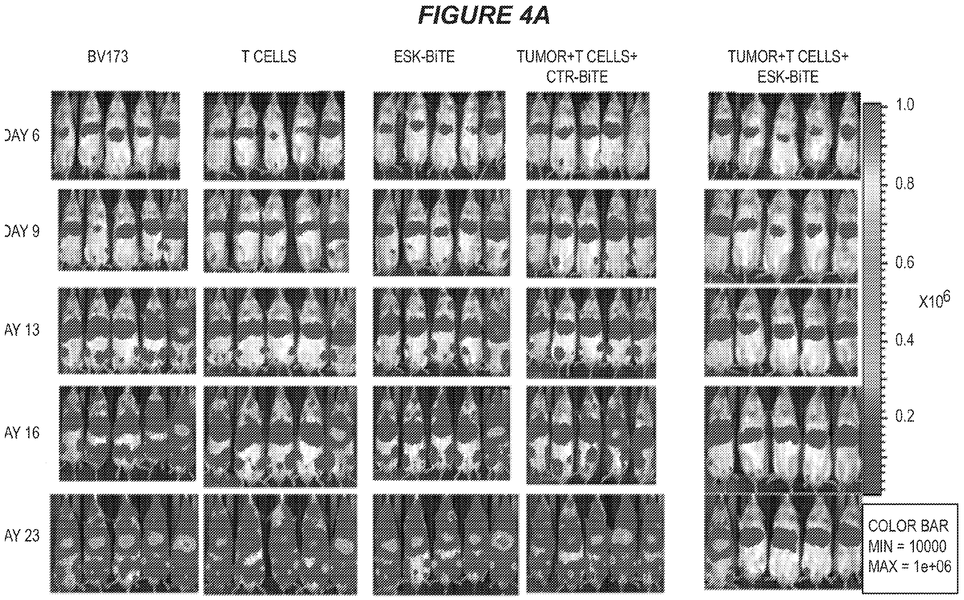

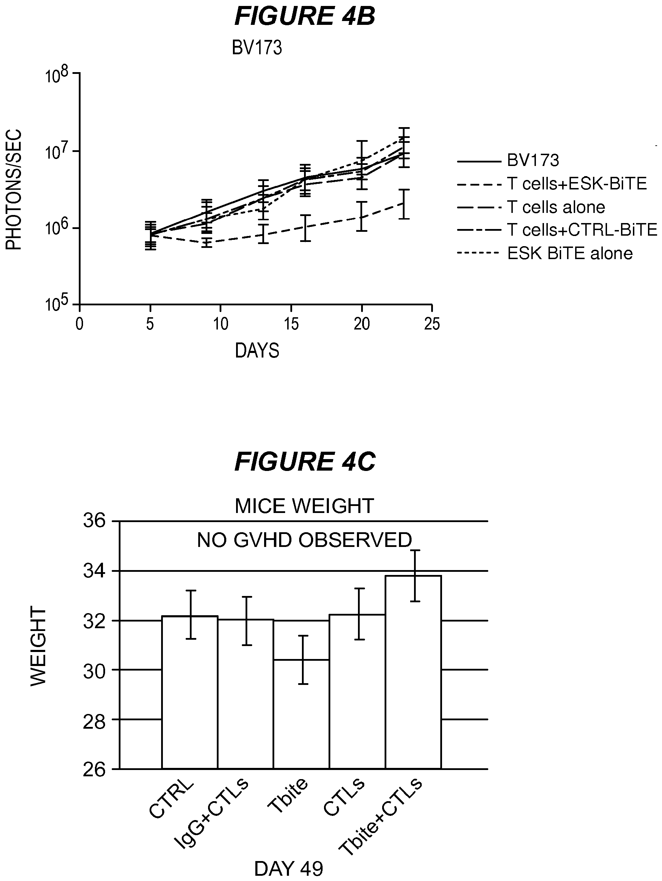

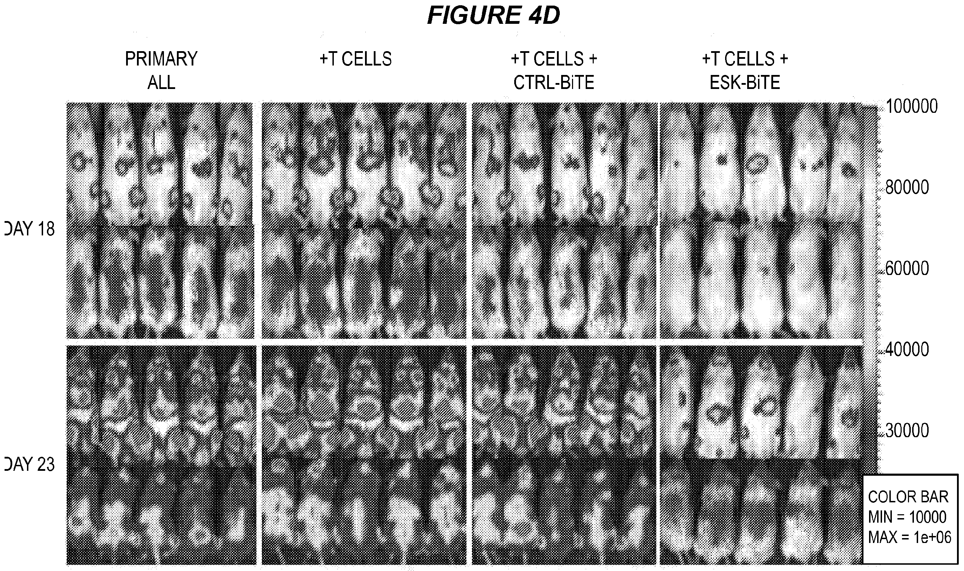

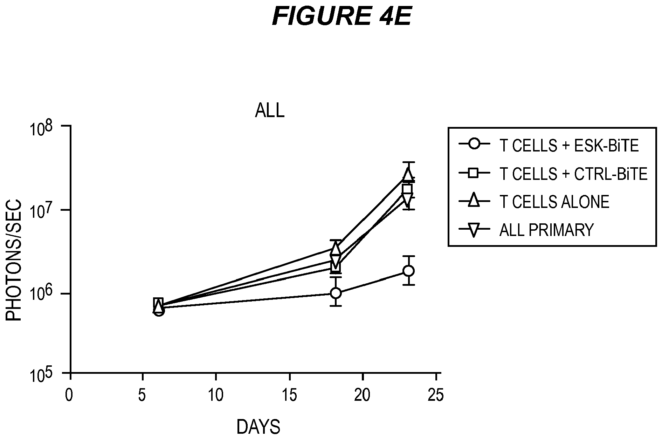

**Figure 4** shows that ESK-bi-specific antibody effectively treats BV173 and ALL in NSG mice. Two million BV173 cells were injected i.v. into mice on day 0, the tumor engraftment was confirmed on day 5, and mice were randomized into treatment groups. Ten million EBV-specific T cells were given intravenously on day 6, followed by 20 µg of ESK1 or control bi-specific antibody i.v. injection 4 to 5 hours later. T cells were given once a week and bi-specific antibodies were given twice a week for a total of three weeks. (**4A**) Tumor burden was shown by bioluminescence imaging (BLI) from the front of mice. BLI on day 6 showed tumor engraftment before treatment. The mice that received T cells and ESK-bi-specific antibody showed significant reduction of tumor burden, especially in bone marrow. (**4B**) Tumor burden was calculated by summing the luminescent signal of each mouse in back and front two positions, and average signal for each group (n=5) was plotted. (**4C**) GVHD was assessed by weighing mice every week, and no GVHD was observed up to 49 days. (**4D**) ESK-bi-specific antibody inhibited primary ALL cell growth in NSG mice. Five million primary tumor ALL cells were injected i.v. into NSG mice. On day 6, after confirming tumor engraftment by firefly BLI, mice were randomly divided into different treatment groups. Thirty million EBV-specific T cells were iv injected into mice followed by i.v. injection of 20 µg ESK-bi-specific antibody or its control bi-specific antibody. Bi-specific antibody injection was given daily, and T cells were given twice a week for a total of two weeks. Tumor inhibition on day 18 and day 23 after tumor inoculation is shown in prone and supine views for each time point. (**4E**) Data from the ALL mouse model: average photon/second from five mice showed a nearly hundred-fold reduction of tumor burden in the mice treated with ESK-bi-specific antibody after more than 3 weeks.

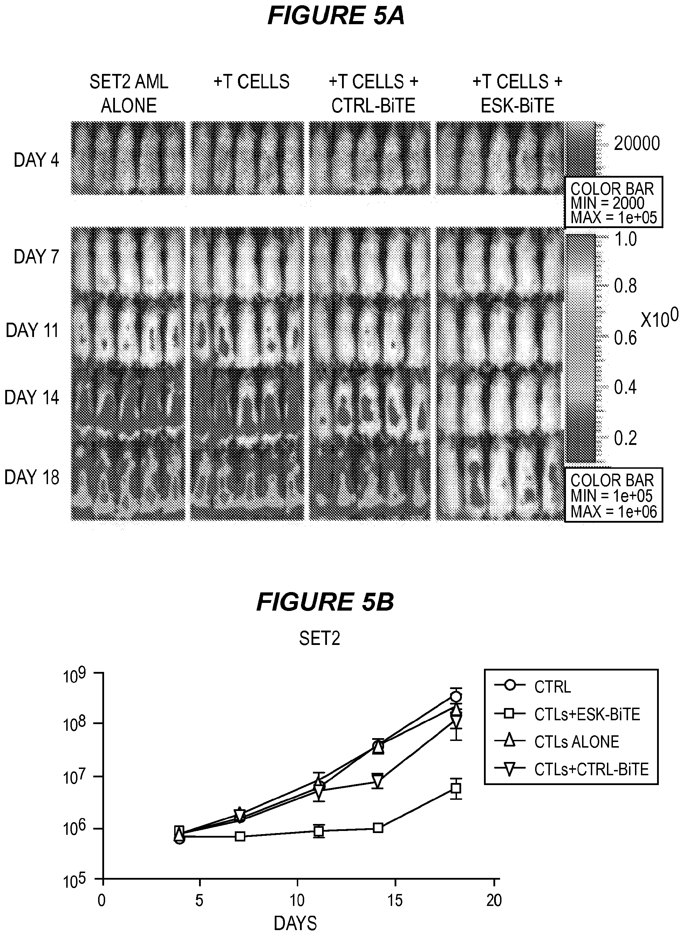

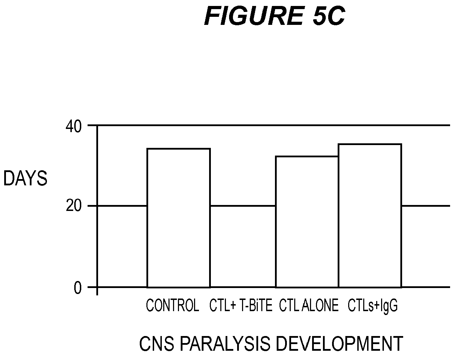

**Figure 5** shows that ESK-bi-specific antibody effectively treats SET-2 AML in NSG mice. In this model, ten million EBV-specific T cells were given i.v. twice a week and 20 µg bi-specific antibodies were injected i.v. daily for a total of 6 days, after confirming tumor engraftment on day 4. (**5A**) Tumor burden was shown from the back of mice to show spinal leukemia infiltration, in order to show the leukemia burden in all groups. BLI scale was increased about 10-fold on the images from day 7 onward. (**5B**) Tumor burden was calculated by summing the luminescent signal of each mouse in back and front two positions, and average signal for each group (n=5) was plotted. (**5C**) Leukemia infiltration was also assessed by limb paralysis caused by central nerve system damage. Mice that received T cells and ESK-bi-specific antibody showed no CNS paralysis. Each bar shows the average of five mice/group.

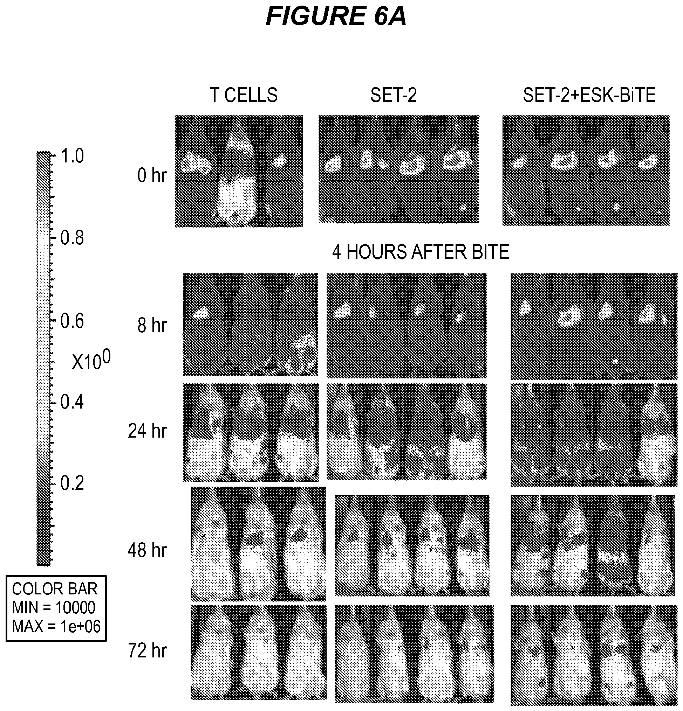

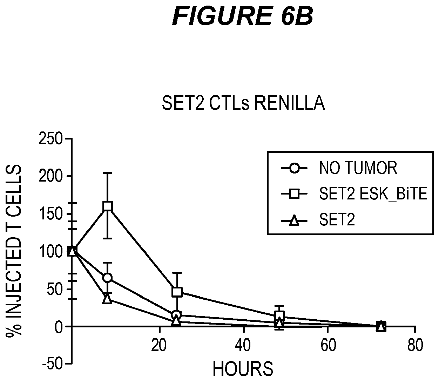

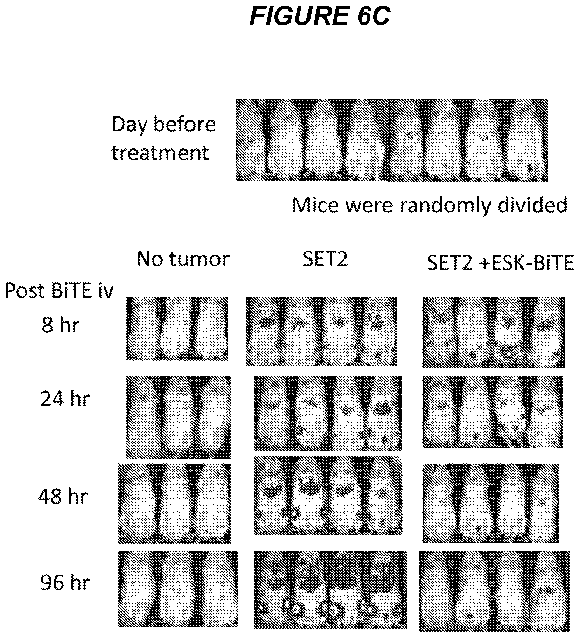

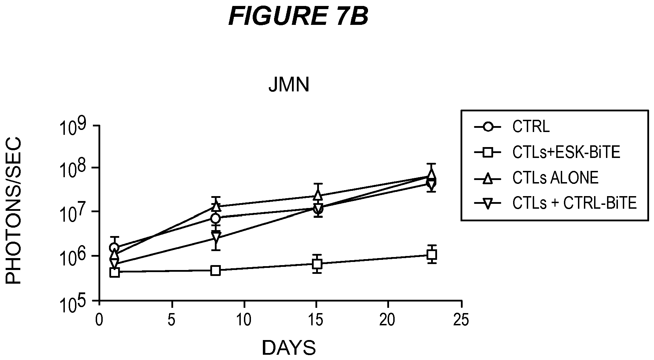

**Figure 6** shows that ESK-bi-specific antibody mediates T cell retention in bone marrow of tumor-bearing mice. (**6A**) Twenty million Renilla-transduced EBV-specific T cells were injected i.v. into mice that had been engrafted with SET-2 cells. Four hours later, 20 µg ESK or control bi-specific antibody was given by i.v. injection. T cell distribution was monitored by Renilla bioluminescence imaging, immediately after T cell injection (0 hr), 4 hrs after bi-specific antibody injection and then every day for a total of three days. (**6B**) T cell signal was calculated by summing the luminescent signal of each mouse and average signal for each group (n=3) was plotted. (**6C**) SET-2 leukemia burden was simultaneously monitored by firefly bioluminescence imaging at the indicated time points. Mice that received T cells alone showed no firefly signal. Mice that received T cells and ESK-bi-specific antibody showed a dramatic reduction of leukemia burden, compared to the mice that received SET-2 cells. Tumor inhibition was correlated with T cell retention as shown in Figure 7B.

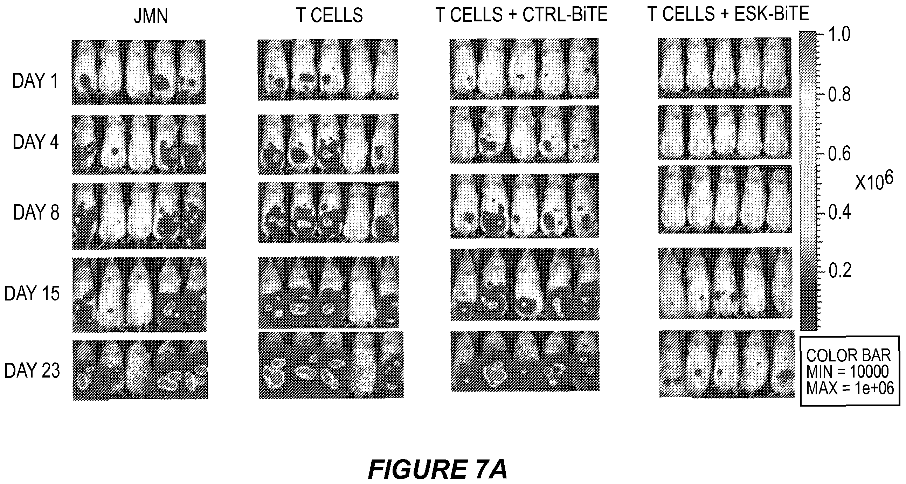

**Figure 7** shows that ESK-bi-specific antibody eliminates peritoneal mesothelioma cells JMN in NSG mice. Three thousand JMN cells were mixed with six thousand EBV-specific T cells and were i.p. injected into mice. One hour later, ESK or control bi-specific antibody was i.v. injected and was repeated for 5 consecutive days. Tumor development was monitored by firefly bioluminescence imaging at the indicated time points. (**7A**) Day 1 (one day after treatment) showed no visible tumor in the mice treated with ESK-bi-specific antibody, suggesting the elimination of tumor cells. (**7B**) Average photon/second from five mice showed nearly hundred fold reduction of tumor burden in the mice treated with ESK-bi-specific antibody after more than 3 weeks.

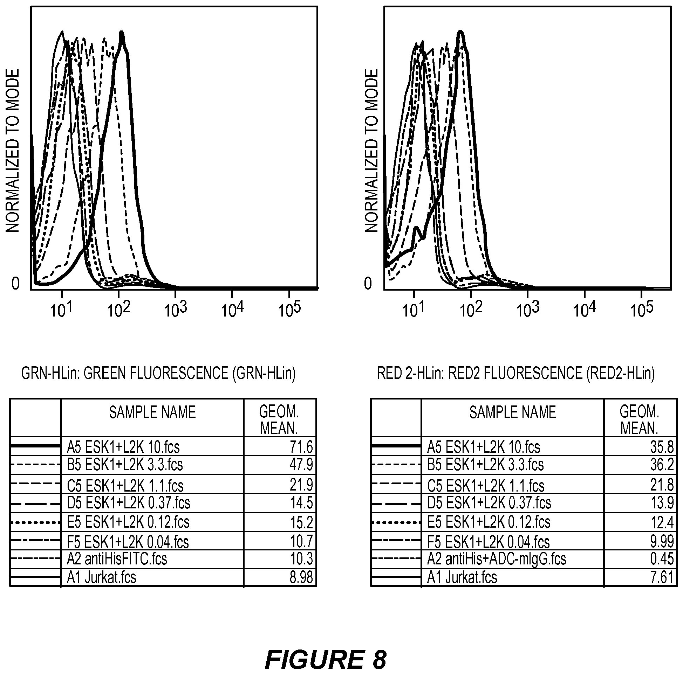

**Figure 8** shows the results of a FACS binding analysis comparing binding of ESK1-L2K bi-specific antibody with ET901+L2K bi-specific antibody and ET901+OKT3 bi-specific antibody.

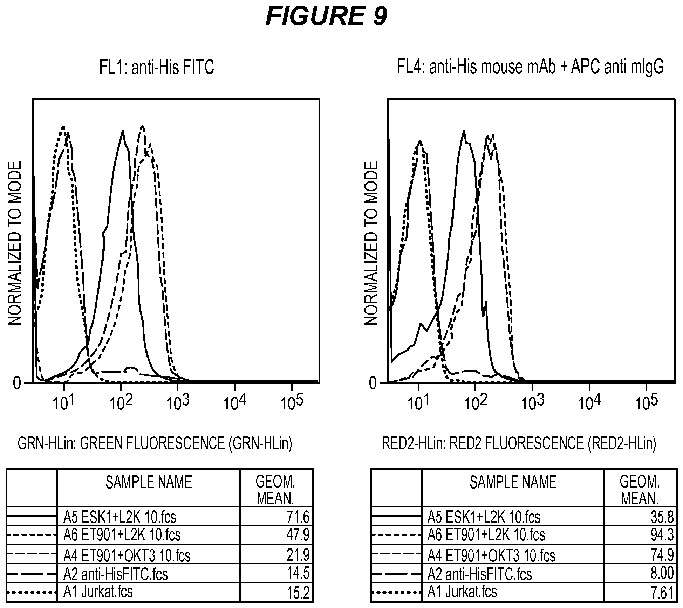

**Figure 9** shows the results of a FACS binding analysis comparing binding of ESK1-L2K bi-specific antibody with ET901+L2K bi-specific antibody and ET901+OKT3 bi-specific antibody.
**Figure 10** shows the amino acid sequence (SEQ ID NO: 110) of an embodiment of a bi-specific antibody comprising a scFv that specifically binds to WT1/HLA-A2 and a scFv that specifically binds to Cd3.

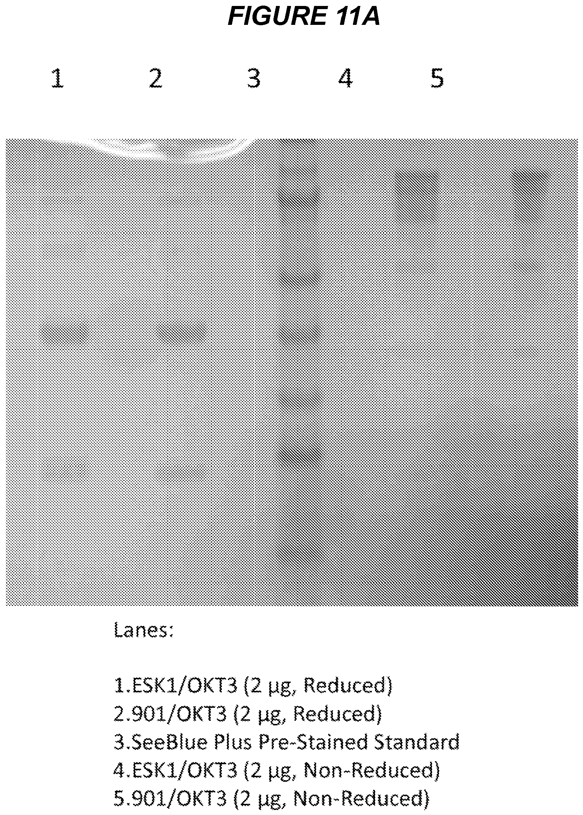

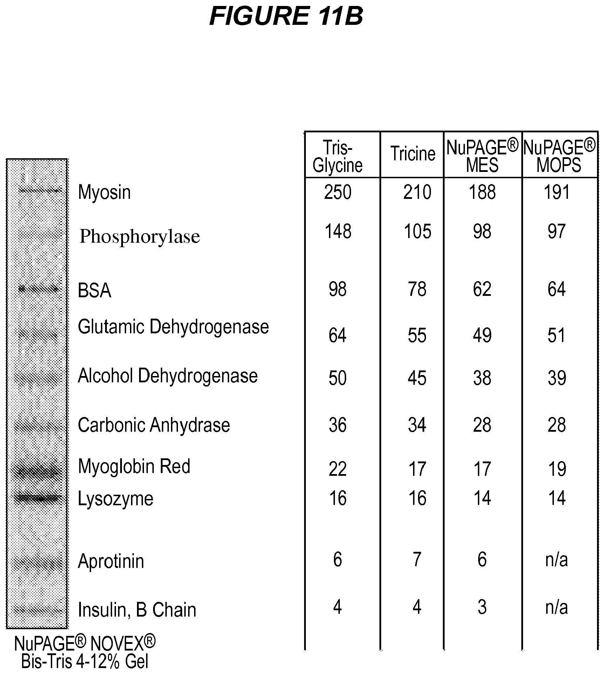

**Figure 11A** is a PAGE of 1) ESK1/OKT3 (2 µg, reduced); 2) 901/OKT3 (2 µg, reduced); 3) SeeBlue Plus Pre-Stained Standard; 4) ESK1/OKT3 (2 µg, non-reduced); 5) 901/OKT3 (2 µg, non-reduced). **11B** shows the standards.

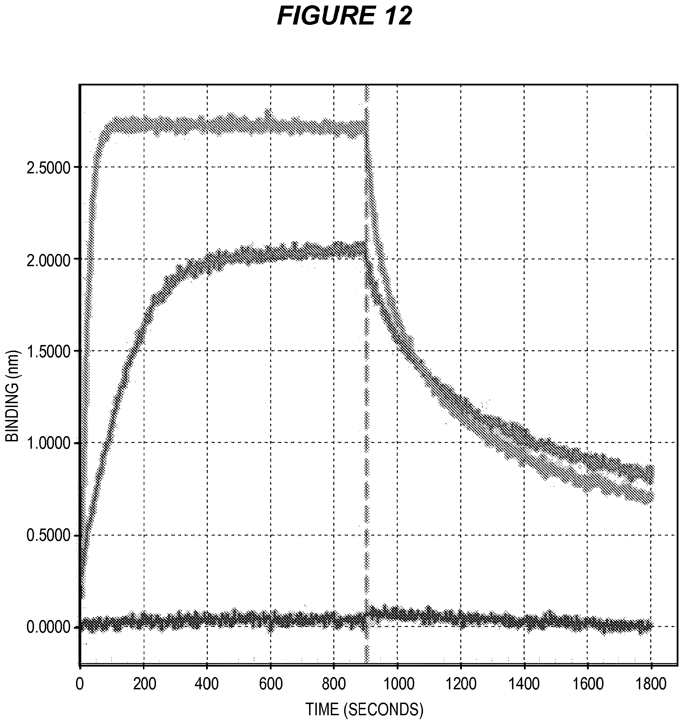

**Figure 12** shows the association and disassociation of ESK1 bi-specific monoclonal antibody against WT1/HLA-A2 complex.

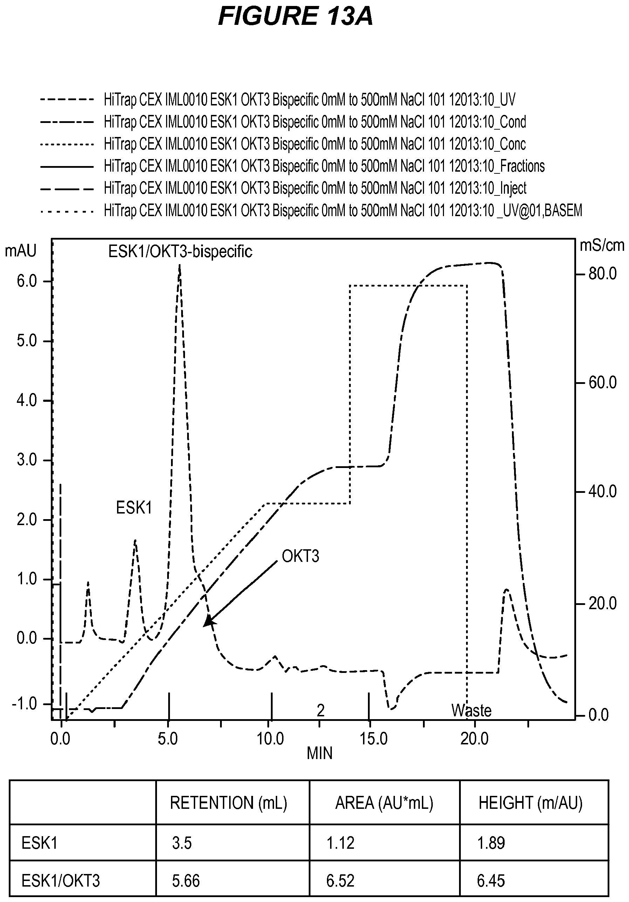

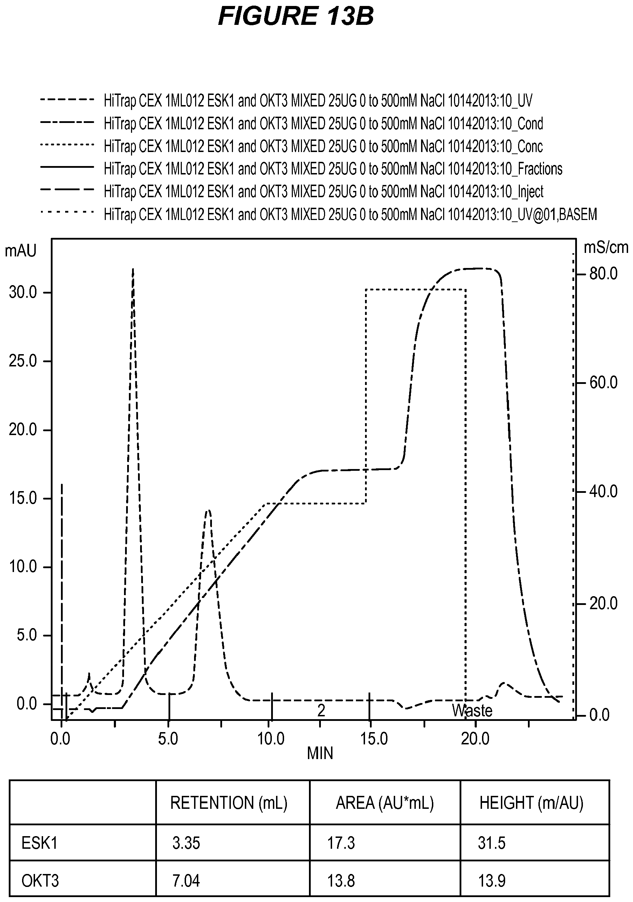

**Figure 13** shows the results of purification by cation exchange chromatography of ESK1/OKT3 bi-specific monoclonal antibody (**13A**) and a mixture of ESK1 and OKT3 (**13B**).

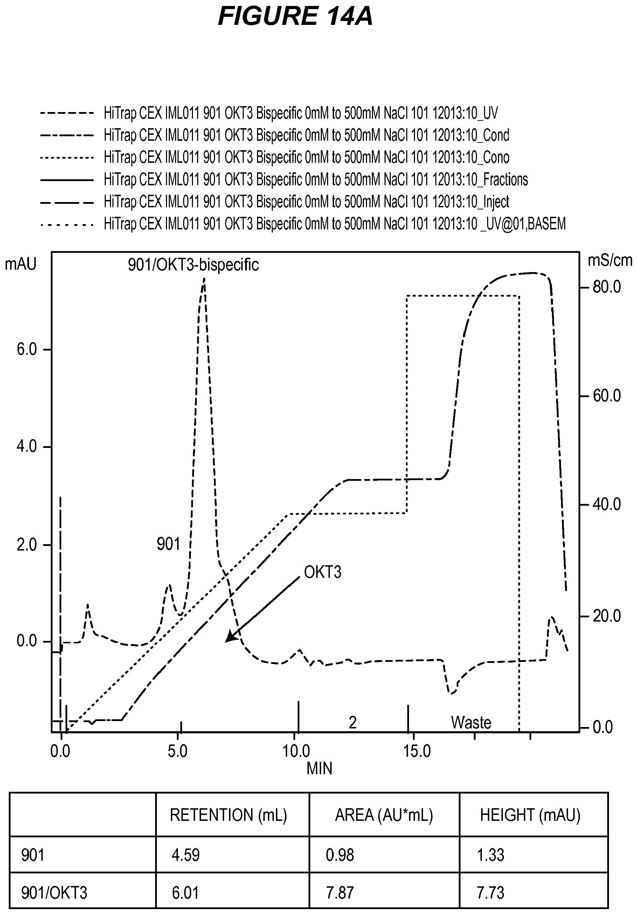

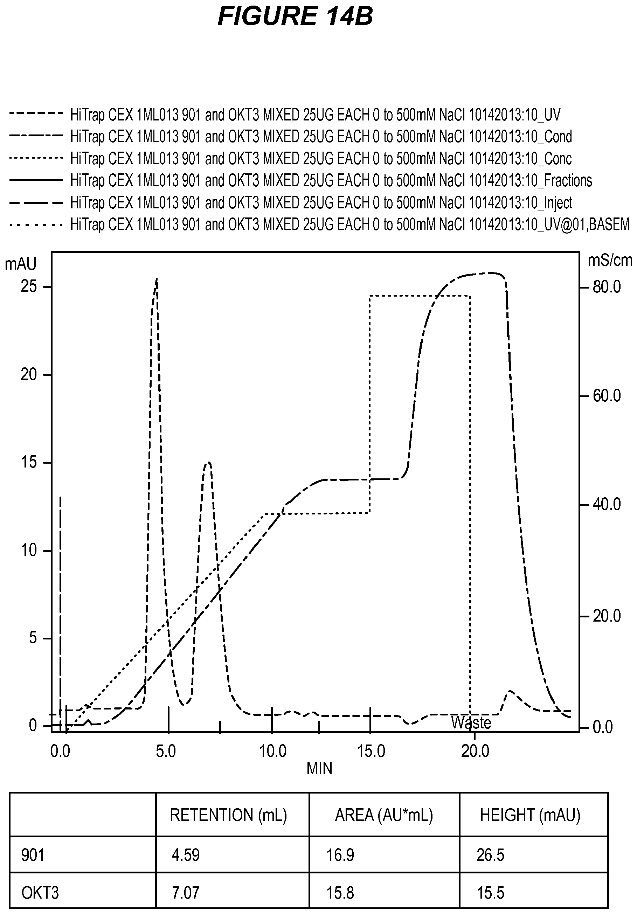

**Figure 14** shows the results of purification by cation exchange chromatography of ET901/OKT3 bi-specific monoclonal antibody (**14A**) and a mixture of 901 and OKT3 (**14B**).

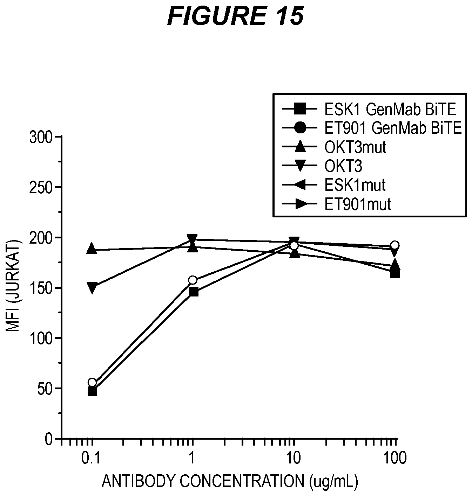

**Figure 15** shows binding towards CD3-positive Jurkat cells by FACS.

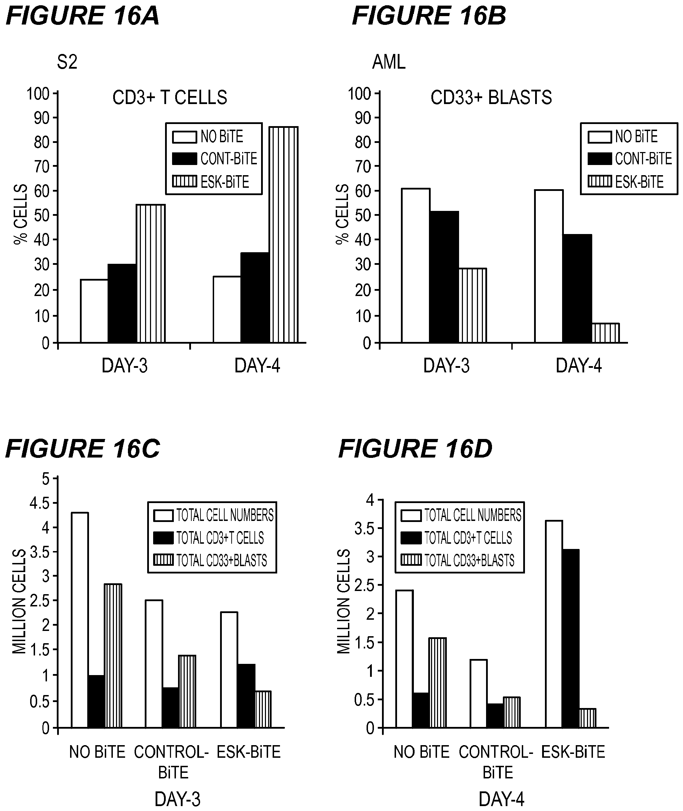

**Figure 16** shows ESK-bi-specific antibodies induce T cell activation in autologous settings. PBMCs from a HLA-A2+ patient with AML (before relapse) were co-cultured with purified autologous CD33+ myeloid blasts (after relapse) in the presence or absence of ESK or control-bi-specific antibody at 20 µg/ml and the cells were harvested and dual stained with CD33 for blasts (**16B**) and CD3 for T cells (**16A**) on day 3 and day 4, to assess the percentage of CD3 T cells and the blasts. Live cells from the CD3+ (**16C**) and CD33+ (**16D**) cultures were counted by trypan blue, and the absolute cell numbers were obtained by multiplying the percentage of each population times total cell numbers.

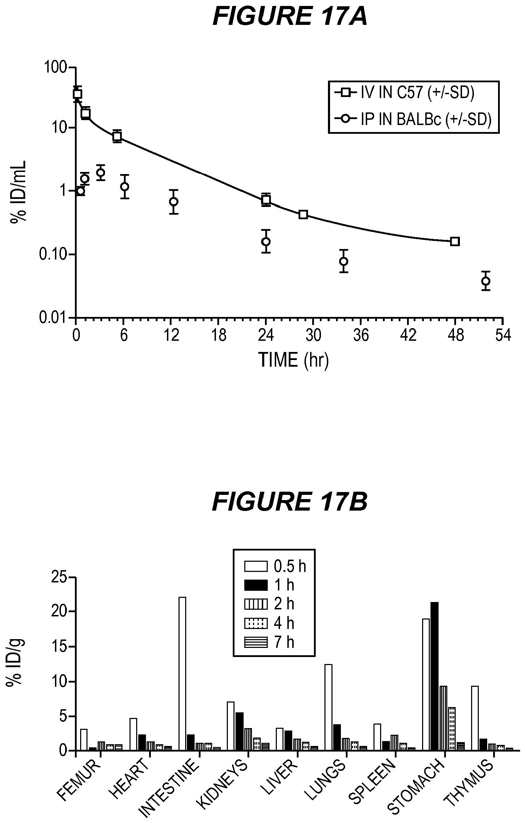

**Figure 17** shows pharmacokinetics of ESK-bi-specific antibody. Data represent the average of 3 mice/group. (**17A**) ESK bi-specific antibody pharmacokinetics was determined using trace ¹²⁵I-labeled construct injected either intravenously into C57 BL6/J, or intraperitoneally into BALB/c mice, and blood radioactivity was measured over 48-52 hours. Intravenously injected bi-specific antibody quickly redistributed from blood to tissue with an alpha half-life of 30 minutes, followed by clearance with a beta half-life of 5 hours. After intraperitoneal delivery, bi-specific antibody levels increased in the blood, peaking at 3 hours post-injection. The construct then cleared with the same beta half-life. Total exposure (Area Under the Curve) from intravenous injection was greater than with intraperitoneal injection. (**17B**) The biodistribution pattern of the antibodies was determined using the same radiolabeled constructs. After 2 hours, 3% or less of the injected dose/gram was detected in major tissues other than the stomach and intestinal tract, which clears dehalogenated iodine. After 4 hours, 2% or less of the injected dose/gram was detected in major tissues other than the stomach. After 7 hours, 1% or less of the injected dose/gram was detected in major tissues other than the stomach.

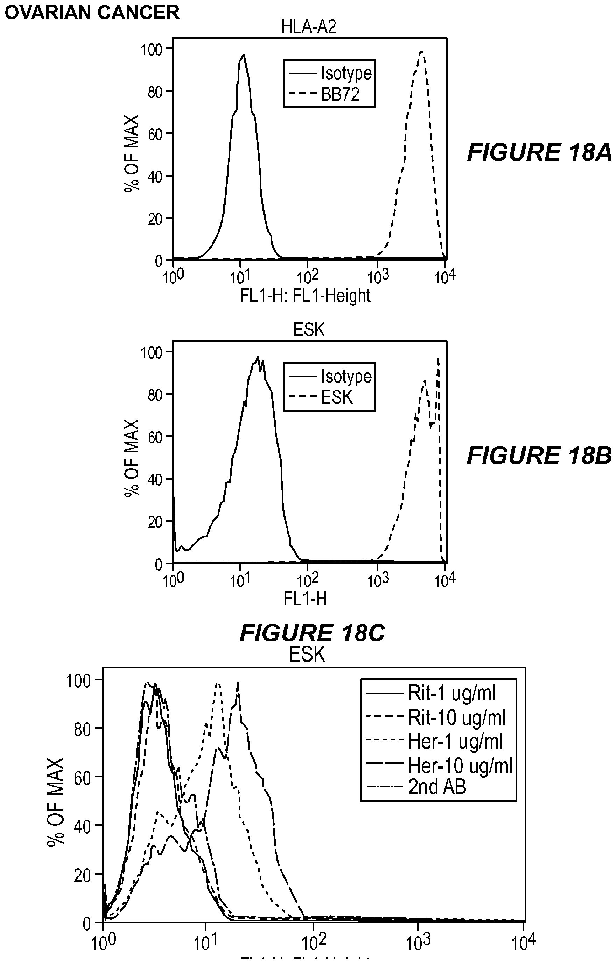

**Figure 18** shows the expression of tumor antigens on primary ovarian cancer cells. (**18A**) The expression of HLA-A2 on primary ovarian cancer cells was measured by staining the tumor cells with anti-human HLA-A2 mAb clone BB7.2 conjugated to FITC and its isotype control mouse IgG2b/FITC. (**18B**) Expression of the WT1 RMF/HLA-A2 complex on the same tumor cells was measured by staining the cells with mAb ESK conjugated to APC at 3 µg/ml and its isotype control, human IgG1/APC. (**18C**) Her2-neu expression on the same tumor cells was measured by staining the cells with Herceptin at 10 µg/ml or 1 µg/ml, followed by goat anti-human IgG1 mAb conjugated to FITC. Rituximab was used as isotype control.

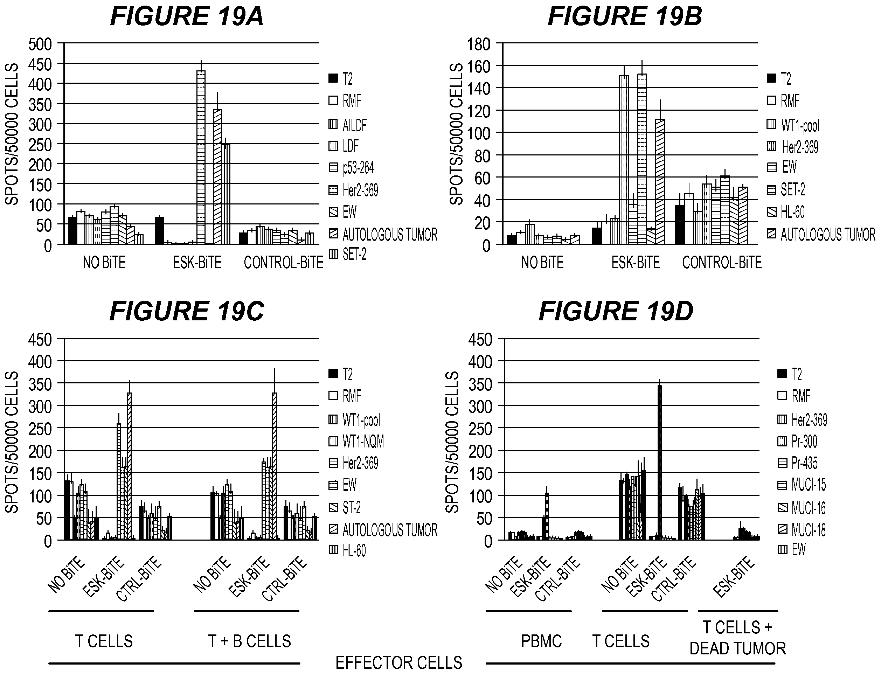

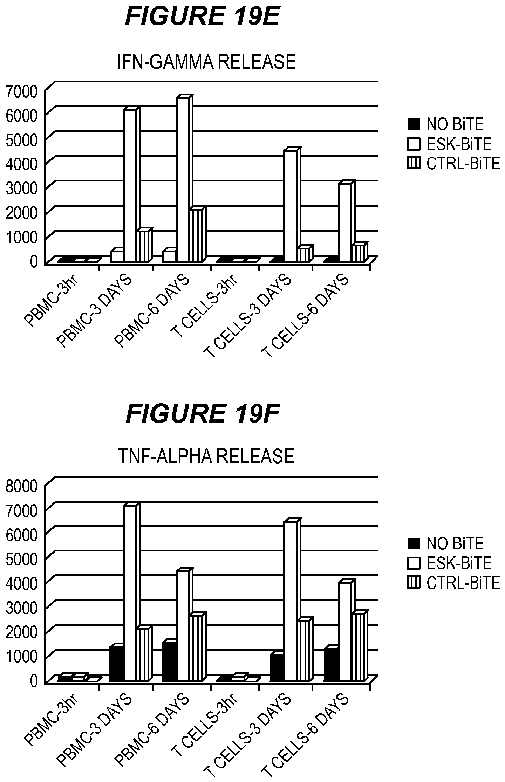

**Figure 19** shows ESK- bi-specific antibody induces secondary T cell responses to epitopes other than WT1 RMF in the context of HLA-A2 molecules. (**19A**) PBMCs from a patient with ovarian cancer were stimulated with autologous tumor cells at an effector: target ratio of 5:1, in the presence of ESK- bi-specific antibody or control bi-specific antibody at 0.1 µg/ml, human IL-5 (5 ng/ml) and human IL-2 (10 unit/ml) for a week and the epitope-specific response was measured by IFN-g elispot assay, against T2 cells, pulsed with indicated peptides at 20 µg/ml. (**19B**) Remaining PBMCs from the experiment in (A) were re-stimulated in the same manner, at an effector: target ratio of 9:1, and epitope-specific T cell response was measured by IFN-g elispot assay. The same stimulation protocol and IFN-g elispot assay were conducted to compare epitope-specific T cell response between (**19C**) purified CD3+ T cells versus PBMCs depleted of NK and macrophage (indicated as T+B), or (**19D**) PBMCs versus purified CD3+ T cells, or T cells stimulated with dead autologous tumor cells. Dead tumor cells were generated by frequent freeze thawing without DMSO. The data represent the average of triplicate culture +/-SD. Supernatant from the co-cultures of PBMCs or purified T cells with autologous ovarian cancer cells in the presence or absence of bi-specific antibodies at 0.1 µg/ml, were collected after 3 hours, 3 and 6 days and IFN-gamma (**19E**) and TNF-alpha (**19F**) were measured by ELISA kits.

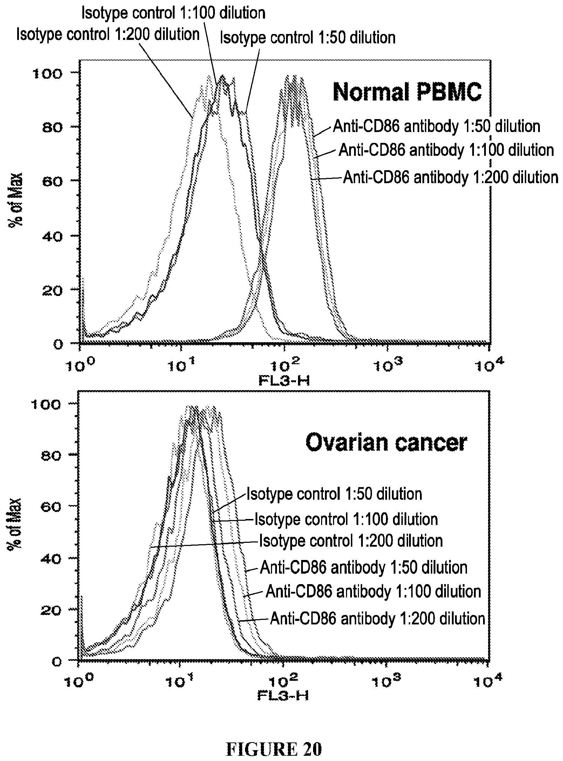

**Figure 20** shows co-stimulatory molecule CD86 expression on PBMCs (**top panel**) from a normal donor and ovarian cancer cells from a patient (**bottom panel**), measured by staining the cells with mouse anti-human CD86 mAb/PerCp or its isotype control at different concentrations. Isotype control: 1:50, 100 and 200 dilution. Anti-CD86 antibody: 1:50, 100 and 200 dilution.

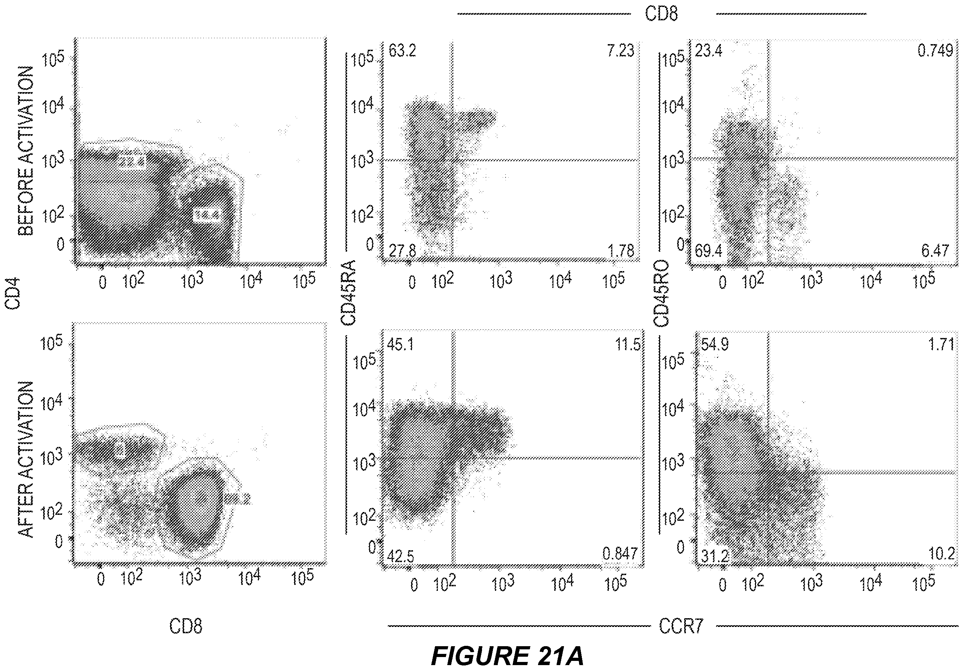

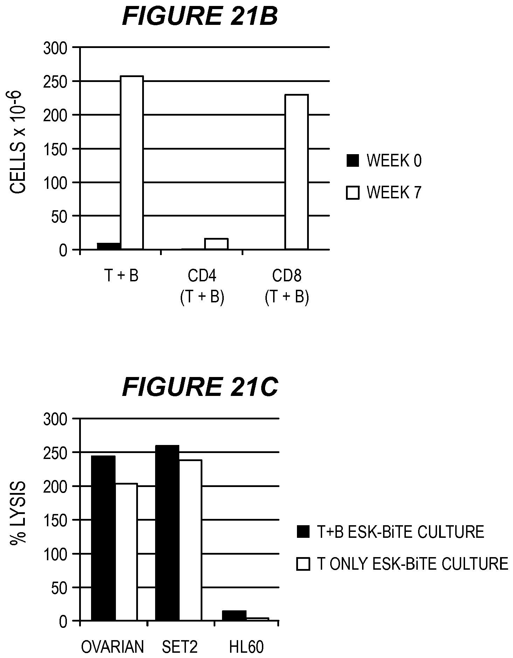

**Figure 21** shows generation of long-lived, cytotoxic effector cells. (**21A**) PBMCs from the patient in Figure 19 were stained with CD4, CD8, CD45RA, CD45RO and CCR7, before and 7 weeks after activated with ESK- bi-specific antibody (0.1 µg/ml) in the presence of autologous tumor cells. CD45RA and CD45RO versus CCR 7 were shown on gated CD8 T cells. (**21B**) The large selective increase in CD8 T cells 7 weeks after bi-specific antibody activation as measured by flow cytometry and cell counting. (**21C**) Effector cells from the experiments shown in Figure 19 were expanded by weekly supplement of IL-15 (5 ng/ml) and IL-2 (10 U/ml) for 5 weeks, and cytotoxicity was measured against autologous tumor, SET-2 and HL-60 cells by standard ⁵¹Cr-release assay. The data represent the average of triplicate wells.

### Detailed Description of the Invention

Unless otherwise defined herein, scientific and technical terms used in connection with the present invention shall have the meanings that are commonly understood by those of ordinary skill in the art. Further, unless otherwise required by context, singular terms shall include pluralities, and plural terms shall include the singular. Generally, nomenclatures used in connection with, and techniques of, cell and tissue culture, molecular biology, immunology, microbiology, genetics and protein and nucleic acid chemistry and hybridization described herein are those well-known and commonly used in the art. In practicing the present invention, many conventional techniques in immunology are used, which are within the skill of the ordinary artisan. These techniques are described in greater detail in, for example, "Current Protocols in Immunology" (John E. Coligan et al., eds., John Wiley & Sons, Inc. 1991 and periodic updates); Recombinant Antibodies for Immunotherapy, Melvyn Little, ed. Cambridge University Press 2009.

The following abbreviations are used throughout the application and are generally intended to be interpreted consistently with the meaning of the terms as known in the art:
Ab: Antibody
ADCC: Antibody-dependent cellular cytotoxicity
ALL: Acute lymphocytic leukemia
AML: Acute myeloid leukemia
BiTE: Bi-specific T-cell engager
CDC: Complement dependent cytotoxicity
CMC: Complement mediated cytotoxicity
CDR: Complementarity determining region (see also HVR below)
CL: Constant domain of the light chain
CH1: 1st constant domain of the heavy chain
CH1, 2, 3: 1st, 2nd and 3rd constant domains of the heavy chain
CH2, 3: 2nd and 3rd constant domains of the heavy chain
CHO: Chinese hamster ovary
CML: chronic myelogenous leukemia; also referred to as chronic myelocytic leukemia and chronic myeloid leukemia
CTL: Cytotoxic T cell
E:T Ratio: Effector:Target ratio
Fab: Antibody binding fragment
FACS: Fluorescence-activated cell sorting
FBS: Fetal bovine serum
FR: Framework region
GVHD: Graft-versus-host disease
HC: Heavy chain
HLA: Human leukocyte antigen
HVR-H: Hypervariable region-heavy chain (see also CDR)
HVR-L: Hypervariable region-light chain (see also CDR)
Ig: Immunoglobulin
KD: Dissociation constant
k_{off}: Dissociation rate
kₒₙ: Association rate
MHC: Major histocompatibility complex
MM: Multiple myeloma
scFv: Single-chain variable fragment
V_{H}: Variable heavy chain includes heavy chain hypervariable region and heavy chain variable framework region
V_{L}: Variable light chain includes light chain hypervariable region and light chain variable framework region
WT1: Wilms tumor protein 1

In the description that follows, terms used herein are intended to be interpreted consistently with the meaning of those terms as they are known to those of skill in the art. The definitions provided herein below are meant to clarify, but not limit, the terms defined.

As used herein, "administering" and "administration" refer to the application of an active ingredient to the body of a subject.

"Antibody" and "antibodies" as those terms are known in the art refer to antigen binding proteins of the immune system. The term "antibody" as referred to herein includes whole, full length antibodies having an antigen-binding region, and any fragment thereof in which the "antigen-binding portion" or "antigen-binding region" is retained, or single chains, for example, single chain variable fragment (scFv), thereof. A naturally occurring "antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant (CH) region. The heavy chain constant region is comprised of three domains, CH1, CH2 and CH3. Each light chain is comprised of a light chain variable region (abbreviated herein as VL) and a light chain constant CL region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The term "antigen-binding portion" or "antigen-binding region" of an antibody, as used herein, refers to that region or portion of the antibody that binds to the antigen and which confers antigen specificity to the antibody; fragments of antigen-binding proteins, for example, antibodies includes one or more fragments of an antibody that retain the ability to specifically bind to an antigen (e.g., an peptide/HLA complex). It has been shown that the antigen-binding function of an antibody can be performed by fragments of a full-length antibody. Examples of antigen-binding fragments encompassed within the term "antibody fragments" of an antibody include a Fab fragment, a monovalent fragment consisting of the VL, VH, CL and CH1 domains; a F(ab)2 fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., 1989 Nature 341:544-546), which consists of a VH domain; and an isolated complementarity determining region (CDR).

Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules. These are known as single chain Fv (scFv); see e.g., Bird et al., 1988 Science 242:423-426; and Huston et al., 1988 Proc. Natl. Acad. Sci. 85:5879-5883. These antibody fragments are obtained using conventional techniques known to those of skill in the art, and the fragments are screened for utility in the same manner as are intact antibodies.

As used herein, the term "effective amount" means that amount of a compound or therapeutic agent that will elicit the biological or medical response of a tissue, system, animal, or human that is being sought, for instance, by a researcher or clinician.

The term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

The present disclosure relates to compositions and methods of treatment relating to recombinant bi-specific antibodies. In one aspect, the disclosure relates to a method of stimulating a primary T cell response and a secondary T cell response in a subject comprising administering a composition comprising a recombinant antibody, said recombinant antibody comprising a first antigen-binding portion and second antigen-binding portion, wherein said first antigen-binding portion specifically binds to a first tumor antigen and said second antigen-binding portion specifically binds to an immune effector cell surface antigen, wherein the primary T cell response comprises stimulating cytotoxic T cells against the first tumor antigen, and wherein the secondary T cell response comprises stimulating effector T cells and/or memory T cells against the first tumor antigen and/or against a second tumor antigen. In one aspect, the first tumor antigen is WT1/HLA and the second tumor antigen is a nont-WT1-RMF tumor antigen. In a related aspect, the disclosure relates to a method of producing effector T cells and/or memory T cells against a tumor antigen, optionally a non-WT1-RMF tumor antigen such as HER2-neu. The methods of the present disclosure may further comprise administering an immune effector cell, for example, a cytotoxic cell such as a CD3+ cytotoxic T cell. Through the production of effector T cells, e.g., cytotoxic T cells, and/or memory T cells against a tumor antigen, the bi-specific antibodies of the present disclosure are thus able to achieve a vaccinal effect against tumor cells and can be used to generate cells for use in adoptive T cell therapy.

In another aspect, the present disclosure relates to an improved anti-WT1 antibody useful for killing WT1 positive cells. The improved anti-WT1 antibody is a bi-specific antibody with a first antigen binding portion that specifically binds WT1 when it is presented in a histocompatibility-restricted fashion with HLA and a second antigen-binding portion that specifically binds to a cell surface antigen on the surface of an immune effector cell, for example, CD3 and, therefore, is able to engage immune effector cells, for example, CD3⁺ T cells (i.e., cytotoxic T cells). In one aspect, a recombinant antibody or derivative or fragment thereof according to the present disclosure comprises: (i) a first antigen-binding portion comprising: (A) a heavy chain (HC) variable region comprising HC-CDR1, HC-CDR2 and HC-CDR3; and a light chain (LC) variable region comprising LC-CDR1, LC-CDR2 and LC-CDR3, comprising amino acid sequences set forth in Tables 1-6; (B) a VH and a VL comprising first and second amino acid sequences as set forth in Tables 1-6; or (C) an scFv comprising an amino acid sequence as set forth in Tables 1-6; and (ii) a second antigen-binding portion comprising an amino acid sequence set forth in Table 7. In one aspect, the first antigen binding portion and/or second antigen binding portion is an antibody fragment. Examples of antibody fragments include, but are not limited to, a Fab fragment; a monovalent fragment consisting of the VL, VH, CL and CH1 domains; a F(ab)2 fragment; a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment; an isolated CDR; and a scFv.

In one embodiment, the bi-specific antibody has a first binding portion with a variable heavy chain region comprising the amino acid sequence of SEQ ID NO: 50 and a variable light chain region comprising the amino acid sequence SEQ ID NO: 52 or a scFv with the amino acid sequence of SEQ ID NO: 54. Furthermore, the bi-specific antibody has a second binding portion comprising an L2K light chain variable region comprising the amino acid sequence of SEQ ID NO: 111 and an L2K heavy chain variable region comprising the amino acid sequence of SEQ ID NO: 112.

In other embodiments, the WT1/HLA binding portion of the bi-specific anti-WT1 antibody of the invention comprises one or more of the amino acid sequences (scFv, VH and VL regions or CDRs) listed in Tables 1-6 or 8.

In the sequences that follow in Tables 1-7, bolded text indicates a linker sequence between hypervariable heavy and light chain sequences.

In some embodiments, the antibody comprises a histidine tag for purification. In some embodiments, the antibody comprises a signal peptide and one or more linkers comprising glycine and serine residues.

**Table 1**

| Antigen | WT1 | | |
|---|---|---|---|
| Peptide | RMFPNAPYL (SEQ ID NO: 1) | | |
| CDRs: | 1 | 2 | 3 |
| VH | GGTFSSYAIS (SEQ ID NO: 2) | GIIPIFGTANYAQKFQG (SEQ ID NO: 3) | RIPPYYGMDV (SEQ ID NO: 4) |
| DNA | | | |
| VL | SGSSSNIGSNYVY (SEQ ID NO: 8) | RSNQRPS (SEQ ID NO: 9) | AAWDDSLNGVV (SEQ ID NO: 10) |
| DNA | | aggagtaatcagcggccctca (SEQ ID NO: 12) | |
| Full VH | | | |
| DNA | | | |
| Full VL | | | |
| DNA | | | |
| scFv | | | |
| DNA | | | |

**Table 2**

| Antigen | WT1 (Ext002 #5) | | |
|---|---|---|---|
| Peptide | RMFPNAPYL (SEQ ID NO: 1) | | |
| CDRs: | 1 | 2 | 3 |
| VH | GDSVSSNSAAWN (SEQ ID NO: 20) | RTYYGSKWYNDYAVS VKS (SEQ ID NO: 21) | GRLGDAFDI (SEQ ID NO: 22) |
| DNA | | | |
| VL | RASQSISSYN (SEQ ID NO: 26) | AASSLQS (SEQ ID NO: 27) | QQSYSTPLT (SEQ ID NO: 28) |
| DNA | | gctgcatccagtttgcaaagt (SEQ ID NO: 30) | |
| Full VH | | | |
| DNA | | | |
| Full VL | | | |
| DNA | | | |
| scFv | | | |
| DNA | | | |

**Table 3**

| Antigen | WT1 (Ext002 #13) | | |
|---|---|---|---|
| Peptide | RMFPNAPYL (SEQ ID NO: 1) | | |
| CDRs: | 1 | 2 | 3 |
| VH | GYSFTNFWIS (SEQ ID NO: 38) | RVDPGYSYSTYSPSFQ G (SEQ ID NO: 39) | VQYSGYYDWFDP (SEQ ID NO: 40) |
| DNA | | | |
| VL | SGSSSNIGSNTVN (SEQ ID NO: 44) | SNNQRPS (SEQ ID NO: 45) | AAWDDSLNGWV (SEQ ID NO: 46) |
| DNA | | agtaataatcagcggccctca (SEQ ID NO: 48) | |
| Full VH | | | |
| DNA | | | |
| Full VL | | | |
| DNA | | | |
| scFv | | | |
| DNA | | | |

**Table 4**

| Antigen | WT1 (Ext002 #15) | | |
|---|---|---|---|
| Peptide | RMFPNAPYL (SEQ ID NO: 1) | | |
| CDRs: | 1 | 2 | 3 |
| VH | GYNFSNKWIG (SEQ ID NO: 56) | IIYPGYSDITYSPSFQG (SEQ ID NO: 57) | HTALAGFDY (SEQ ID NO: 58) |
| DNA | | | |
| VL | RASQNINKWLA (SEQ ID NO: 62) | KASSLES (SEQ ID NO: 63) | QQYNSYAT (SEQ ID NO: 64) |
| DNA | | aaggcgtctagtttagaaagt (SEQ ID NO: 66) | caacaatataatagttatgcgacg (SEQ ID NO: 67) |
| Full VH | | | |
| DNA | | | |
| Full VL | | | |
| DNA | | | |
| scFv | | | |
| DNA | | | |

**Table 5**

| Antigen | WT1 (Ext002 #18) | | |
|---|---|---|---|
| Peptide | RMFPNAPYL (SEQ ID NO: 1) | | |
| CDRs: | 1 | 2 | 3 |
| VH | GFTFDDYGMS (SEQ ID NO: 74) | | ERGYGYHDPHDY (SEQ ID NO: 76) |
| DNA | | | |
| VL | GRNNIGSKSVH (SEQ ID NO: 80) | DDSDRPS (SEQ ID NO: 81) | QVWDSSSDHVV (SEQ ID NO: 82) |
| DNA | | gatgatagcgaccggccctca (SEQ ID NO: 84) | |
| Full VH | | | |
| DNA | | | |
| Full VL | | | |
| DNA | | | |
| scFv | | | |
| DNA | | | |

**Table 6**

| Antigen | WT1 (Ext002 #23) | | |
|---|---|---|---|
| Peptide | RMFPNAPYL (SEQ ID NO. 1) | | |
| CDRs: | 1 | 2 | 3 |
| VH | GFSVSGTYMG (SEQ ID NO. 92) | LLYSGGGTYHPASLQG (SEQ ID NO. 93) | GGAGGGHFDS (SEQ ID NO. 94) |
| DNA | | | |
| VL | TGSSSNIGAGYDVH (SEQ ID NO. 98) | GNSNRPS (SEQ ID NO. 99) | AAWDDSLNGYV (SEQ ID NO. 100) |
| DNA | | ggtaacagcaatcggccctca (SEQ ID NO. 102) | |
| Full VH | | | |
| DNA | | | |
| Full VL | | | |
| DNA | | | |
| scFv | | | |
| DNA | | | |

**Table 7**

| Antigen | CD3/L2K |
|---|---|
| Full VL | |
| Full VH | |
| scFv | |
| DNA (without signal sequence and his tag) | |
| DNA (with signal sequence and hexahistidine tag | |

In one embodiment, the ESK1-bi-specific antibody comprises a first antigen - binding portion that specifically binds to WT1/HLA-A2 and comprises one of the amino acid sequences of an WT1/HLA-A2 antibody as set forth in Tables 1-6 and an antigen-binding portion that binds to CD3 and comprises an amino acid sequence shown in Table 7 above. In one embodiment, the antibody has the amino acid sequence shown in Figure 10 (SEQ ID NO: 110). In another embodiment, the ESK1-bi-specific antibody comprises a first antigen-binding portion that specifically binds to WT1/HLA-A2 and comprises one of the amino acid sequences of an WT1/HLA-A2 antibody as set forth in Table 8 and an antigen-binding portion that binds to OKT3 and comprises an amino acid sequence shown in Table 9.

**Table 8**

| Antigen | WT1 | | |
|---|---|---|---|
| CDRs: | 1 | 2 | 3 |
| VH | GYSFTNFWIS (SEQ ID NO: 116) | RVDPGYSYSTYSPSFQ (SEQ ID NO: 117) | VQYSGYYDWFDP (SEQ ID NO: 118) |
| DNA | | | |
| VL | SGSSSNIGSNTVN (SEQ ID NO: 122) | SNNQRPS (SEQ ID NO: 123) | AAWDDSLNGWV (SEQ ID NO: 124) |
| DNA | | agtaataatcagcggccctca (SEQ ID NO: 126) | |
| Full HC | | | |
| DNA | | | |
| Full LC | | | |
| DNA | | | |
| scFv | | | |
| scFv DNA | | | |

**Table 9**

| Antigen | OKT3 |
|---|---|
| Full HC | |
| DNA | |
| Full LC | |
| DNA | |

The recombinant bi-specific antibodies of the present disclosure also include substantially homologous polypeptides having antigen-binding portions that are at least 70%, at least 75%, at least 80%, at least 85%, at least 90%, at least 95%, at least 97%, at least 98%, or at least 99% identical to the peptides described in Tables 1-9.

Bi-specific antibodies according to the present disclosure may be prepared by any of a number of conventional techniques. For example, they may be produced in recombinant expression systems, using any technique known in the art. See, for example, Monoclonal Antibodies, Hybridomas: A New Dimension in Biological Analyses, Kennet et al. (eds.), Plenum Press, New York (1980); and Antibodies: A Laboratory Manual, Harlow and Land (eds.), Cold Spring Harbor Laboratory Press, Cold Spring Harbor, N.Y., (1988). Certain of the techniques involve isolating a nucleic acid encoding a polypeptide chain (or portion thereof) of an antibody of interest, and manipulating the nucleic acid through recombinant DNA technology. The nucleic acid may be fused to another nucleic acid of interest, or altered (e.g., by mutagenesis or other conventional techniques) to add, delete, or substitute one or more amino acid residues.

Any expression system known in the art can be used to make the recombinant bi-specific antibodies of the present disclosure. In general, host cells are transformed with a recombinant expression vector that comprises DNA encoding a desired polypeptide. Among the host cells that may be employed are prokaryotes, yeast or higher eukaryotic cells. Prokaryotes include gram negative or gram positive organisms, for example E. coli or bacilli. Higher eukaryotic cells include insect cells and established cell lines of mammalian origin. Examples of suitable mammalian host cell lines include the COS-7 line of monkey kidney cells (ATCC CRL 1651) (Gluzman et al., 1981, Cell 23:175), L cells, 293 cells, C127 cells, 3T3 cells (ATCC CCL 163), Chinese hamster ovary (CHO) cells, HeLa cells, BHK (ATCC CRL 10) cell lines, and the CVI/EBNA cell line derived from the African green monkey kidney cell line CVI (ATCC CCL 70) as described by McMahan et al., 1991, EMBO J. 10: 2821. Appropriate cloning and expression vectors for use with bacterial, fungal, yeast, and mammalian cellular hosts are described in the art, e.g., by Pouwels et al., Cloning Vectors: A Laboratory Manual, Elsevier, N.Y., 1985.

The transformed cells can be cultured under conditions that promote expression of the polypeptide, and the polypeptide recovered by conventional protein purification procedures. One such purification procedure includes the use of affinity chromatography, e.g., over a matrix having all or a portion of the antigen bound thereto. Polypeptides contemplated for use herein include substantially homogeneous recombinant bi-specific antibodies substantially free of contaminating endogenous materials.

In one aspect, bi-specific antibodies according to the present disclosure are produced from host cells transformed with a recombinant expression vector encoding a peptide having a first antigen-binding portion and a second antigen binding portion. In another aspect, a bi-specific antibody of the present disclosure is produced from two separate antibodies, i.e., an antibody having a first antigen-binding portion and an antibody having a second antigen binding portion, that are linked, e.g., using disulfide bonds.

The amino acid sequence of the bi-specific antibodies disclosed herein may be verified by any means known in the art, and may be identical to the sequences disclosed herein in Tables 1-9, or may differ from those sequences at one or more amino acid residues as result of processing. For example, on all or a portion of the substantially homogenous bi-specific antibodies, a C-terminal amino acid from either the light chain or the heavy chain (or relevant single-chain molecule) may be removed, by proteolytic processing or other processing that occurs during culture, for example, processing of C-terminal Lys residues. Alternatively, more than one C-terminal amino acid residue may be removed, for example two C-terminal amino acids, or three, four or five C-terminal amino acids. Similarly, N-terminal amino acids may be absent, for example, one, two, three, four or five N-terminal amino acids may be absent.

Alternatively, or additionally, the bi-specific antibodies may undergo post-translational modifications, for example but not limited to, a glutamine may be cyclized or converted to pyroglutamic acid; additionally or alternatively, amino acids may undergo deamidation, isomerization, glycation and/or oxidation. The polypeptides of the invention may undergo additional post-translational modification, including glycosylation, for example N-linked or O-linked glycosylation, at sites that are well-known in the art. As described previously, changes may be made in the amino acid sequence of a polypeptide to preclude or minimize such alterations, or to facilitate them in circumstances where such processing is beneficial.

Bi-specific antibodies according to the present disclosure include polypeptides that have been modified in any way and for any reason, for example, to: (1) reduce susceptibility to proteolysis, (2) reduce susceptibility to oxidation, (3) alter binding affinity for forming protein complexes, (4) alter binding affinities, and (4) confer or modify other physicochemical or functional properties. Additionally, single or multiple amino acid substitutions (e.g., conservative amino acid substitutions) made in a sequence described in any of Tables 1-9 (e.g., in the portion of the polypeptide outside the domain(s) forming intermolecular contacts) are encompassed by the present disclosure. Systematic substitution of one or more amino acids of a consensus sequence with a D-amino acid of the same type (e.g., D-lysine in place of L-lysine) may also be used, e.g., to generate more stable peptides. In addition, consensus sequences can be used to select amino acid residues for substitution; those of skill in the art recognize that additional amino acid residues may also be substituted. Constrained peptides comprising a consensus sequence or a substantially identical consensus sequence variation may be generated by methods known in the art (Rizo and Gierasch Ann. Rev. Biochem. 61:387 (1992)).

Bi-specific antibodies according to the present disclosure can comprise any constant region known in the art. The light chain constant region can be, for example, a kappa- or lambda-type light chain constant region, e.g., a human kappa- or lambda-type light chain constant region. The heavy chain constant region can be, for example, an alpha-, delta-, epsilon-, gamma-, or mu-type heavy chain constant regions, e.g., a human alpha-, delta-, epsilon-, gamma-, or mu-type heavy chain constant region. In one aspect, the light or heavy chain constant region is a fragment, derivative, variant, or mutein of a naturally occurring constant region.

In another aspect, the disclosure relates to a derivative or analog of a bi-specific antibody of the present disclosure. A derivative can comprise any molecule or substance that imparts a desired property, such as increased half-life in a particular use. Examples of molecules that can be used to form a derivative include, but are not limited to, albumin (e.g., human serum albumin) and polyethylene glycol (PEG). Derivatives such as albumin-linked and PEGylated derivatives of antibodies can be prepared using techniques well known in the art. An analog may be a non-peptide analog of a bi-specific antibody described herein. Non-peptide analogs are commonly used in the pharmaceutical industry as drugs with properties analogous to those of the template peptide. These types of non-peptide compound are termed "peptide mimetics" or "peptidomimetics," see, for example, Fauchere, J. Adv. Drug Res. 15:29 (1986); Veber and Freidinger TINS p. 392 (1985); and Evans et al. J. Med. Chem. 30:1229 (1987). Peptide mimetics that are structurally similar to the bi-specific antibodies of the present disclosure may be used to produce an equivalent therapeutic or prophylactic effect. Generally, peptidomimetics are structurally similar to a paradigm polypeptide (i.e., a polypeptide that has a desired biochemical property or pharmacological activity), such as a human antibody, but have one or more peptide linkages optionally replaced by a linkage selected from the group consisting of: -CH₂NH-, -CH₂S-, -CH₂-CH₂-, -CH=CH-(cis and trans), -COCH₂-, -CH(OH)CH₂-, and -CH₂SO-, by methods well known in the art.

In one aspect, a recombinant bi-specific antibody according to the present disclosure comprises: (I) a first antigen-binding portion comprising one of: (A) a scFV comprising an amino acid sequence selected from the group consisting of SEQ ID NOS: 18, 36, 54, 72, 90, 108, and 132; or (B) a heavy chain variable domain (VH) and a light chain variable domain (VL), wherein the VH and VL, respectively, comprise amino acid sequences selected from the group consisting of SEQ ID NOS: (i) 14 and 16; (ii) 32 and 34; (iii) 50 and 52; (iv) 68 and 70; (v) 86 and 88; (vi) 104 and 106; and (vii) 128 and 130; or (C) (i) the following three VH complementarity determining regions (CDRs): (a) a VH CDR1 comprising the amino acid sequence selected from the group consisting of SEQ ID NOS: 2, 20, 38, 56, 74, 92 and 116; and (b) a VH CDR2 comprising an amino acid sequence selected from the group consisting of SEQ ID NOS: 3, 21, 39, 57, 75, 93, and 117; and (c) a VH CDR3 comprising an amino acid sequence selected from the group consisting of SEQ ID NOS: 4, 22, 40, 58, 76, 94, and 118; and (ii) the following three VL CDRs: (a) a VL CDR1 comprising an amino acid sequence selected from the group consisting of SEQ ID NOS: 8, 26, 44, 62, 80, 98, and 122; and (b) a VL CDR2 comprising an amino acid sequence selected from the group consisting of SEQ ID NOS: 9, 27, 45, 63, 81, 99, and 123; and (c) a VL CDR3 comprising an amino acid sequence selected from the group consisting of SEQ ID NOS: 10, 28, 46, 64, 82, 100, and 124; and (II) a second antigen-binding portion comprising (A) a single chain variable fragment (scFV) comprising the amino acid sequence set forth in SEQ ID NO: 113; or (B) a heavy chain variable domain (VH) and a light chain variable domain (VL), wherein the VH and VL, respectively, comprise the amino acid sequences set forth in SEQ ID NOS: 112 and 111 or SEQ ID NOS: 134 and 136.

In one aspect, the recombinant antibody comprises a first antigen-binding portion comprising one of: (A) a scFV comprising the amino acid sequence set forth in SEQ ID NO: 18; or (B) a VH comprising the amino acid sequence set forth in SEQ ID NO: 14 and a VL comprising the amino acid sequences set forth in SEQ ID NO: 16; or (C) a VH CDR1 comprising the amino acid sequence set forth in SEQ ID NO: 2; a VH CDR2 comprising the amino acid sequence set forth in SEQ ID NO: 3; a VH CDR3 comprising the amino acid sequence set forth in SEQ ID NO: 4; a VL CDR1 comprising the amino acid sequence set forth in SEQ ID NO: 8; a VL CDR2 comprising the amino acid sequence set forth in SEQ ID NO: 9; and a VL CDR3 comprising the amino acid sequence set forth in SEQ ID NO: 10.

In another aspect, the recombinant antibody comprises a first antigen-binding portion comprising one of: (A) a scFV comprising the amino acid sequence set forth in SEQ ID NO: 36; or (B) a VH comprising the amino acid sequence set forth in SEQ ID NO: 32 and a VL comprising the amino acid sequence set forth in SEQ ID NO: 34; or (C) a VH CDR1 comprising the amino acid sequence set forth in SEQ ID NO: 20; a VH CDR2 comprising the amino acid sequence set forth in SEQ ID NO: 21; a VH CDR3 comprising the amino acid sequence set forth in SEQ ID NO: 22; a VL CDR1 comprising the amino acid sequence set forth in SEQ ID NO: 26; a VL CDR2 comprising the amino acid sequence set forth in SEQ ID NO: 27; and a VL CDR3 comprising the amino acid sequence set forth in SEQ ID NO: 28.

In another aspect, the recombinant antibody comprises a first antigen-binding portion comprising one of: (A) scFV comprising the amino acid sequence set forth in SEQ ID NO: 54; or (B) a VH comprising the amino acid sequence set forth in SEQ ID NO: 50 and a VL comprising the amino acid sequences set forth in SEQ ID NO: 52; or (C) a VH CDR1 comprising the amino acid sequence set forth in SEQ ID NO: 38; a VH CDR2 comprising the amino acid sequence set forth in SEQ ID NO: 39; a VH CDR3 comprising the amino acid sequence set forth in SEQ ID NO: 40; a VL CDR1 comprising the amino acid sequence set forth in SEQ ID NO: 44; a VL CDR2 comprising the amino acid sequence set forth in SEQ ID NO: 45; and a VL CDR3 comprising the amino acid sequence set forth in SEQ ID NO: 46.

In another aspect, the recombinant antibody comprises a first antigen-binding portion comprising one of: (A) scFV comprising the amino acid sequence set forth in SEQ ID NO: 72; or (B) a VH comprising the amino acid sequence set forth in SEQ ID NO: 68 and a VL comprising the amino acid sequences set forth in SEQ ID NO: 70; or (C) a VH CDR1 comprising the amino acid sequence set forth in SEQ ID NO: 56; a VH CDR2 comprising the amino acid sequence set forth in SEQ ID NO: 57; a VH CDR3 comprising the amino acid sequence set forth in SEQ ID NO: 58; a VL CDR1 comprising the amino acid sequence set forth in SEQ ID NO: 62; a VL CDR2 comprising the amino acid sequence set forth in SEQ ID NO: 63; and a VL CDR3 comprising the amino acid sequence set forth in SEQ ID NO: 64.

In another aspect, the recombinant antibody comprises a first antigen-binding portion comprising one of: (A) scFV comprising the amino acid sequence set forth in SEQ ID NO: 90; or (B) a VH comprising the amino acid sequence set forth in SEQ ID NO: 86 and a VL comprising the amino acid sequences set forth in SEQ ID NO: 88; or (C) a VH CDR1 comprising the amino acid sequence set forth in SEQ ID NO: 74; a VH CDR2 comprising the amino acid sequence set forth in SEQ ID NO: 75; a VH CDR3 comprising the amino acid sequence set forth in SEQ ID NO: 76; a VL CDR1 comprising the amino acid sequence set forth in SEQ ID NO: 80; a VL CDR2 comprising the amino acid sequence set forth in SEQ ID NO: 81; and a VL CDR3 comprising the amino acid sequence set forth in SEQ ID NO: 82.

In another aspect, the recombinant antibody comprises a first antigen-binding portion comprising one of: (A) scFV comprising the amino acid sequence set forth in SEQ ID NO: 108; or (B) a VH comprising the amino acid sequence set forth in SEQ ID NO: 104 and a VL comprising the amino acid sequence set forth in SEQ ID NO: 106; or (C) a VH CDR1 comprising the amino acid sequence set forth in SEQ ID NO: 92; a VH CDR2 comprising the amino acid sequence set forth in SEQ ID NO: 93; a VH CDR3 comprising the amino acid sequence set forth in SEQ ID NO: 94; a VL CDR1 comprising the amino acid sequence set forth in SEQ ID NO: 98; a VL CDR2 comprising the amino acid sequence set forth in SEQ ID NO: 99; and a VL CDR3 comprising the amino acid sequence set forth in SEQ ID NO: 100.

In another aspect, the recombinant antibody comprises a first antigen-binding portion comprising one of: (A) scFV comprising the amino acid sequence set forth in SEQ ID NO: 132; or (B) a VH comprising the amino acid sequence set forth in SEQ ID NO: 128 and a VL comprising the amino acid sequence set forth in SEQ ID NO: 130; or (C) a VH CDR1 comprising the amino acid sequence set forth in SEQ ID NO: 116; a VH CDR2 comprising the amino acid sequence set forth in SEQ ID NO: 117; a VH CDR3 comprising the amino acid sequence set forth in SEQ ID NO: 118; a VL CDR1 comprising the amino acid sequence set forth in SEQ ID NO: 122; a VL CDR2 comprising the amino acid sequence set forth in SEQ ID NO: 123; and a VL CDR3 comprising the amino acid sequence set forth in SEQ ID NO: 124.

In one aspect, a recombinant antibody according to the present disclosure comprises a first antigen-binding portion comprising a heavy chain variable region comprising CDR1, CDR2, and CDR3 from a VH sequence and a light chain variable region comprising CDR1, CDR2, and CDR3 from a VL sequence in any of Tables 1-6 or 8. For example, in one aspect, a recombinant antibody according to the present disclosure comprises a heavy chain variable region comprising CDR1, CDR2, and CDR3 from SEQ ID NO: 50 and a light chain variable region comprising CDR1, CDR2, and CDR3 from SEQ ID NO: 52. In another aspect, a recombinant antibody according to the present disclosure comprises a first antigen-binding portion comprising a heavy chain variable region comprising CDR1, CDR2, and CDR3 from a VH sequence in any of Tables 1-6 or 8 that is at least 90% identical to that VH sequence and/or comprises a light chain variable region comprising CDR1, CDR2, and CDR3 from a VL sequence in that same Table that is at least 90% identical to that VL sequence. For example, in one aspect, a recombinant antibody according to the present disclosure comprises a heavy chain variable region comprising CDR1, CDR2, and CDR3 from SEQ ID NO: 50 that is at least 90% identical to SEQ ID NO: 50 and/or comprises a light chain variable region comprising CDR1, CDR2, and CDR3 from SEQ ID NO: 52 that is at least 90% identical to SEQ ID NO: 52.

In another aspect, a recombinant antibody according to the present disclosure comprises a second antigen-binding portion comprising a heavy chain variable region comprising CDR1, CDR2, and CDR3 from a VH sequence and a light chain variable region comprising CDR1, CDR2, and CDR3 from a VL sequence in Table 7 or Table 9. For example, in one aspect, a recombinant antibody according to the present disclosure comprises a heavy chain variable region comprising CDR1, CDR2, and CDR3 from SEQ ID NO: 111 and a light chain variable region comprising CDR1, CDR2, and CDR3 from SEQ ID NO: 111. In another aspect, a recombinant antibody according to the present disclosure comprises a first antigen-binding portion comprising a heavy chain variable region comprising CDR1, CDR2, and CDR3 from a VH sequence in Table 7 or Table 9 that is at least 90% identical to that VH sequence and/or comprises a light chain variable region comprising CDR1, CDR2, and CDR3 from a VL sequence in that same Table that is at least 90% identical to that VL sequence. For example, in one aspect, a recombinant antibody according to the present disclosure comprises a heavy chain variable region comprising CDR1, CDR2, and CDR3 from SEQ ID NO: 112 that is at least 90% identical to SEQ ID NO: 112 and/or comprises a light chain variable region comprising CDR1, CDR2, and CDR3 from SEQ ID NO: 111 that is at least 90% identical to SEQ ID NO: 111.

In one aspect, the first antigen-binding portion and/or second antigen-binding portion of a bi-specific antibody of the present disclosure are scFvs. In one aspect, the bi-specific antibody according to the present invention comprises a first antigen-binding portion comprising a scFV comprising the amino acid sequence set forth in SEQ ID NO: 18, 36, 54, 72, 90, 108 or 132 and/or a second antigen-binding portion comprising a scFV comprising the amino acid sequence set forth in SEQ ID NO: 113. In another aspect, a bi-specific antibody according to the present invention comprises a first antigen-binding portion comprising the six CDRs (VH CDR1, CDR2, and CDR3 and VL CDR1, CDR2, and CDR3) from the amino acid sequence set forth in SEQ ID NO: 18, 36, 54, 72, 90, 108 or 132 and is at least 90% identical to that scFV sequence and/or comprises a second antigen-binding portion comprising the six CDRs (VH CDR1, CDR2, and CDR3 and VL CDR1, CDR2, and CDR3) from the amino acid sequence set forth in SEQ ID NO: 113 and is at least 90% identical to that scFV sequence.

In one aspect, the first antigen-binding portion of a recombinant bi-specific antibody of the present disclosure specifically binds to WT1 HLA and the second antigen-binding portion specifically binds to an antigen on the surface of an immune effector cell. In another aspect, the immune effector cell is selected from the group consisting of natural killer (NK) cells, macrophages, T cells, and combinations thereof. In another aspect, the immune effector cell is a CD3+ cell. In a further aspect, the recombinant antibody specifically binds to CD3. In one aspect, the recombinant antibody binds to a WT1-HLA2+ cell, for example, a WT1/HLA2+ cell having a low density of WT1/HLA2 on its surface.

In one aspect, the disclosure relates to a nucleic acid that encodes a recombinant antibody described herein. In a related aspect, a host cell is transformed with an expression vector comprising a nucleic acid of the present disclosure. In one aspect, the nucleic acid comprises a DNA sequence set forth in any of Tables 1-9.

In another aspect, the present disclosure relates to a pharmaceutical composition comprising a recombinant bi-specific antibody described herein and a physiologically acceptable diluent, excipient, or carrier. Optionally, the composition additionally comprises one or more physiologically active agents, for example, an anticancer agent, an adjuvant or other immune-stimulating substance, an anti-angiogenic substance, an analgesic substance, etc. In various particular embodiments, the composition comprises one, two, three, four, five, or six physiologically active agents in addition to a recombinant bi-specific antibody.

In one aspect, a pharmaceutical composition of the present disclosure comprises a recombinant antibody described herein with one or more substances selected from the group consisting of a buffer, an antioxidant such as ascorbic acid, a low molecular weight polypeptide (such as those having fewer than 10 amino acids), a protein, an amino acid, a carbohydrate such as glucose, sucrose or dextrin, a chelating agent such as EDTA, glutathione, a stabilizer, and an excipient. Neutral buffered saline or saline mixed with serum albumin are examples of appropriate diluents. In accordance with appropriate industry standards, preservatives such as benzyl alcohol may also be added. A liquid pharmaceutical composition may include, for example, one or more of the following: a sterile diluent such as water for injection, saline solution, preferably physiological saline, Ringer's solution, isotonic sodium chloride, fixed oils that may serve as the solvent or suspending medium, polyethylene glycols, glycerin, propylene glycol or other solvents; antibacterial agents; antioxidants; chelating agents; buffers and agents for the adjustment of tonicity such as sodium chloride or dextrose. A parenteral preparation can be enclosed in ampoules, disposable syringes or multiple dose vials made of glass or plastic. The use of physiological saline is preferred, and an injectable pharmaceutical composition is preferably sterile. In one aspect, the composition may be formulated as a lyophilizate using appropriate excipient solutions (e.g., sucrose) as diluents. Suitable components are nontoxic to recipients at the dosages and concentrations employed. Further examples of components that may be employed in pharmaceutical formulations are presented in Remington's Pharmaceutical Sciences, 16th Ed. (1980) and 20th Ed. (2000), Mack Publishing Company, Easton, Pa.

As is understood in the art, pharmaceutical compositions comprising the bi-specific antibodies of the present disclosure are administered to a subject in a manner appropriate to the indication. A pharmaceutical composition of the present disclosure comprising a recombinant antibody described herein may be formulated for delivery by any route that provides an effective dose of the bi-specific antibody. Pharmaceutical compositions may be administered by any suitable technique, including but not limited to parenterally, topically, or by inhalation. If injected, the pharmaceutical composition can be administered, for example, via intra-articular, intravenous, intramuscular, intralesional, intraperitoneal or subcutaneous routes, by bolus injection, or continuous infusion. Localized administration, e.g. at a tumor site is contemplated, as are transdermal delivery and sustained release from implants. Delivery by inhalation includes, for example, nasal or oral inhalation, use of a nebulizer, inhalation of the antagonist in aerosol form, and the like. Other alternatives include eyedrops; oral preparations including tablets, capsules, syrups, lozenges or chewing gum; and topical preparations such as lotions, gels, sprays, patches, and ointments.

In one aspect, the present disclosure relates to a method of killing a WT1-positive cell comprising contacting the cell with a recombinant bi-specific antibody described herein and an immune effector cell. The present disclosure also relates to a method of treatment of a subject having a WT1-positive disease comprising administering to the subject a therapeutically effective amount of a recombinant bi-specific antibody described herein. In one aspect, the method further comprises administering to the subject an immune effector cell. In one aspect, the immune effector cell is a cytotoxic cell, for example, a natural killer cell, macrophage, or T cell. In one aspect, the cytotoxic T cells is a CD3+ cytotoxic T cell, optionally, an autologous T cell. In one aspect, the WT1 positive disease is leukemia (chronic or acute) or a WT1-positive cancer. Examples of WT1 positive cancers include, but are not limited to, chronic myelocytic leukemia, multiple myeloma (MM), acute lymphoblastic leukemia (ALL), acute myeloid/myelogenous leukemia (AML), myelodysplastic syndrome (MDS), mesothelioma, ovarian cancer, gastrointestinal cancers, breast cancer, prostate cancer and glioblastoma.

The present disclosure thus relates to a method of treating a cancer comprising administering a therapeutically effective amount of a recombinant antibody or composition described herein to a patient in need thereof. In one aspect, the cancer is selected from the group consisting of adrenal cancer, acinic cell carcinoma, acoustic neuroma, acral lentigious melanoma, acrospiroma, acute eosinophilic leukemia, acute erythroid leukemia, acute lymphoblastic leukemia, acute megakaryoblastic leukemia, acute monocytic leukemia, acute promyelocytic leukemia, adenocarcinoma, adenoid cystic carcinoma, adenoma, adenomatoid odontogenic tumor, adenosquamous carcinoma, adipose tissue neoplasm, adrenocortical carcinoma, adult T-cell leukemia/lymphoma, aggressive NK-cell leukemia, AIDS-related lymphoma, alveolar rhabdomyosarcoma, alveolar soft part sarcoma, ameloblastic fibroma, anaplastic large cell lymphoma, anaplastic thyroid cancer, angioimmunoblastic T-cell lymphoma, angiomyolipoma, angiosarcoma, astrocytoma, atypical teratoid rhabdoid tumor, B-cell chronic lymphocytic leukemia, B-cell prolymphocytic leukemia, B-cell lymphoma, basal cell carcinoma, biliary tract cancer, bladder cancer, blastoma, bone cancer, Brenner tumor, Brown tumor, Burkitt's lymphoma, breast cancer, brain cancer, carcinoma, carcinoma in situ, carcinosarcoma, cartilage tumor, cementoma, myeloid sarcoma, chondroma, chordoma, choriocarcinoma, choroid plexus papilloma, clear-cell sarcoma of the kidney, craniopharyngioma, cutaneous T-cell lymphoma, cervical cancer, colon cancer, colorectal cancer, Degos disease, desmoplastic small round cell tumor, diffuse large B-cell lymphoma, dysembryoplastic neuroepithelial tumor, dysgerminoma, embryonal carcinoma, endocrine gland neoplasm, endodermal sinus tumor, enteropathy-associated T-cell lymphoma, esophageal cancer, Ewing's sarcoma, fetus in fetu, fibroma, fibrosarcoma, follicular lymphoma, follicular thyroid cancer, ganglioneuroma, gastrointestinal cancer, germ cell tumor, gestational choriocarcinoma, giant cell fibroblastoma, giant cell tumor of the bone, glial tumor, glioblastoma, glioma, gliomatosis cerebri, glucagonoma, gonadoblastoma, granulosa cell tumor, gynandroblastoma, gallbladder cancer, gastric cancer, hairy cell leukemia, hemangioblastoma, head and neck cancer, hemangiopericytoma, hematological malignancy, hepatoblastoma, hepatosplenic T-cell lymphoma, Hodgkin's lymphoma, non-Hodgkin's lymphoma, invasive lobular carcinoma, intestinal cancer, kidney cancer, laryngeal cancer, lentigo maligna, lethal midline carcinoma, leukemia, leydig cell tumor, liposarcoma, lung cancer, lymphangioma, lymphangiosarcoma, lymphoepithelioma, lymphoma, acute lymphocytic leukemia, acute myelogeous leukemia, chronic lymphocytic leukemia, liver cancer, small cell lung cancer, non-small cell carcinoma, non-small cell lung cancer, MALT lymphoma, malignant fibrous histiocytoma, malignant peripheral nerve sheath tumor, malignant triton tumor, mantle cell lymphoma, marginal zone B-cell lymphoma, mast cell leukemia, mediastinal germ cell tumor, medullary carcinoma of the breast, medullary thyroid cancer, medulloblastoma, melanoma, meningioma, merkel cell cancer, mesothelioma, metastatic urothelial carcinoma, mixed Mullerian tumor, mucinous tumor, multiple myeloma, muscle tissue neoplasm, mycosis fungoides, myxoid liposarcoma, myxoma, myxosarcoma, nasopharyngeal carcinoma, neurinoma, neuroblastoma, neurofibroma, neuroma, nodular melanoma, ocular cancer, oligoastrocytoma, oligodendroglioma, oncocytoma, optic nerve sheath meningioma, optic nerve tumor, oral cancer, osteosarcoma, ovarian cancer, Pancoast tumor, papillary thyroid cancer, paraganglioma, pinealoblastoma, pineocytoma, pituicytoma, pituitary adenoma, pituitary tumor, plasmacytoma, polyembryoma, precursor T-lymphoblastic lymphoma, primary central nervous system lymphoma, primary effusion lymphoma, preimary peritoneal cancer, prostate cancer, pancreatic cancer, pharyngeal cancer, pseudomyxoma periotonei, renal cell carcinoma, renal medullary carcinoma, retinoblastoma, rhabdomyoma, rhabdomyosarcoma, Richter's transformation, rectal cancer, sarcoma, Schwannomatosis, seminoma, Sertoli cell tumor, sex cord-gonadal stromal tumor, signet ring cell carcinoma, skin cancer, small blue round cell tumors, small cell carcinoma, soft tissue sarcoma, somatostatinoma, soot wart, spinal tumor, splenic marginal zone lymphoma, squamous cell carcinoma, synovial sarcoma, Sezary's disease, small intestine cancer, squamous carcinoma, stomach cancer, T-cell lymphoma, testicular cancer, thecoma, thyroid cancer, transitional cell carcinoma, throat cancer, urachal cancer, urogenital cancer, urothelial carcinoma, uveal melanoma, uterine cancer, verrucous carcinoma, visual pathway glioma, vulvar cancer, vaginal cancer, Waldenstrom's macroglobulinemia, Warthin's tumor, and Wilms tumor.

In another aspect, the disclosure relates to a method of stimulating a primary T cell response and a secondary T cell response in a subject comprising administering a composition comprising a recombinant antibody, said recombinant antibody comprising a first antigen-binding portion and second antigen-binding portion, wherein said first antigen-binding portion specifically binds to a first tumor antigen and said second antigen-binding portion specifically binds to an immune effector cell surface antigen, wherein the primary T cell response comprises stimulating cytotoxic T cells against the first tumor antigen, and wherein the secondary T cell response comprises stimulating effector T cells and/or memory T cells against the first tumor antigen and/or against a second tumor antigen. In one aspect, the first tumor antigen is WT1-HLA and the second tumor antigen is a non WT1/RMF tumor antigen. Examples of non-WT1/RMF tumor antigens include, but are not limited to, HER2-neu, mesothelin, Tert, Muc 16, Muc1, PSMA, and others known in the art (Cheever et al. Clinical cancer research : an official journal of the American Association for Cancer Research. 2009;15(17):5323-37). In one aspect, the primary T cell response and/or the secondary T cell response comprises an increase in T cells at a tumor site, for example, bone marrow, lung, liver, brain, genitourinary tract, gastrointestinal tract and/or spleen.

In one aspect, the secondary T cell response comprises stimulating effector T cells and/or memory T cells against the first tumor antigen and a second tumor antigen. In another aspect, the secondary T cell response comprises stimulating effector T cells against the second tumor antigen and not against the first tumor antigen. In one aspect, the secondary T cell response does not require antigen-presenting cells (i.e., cross-presentation) or co-stimulatory molecules such as CD86 or inducible costimulator ligand (ICOSL). In another aspect, the secondary T cell response comprises an increase in CD8 T cells. In a related aspect, the secondary T cell response is long-lived, e.g., lasting for more than one week, more than two weeks, more than three weeks, more than one month, or more than two months. In one aspect, the secondary T cell response comprises T cells that were previously anergic.

In one aspect, a method of stimulating a primary T cell response and a secondary T cell response according to the present disclosure comprises administering a composition comprising a recombinant bi-specific antibody described herein, for example, a recombinant bi-specific antibody comprising the amino acid sequence set forth in SEQ ID NO: 110. In another aspect, the method comprises administering a bi-specific antibody comprising a first antigen binding portion that specifically binds to WT1/HLA and the secondary T cell response comprises T cells previously activated with a non-WT1-RMF antigen.

In one aspect, the primary T cell response and/or secondary T cell response occurs *in vivo.* For example, a bi-specific antibody described herein can be administered to a patient in need thereof in an amount effective to stimulate a primary T cell response against WT1/HLA and a secondary T cell response against a non-WT1-RMF tumor antigen. In another aspect, the primary T cell response and/or secondary T cell response occurs ex *vivo.* For example, a bi-specific antibody according to the present disclosure can be administered to cells *in vitro* to activate and expand a population of T cells having specificity for a tumor antigen, e.g., a non-WT1-RMF tumor antigen. A therapeutically effective amount of the T cells can then be administered to a subject in need thereof in, e.g., autologously, to treat cancer.

Without intending to be bound by theory, the primary T cell and/or secondary T cell response may result from the ability of the bi-specific antibody to bring a TCR of a T cell close enough to a tumor cell to recognize additional MHC/peptide epitopes directly on the tumor. In one aspect, the binding of the first antigen binding portion of the bi-specific antibody to a first tumor antigen, e.g., WT1/HLA, during the T cell stimulation phase can block recognition of the first tumor antigen from the cognate TCR of the T cells, thereby resulting in a secondary T cell response specific for a tumor antigen other than the first tumor antigen.

The present disclosure therefore relates to a method of producing effector T cells and/or memory T cells against a tumor antigen comprising activating a T cell with the recombinant antibody described herein. In one aspect, said T cells produced following activation are viable for an extended period of time, e.g., at least one week, at least two weeks, at least three weeks, at least one month, or at least two months. In one aspect, the recombinant antibody comprises a first antigen binding portion that binds to WT1/HLA, but the effector T cells and/or memory T cells produced are against a non-WT1-RMF tumor antigen, for example, HER2-neu, mesothelin, Tert, Muc 16, Muc1, PSMA, and others known in the art. In one aspect, the effector T cells and/or memory T cells are produced *in vivo.* In another aspect, the effector T cells and/or memory T cells are produced *ex vivo*, for example, for use in adoptive T cell therapy. In one aspect, administering a bi-specific antibody increases production of CD8 T cells, resulting in long-lived effector memory. The bi-specific antibodies according to the present disclosure having a first binding portion specific for WT1/HLA are therefore capable of inducing a vaccinal response against non-WT1 tumor antigens, resulting in broad and effective anti-tumor therapy.

The methods of treatment of the present disclosure encompass alleviation or prevention of at least one symptom or other aspect of a disorder, or reduction of disease severity, and the like. In one aspect, a therapeutically effective amount of a recombinant bi-specific antibody or pharmaceutical composition of the invention is an amount effective to inhibit growth of WT1-positive cells, reduce tumor size/burden, prevent tumor cell metastasis/infiltration, and/or result in cell death, e.g., via apoptosis or necrosis. In another aspect, a method comprising administering a bi-specific antibody prophylactically, e.g., to induce a secondary T cell response and produce memory T cells, is effective to prevent onset or recurrence or reduce the severity of a disease. A bi-specific antibody or pharmaceutical composition described herein need not effect a complete cure, or eradicate every symptom or manifestation of a disease, to constitute a viable therapeutic agent. As is recognized in the art, therapeutic agents may reduce the severity of a given disease state, but need not abolish every manifestation of the disease to be regarded as useful. Similarly, a prophylactically administered treatment need not be completely effective in preventing the onset of a condition in order to constitute a viable prophylactic agent. Simply reducing the impact of a disease (for example, by reducing the number or severity of its symptoms, or by increasing the effectiveness of another treatment, or by producing another beneficial effect), or reducing the likelihood that the disease will occur or worsen in a subject, is sufficient.

Dosages and the frequency of administration for use in the methods of the present disclosure may vary according to such factors as the route of administration, the particular bi-specific antibodies employed, the nature and severity of the disease to be treated, whether the condition is acute or chronic, and the size and general condition of the subject. Appropriate dosages can be determined by procedures known in the pertinent art, e.g. in clinical trials that may involve dose escalation studies.

A recombinant bi-specific antibody of the present disclosure may be administered, for example, once or more than once, e.g., at regular intervals over a period of time. In general, the recombinant antibody or pharmaceutical composition is administered to a subject until the subject manifests a medically relevant degree of improvement over baseline for the chosen indicator or indicators.

In general, the amount of a recombinant bi-specific antibody described herein present in a dose, or produced *in situ* by an encoding polynucleotide present in a dose, ranges from about 10 µg per kg to about 20 mg per kg of host. The use of the minimum dosage that is sufficient to provide effective therapy is usually preferred. Patients may generally be monitored for therapeutic or prophylactic effectiveness using assays suitable for the condition being treated or prevented; assays will be familiar to those having ordinary skill in the art and some are described herein.

The methods disclosed herein may include oral administration of bi-specific antibody described herein or delivery by injection of a liquid pharmaceutical composition. When administered in a liquid form, suitable dose sizes will vary with the size of the subject, but will typically range from about 1 ml to about 500 ml (comprising from about 0.01 µg to about 1000 µg per kg) for a 10-60 kg subject. Optimal doses may generally be determined using experimental models and/or clinical trials. The optimal dose may depend upon the body mass, body area, weight, or blood volume of the subject. As described herein, the appropriate dose may also depend upon the patient's condition, that is, stage of the disease, general health status, age, gender, weight, and other factors familiar to a person skilled in the medical art.

In particular embodiments of the methods described herein, the subject is a human or non-human animal. A subject in need of the treatments described herein may exhibit symptoms or sequelae of a disease, disorder, or condition described herein or may be at risk of developing the disease, disorder, or condition. Non-human animals that may be treated include mammals, for example, non-human primates (e.g., monkey, chimpanzee, gorilla), rodents (e.g., rats, mice, gerbils, hamsters, ferrets, rabbits), lagomorphs, swine (e.g., pig, miniature pig), equine, canine, feline, bovine, and other domestic farm and zoo animals.

The present disclosure will be more readily understood by reference to the following Example, which is provided by way of illustration and are not intended to be limiting.

### Example

### Materials and Methods

**Cell samples, cell lines and antibodies.** Peripheral blood mononuclear cells (PBMCs) from HLA-typed healthy donors and patients were obtained by Ficoll density centrifugation. The sources for obtaining human leukemia and solid tumor cell lines were described previously (Dao et al., *supra*)*.* The cell lines for this study included: AML lines HL60, SET-2, Ph+ ALL line BV173, mesothelioma cell lines JMN and MSTO. All cells were HLA typed. The cell lines were cultured in RPMI 1640 supplemented with 5% FCS, penicillin, streptomycin, 2 mmol/L glutamine, and 2-mercaptoethanol at 37 C/5% CO₂. Tumor cells for all animal studies were transduced with GFP/luciferase as described previously (Dao et al., *supra*)*.* ESK1 and its control human IgG1 were produced by Eureka Therapeutics Inc (Emeryville, CA) and APC conjugation was done according to the instructions of the manufacture (Dao et al., *supra*)*.* Monoclonal antibody (mAb) against human HLA-A2 (clone BB7.2) conjugated to FITC or APC, and its isotype control mouse IgG2b/FITC or APC were purchased from BD Biosciences (San Diego, CA). Mouse anti-His tag mAb conjugated to FITC or PE and ELISA kits for human IFN-γ were purchased from Invitrogen, (NY). Renilla luciferase substrate ViviRen™ was purchased from Promega (Madison, WI).

**Peptides.** All peptides were purchased and synthesized by Genemed Synthesis, Inc. (San Antonio, TX). Amino acid sequences for HER2-neu-369-377: KIFGSLAFL (SEQ ID NO: 138); p53 264-272: LFEVRVCAC (SEQ ID NO: 139); WT1: RMFPNAPYL (SEQ ID NO: 1); Prame-300: ALYVDSLFFL (SEQ ID NO: 140); p435: NLTHVLYPV (SEQ ID NO: 141). WT1-NQM, AILDF, LDF and total pooled peptides were previously described (Doubrovina et al. Blood. 2012 Aug 23; 120(8): 1633-46). Control HLA-A2-binding peptide was derived from Ewing's sarcoma: QLQNPSYDK (SEQ ID NO: 142).

**Construction, expression and purification of ESK-Bi-specific antibody.** In one embodiment, an ESK1 bi-specific antibody is a single-chain bi-specific antibody comprising ESK1 scFv at the N-terminal end and an anti-human CD3ε scFv of a mouse monoclonal antibody at the C-terminal end (Figure 10). The DNA fragments coding for the ESK1 scFv antibody and the anti-human CD3ε scFv antibody were synthesized by GeneArt (Invitrogen) and subcloned into Eureka's mammalian expression vector pGSN-Hyg using standard DNA technology. A hexahistidine (His) tag was inserted downstream of the ESK1 bi-specific antibody at the C-terminal end for antibody purification and detection.

Chinese hamster ovary (CHO) cells were transfected with the ESK1 bi-specific antibody expression vector and stable expression was achieved by standard drug selection with methionine sulfoximine (MSX), a glutamine synthetase (GS)-based method (Fan, et al. Biotechnology Bioengineering. 109 (4), 1007-1005 (2012)). CHO cell supernatants containing secreted ESK1-bi-specific antibody molecules were collected. ESK1 bi-specific antibody was purified using HisTrap HP column (GE healthcare) by FPLC AKTA system. Briefly, CHO cell culture was clarified and loaded onto the column with low imidazole concentration (20 mM), and then an isocratic high imidazole concentration elution buffer (500 mM) was used to elute the bound ESK1 bi-specific antibody protein. A negative control bi-specific antibody, was constructed from an irrelevant human IgG1 antibody (Cat#ET901, Eureka Therapeutics) replacing ESK1 scFv.

**Flow cytometry analysis.** For ESK-bi-specific antibody staining, human T cells, tumor cells or cell lines were incubated with different concentrations of ESK- bi-specific antibody or control bi-specific antibody for 30 minutes on ice, washed, and incubated with secondary mAbs against His-Tag. HER2-neu expression on primary ovarian cancer cells was measured by staining the tumor cells with Trastuzumab, followed by secondary goat anti-human IgG. HLA-A2 expression and ESK binding was determined by direct staining of the cells with respective mAbs. Phenotype of PBMCs or T cells from patient samples were characterized by direct staining of the cells with mAbs for CD3, CD4, CD8, CD45 RA, CD45RO, CCR7, CD19 or CD33 conjugated to various florophores. Flow cytometry data were collected on a FACS Calibur (Becton Dickinson) and analyzed with FlowJo V8.7.1 and 9.4.8 software.

A binding study was performed to examine ESK1+ L2K bi-specific antibody binding to Jurkat (a CD3⁺ human cancer cell line) cells. Briefly, bi-specific antibody in 3x serial dilution, starting from 10 µg/mL was added to 0.5 x 10⁶ Jurkat cells. The cells were incubated with FITC conjugated anti-His tag antibody (Thermo #MA1-81891) at a 100x dilution or mouse anti-His tag antibody with or without allophycocyanin (APC)-anti mouse IgG (Biolegend # Poly4053) at a 1000x dilution and analyzed by flow cytometry on a Guava EasyCyte 6HT (EMD Millipore). The results are shown in Figure 8.

Next, using ESK1+ L2K, ET901 + L2K and ET901 +OKT3 antibodies at 10 µg/mL, staining of Jurkat cells by FITC anti-His was examined. FITC ET901 and APC ET901 (ET901 is a fully human IgG1) was used as the control. The results are shown in Figure 9.

A comparison of ESK1 and ESK1-bi-specific antibody binding to the MHC1/WT1 peptide complex was determined by ForteBio Octe analysis. The results are shown in the following table.

**Table 10**

| **Protein** | **k_{d}[1/s]** | **Error in k_{d}** | **kₐ [1/Ms]** | **K_{D} [nM]** |
|---|---|---|---|---|
| ESK1 ScFv | **1.16E-3** | 1.54E-5 | **3.68E3** | 315 |
| ESK1 BiTE | **7.04E-3** | 1.71E-4 | **1.98E4** | 355 |

**Full length ESK1-bi-specific antibody:** In one embodiment, the ESK-1 bi-specific antibody is a full length antibody. Briefly, ESK1 or ET901 (negative control) and anti-CD3 (OKT3) were combined in equimolar ratios, yielding a final concentration of 0.8 mg/ml of each antibody within 0.5ml. 12.5 µl of 1M 2-mercaptoethylamine (2-MEA) was added to the reaction mixture and the solution was incubated at 37°C for 90 minutes. 2-MEA was removed by Zeba desalting columns (100-200 µl, 7-kDA, Pierce.) The solution was stored at 4°C overnight to allow re-oxidation of the disulfide bonds. Production values are shown in Table 11.

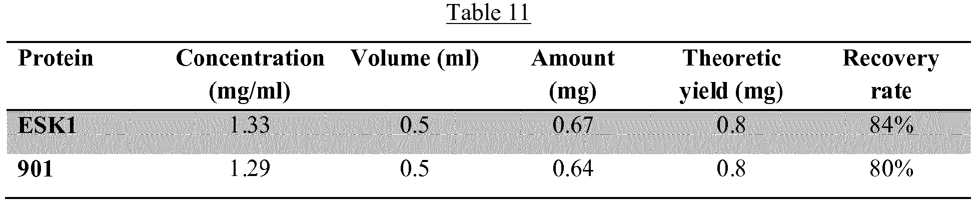

Binding affinity was determined using ForteBio Octet QK. 5 µg/ml biotinylated MHC-WT1 was loaded onto the Streptavidin biosensor. After washing off excess antigen, ESK-1-OKT3, ESK1, and OKT3 solutions were tested at 20 µg/ml, 10 µg/ml and 10 µg/ml respectively for their association and dissociation constants. Binding parameters were calculated using 1:1 binding site model and are shown in Table 12.

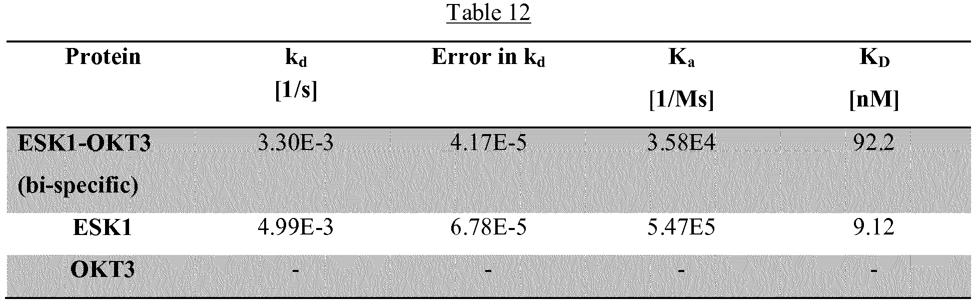

**ESK-Bi-specific antibody- mediated T cell activation.** CD3 T cells were isolated from PBMCs by negative immunomagnetic cell separation using a pan T cell isolation kit (Miltenyi Biotec Inc., San Diego, CA). The ESK-bi-specific antibody or its control bi-specific antibody at various concentrations were incubated with target cells and purified CD3 T cells at different effector: target (E:T) ratio for overnight or different time periods. The supernatant fluids were harvested and cytokine release was measured by ELISA for IFN-γ and TNF-α. In addition, ESK-bi-specific antibody-mediated T cell activation in the presence of autologous tumor cells from a patient with ovarian cancer was evaluated by cell proliferation, measured by overnight 3H-Thymidine incorporation after seven days of co-incubation.

**EBV-specific T cell expansion and reporter gene transduction.** T-cells were enriched from PBMCs by depletion of monocytes by adhesion. Non- adhering cells were stimulated with irradiated autologous EBV-transformed B cells (EBV-BLCLs) generated by transformation with the B95.8 strain of EBV at a 20:1 responder: stimulator (R: S) ratio and cultured in Yssel's medium, containing 5% HS(YH5; Gemini). Beginning on day 7, interleukin-2 (IL-2) at 10 to 30 units/mL was added to the T cell cultures every 2-3 days (Collaborative Biomedical Products, Bedford, MA), and were re-stimulated weekly with the same EBV-BLCLs at a 4:1 R:S ratio.

EBV specific T lymphocytes were transduced with retroviral vector tdrrsRLuc, expanded and enriched by sorting for PE, as previously described (Doubrovina, E. et al. Blood 119 (11), 2644-2655 (2012)). Transduced EBV specific T lymphocytes were cultured in G-rex flask (Wilson Wolf Manufacturing Corporation). For *in vivo* T cell tracing study, ten million cells were injected into mice and 4 hrs later, Renilla luciferase substrate ViviRen™ was given i.v..

**ESK-Bi-specific antibody- redirected T cell cytotoxicity.** The ESK-bi-specific antibody or its control bi-specific antibody at various concentrations were incubated with target cells and PBMCs, purified CD3 T cells or EBV-specific T cells at different effector: target (E:T) ratios for 5 hrs or overnight. The cytotoxicity was measured by ⁵¹Cr-release assay (after 5 hrs incubation) or LDH release assay (after overnight incubation) using Cytotox 96 non-radioreactive kit from Promega following their instruction. In one case of an AML patient, PBMCs and autologous blasts were co-incubated in the presence or absence of ESK or control-bi-specific antibody at 20 µg/ml and the cells were harvested and dual stained with CD33 for leukemia blasts and CD3 for T cells on day 3 and 4. For the case of an ovarian cancer patient, PBMCs were incubated with autologous ovarian cancer cells for a week and the cytotoxicity was measured by ⁵¹Cr-release assay.

**Pharmacokinetic and biodistribution studies.** ESK-bi-specific antibody or control bi-specific antibody were labeled with ¹²⁵I (PerkinElmer) using the chloramine-T method. One hundred (100) µg antibody was reacted with 1 mCi ¹²⁵I and 20µg chloramine-T, quenched with 200µg Na metabisulfite, then separated from free ¹²⁵I using a 10DG column equilibrated with 2% bovine serum albumin in PBS. Specific activity of the product was 6 mCi/mg. Radiolabeled bi-specific antibody (2 µg) was diluted with unlabeled bi-specific antibody to 20 µg per dose, and injected into mice retroorbitally. Blood was collected at various time points, weighed and measured on a gamma counter. At 24-hours, organs were harvested, weighed and measured for activity on a gamma counter.

**Therapeutic trials of the ESK-Bi-specific antibody in human tumor xenograft NSG models.** Human EBV-specific T cells were used for all xenograft models, as their antigenic specificity had been heavily biased towards EBV Ags, wherein they should not induce GVHD. For the BV173 ALL model, two million BV173 human leukemia cells were injected i.v. into NSG mice. On day 5, tumor engraftment was confirmed by firefly luciferase imaging in all mice that were to be treated; mice were then randomly divided into different treatment groups. On day 6, ten million of EBV-specific T cells were iv injected into mice. Four to 6 hours later, 20 µg ESK-bi-specific antibody or its control bi-specific antibody was i.v. injected and was repeated twice a week for total 6 times over the 3 weeks of treatment, along with i.v. injection of T cells once in a week times 2. For the SET-2 AML model, one million cells were i.v. injected into mice, the tumor engraftment was confirmed on day 3 by bioluminescence imaging, and mice were randomized into treatment groups. On day 4, ten million EBV-specific T cells were injected i.v. and 6 hrs later, 20 µg ESK-bi-specific antibody or its control bi-specific antibody was injected i.v. Over the treatment course, T cells were given twice a week and bi-specific antibodies were given every day for a total of 6 days. In the primary ALL model, five million ALL cells were injected i.v. into NSG mice. On day 6, tumor engraftment was confirmed by firefly luciferase imaging in all mice that were to be treated; mice were then randomly divided into different treatment groups. Thirty million EBV-specific T cells were injected i.v. into mice followed by injection i.v. of 20 µg ESK-bispecific antibody or its control bi-specific antibody. Bi-specific antibody injection was given daily, and T cells were given twice a week for a total of two weeks. For the JMN mesothelioma model, three hundred thousand tumor cells were mixed with six hundred thousand EBV-specific T cells and injected intraperitoneally (i.p.) and one hr later, 20 µg ESK-bi-specific antibody or control bi-specific antibody was i.v. injected into mice. The bi-specific antibodies were given every day for a total of 5 days. Tumor growth was monitored by firefly luciferase imaging at least twice a week, for all the animal models.

**ESK-bi-specific antibody induced secondary T cell response.** PBMCs, PBMCs depleted of NK cells and macrophages or purified CD3 T cells from a patient with ovarian cancer were cultured with irradiated (3000 rad) autologous ovarian cancer cells at an E: T ratio of 4-5:1, in the presence or absence of ESK-bi-specific antibody, or control- bi-specific antibody at 0.1 µg/ml, and presence of human IL-5 (5 ng/ml) and human IL-2 (10 µg/ml) in RPMI1640 medium supplemented with 10% autologous plasma (AP) for 6 days. On day 7, the cells were harvested and washed and used as effectors for IFN-g ELISPOT assay. In brief, HA-Multiscreen plates (Millipore) were coated with 100 µL of mouse anti-human IFN-g antibody (10 Ag/mL; clone 1-D1K; Mabtech) in PBS, incubated overnight at 4 °C, washed with PBS to remove unbound antibody, and blocked with RPMI 1640/10% autologous plasma (AP) for 2 h at 37 °C. Effectors cells were plated with either T2 cells (4:1 E: APC ratio) or irradiated autologous tumor cells or other tumor cell lines. Various test peptides were added to the wells at 20 µg/mL. Negative control wells contained APCs and T cells without peptides or with irrelevant peptides. Positive control wells contained T cells plus APCs plus 20 µg/mL phytohemagglutinin (PHA, Sigma). All conditions were done in triplicate. Microtiter plates were incubated for 20 h at 37 °C and then extensively washed with PBS/ 0.05% Tween and 100 µl/well biotinylated detection antibody against human IFN-g (2 µg/mL; clone 7-B6-1; Mabtech) was added. Plates were incubated for an additional 2 h at 37 °C and spot development was done as described (May et al. Clinical cancer research : an official journal of the American Association for Cancer Research. 2007;13(15 Pt 1):4547-55.). Spot numbers were automatically determined with the use of a computer-assisted video image analyzer with KS ELISPOT 4.0 software (Carl Zeiss Vision).

The remaining T cells were expanded by adding fresh medium with IL-15 and IL-2 once a week, up to 7-8 weeks. In some cases, remaining T cells were re-stimulated with autologous tumor at an effector: target ratio of 9:1 for a week in the same conditions as for the first stimulation and T cell response was measured by IFN-g ELISPOT as described.

### Results

**Selective binding to WT1+HLA-A0201+ tumor cells and T cells.** Full length ESK1 mAb binds to a panel of leukemia cell lines and mesothelioma cell lines in a WT1 and HLA-A0201-restricted manner (Dao et al., *supra*). The binding specificity of the ESK-bi-specific antibody, a scFv construct, was tested. ForteBio Octet® (Menlo Park, CA) binding assay showed a Kd of 355nM of the ESK-bi-specific antibody. ESK-bi-specific antibody bound to SET-2, a WT1 and HLA-A0201 double positive AML cell line, in a dose-dependent manner, even at a 0.1 µg/ml concentration, but did not bind to HL-60, a WT1 positive, but HLA-A0201 negative, AML cell line (Fig.1A, 1B). No positive binding of the control bi-specific antibody was seen to either SET-2 or HL-60 cells at all the concentrations tested, indicating the specificity of ESK-bi-specific antibody. Both bi-specific antibodies were able to bind to purified human CD3T cells, however (Fig 1C). These results demonstrated bi-specificity in that the ESK-bi-specific antibody was able to selectively recognize the tumor cells expressing WT1 and HLA-A0201 and also human CD3 T cells.

**T cell activation and cytotoxicity against WT1 and HLA-A0201 positive tumor cells.** It has been shown that the activation of T cells by bi-specific antibody constructs depends on the proximal contact between T cells and target cells expressing the target antigens. This is crucial for avoiding unwanted inflammatory response caused by T cell activation, as one arm of the bi-specific antibody construct recognizes the invariant CD3 signaling complex. In the presence of ESK-bi-specific antibody and target SET-2 cells, a dose-dependent IFN-γ release was observed at indicated concentrations of ESK-bi-specific antibody (Fig. 1D). CD3 T cells alone or incubated with control-bi-specific antibody in the presence of SET-2 cells showed undetectable level of IFN-γ and their values were subtracted from the values of the ESK-bi-specific antibody group.

The potency of ESK-bi-specific antibody-directed T cell cytotoxicity against target cells that co-express WT1 and HLA-A0201 was assessed. Purified resting T cells were co-incubated with target cells with serially diluted ESK-or control bi-specific antibody for 5 hrs and cell lysis was measured by ⁵¹Cr-release. ESK-bi-specific antibody dose-dependently induced T cell cytotoxicity against AML cell line SET-2, but not HL-60, which was consistent with their binding specificity shown in Fig. 1 (Fig. 2A, 2B). When the co-incubation time was extended to 16 hrs, the target cell lysis was increased up to 90% and 80% at 3 µg/ml and 0.3 µg/ml of ESK-bi-specific antibody, respectively. Similarly, BV173 and primary CML blasts (WT1+/HLA-A0201+) were also lysed by PBMCs in the presence of ESK-bi-specific antibody in a dose-dependent manner in a 6 hr co-incubation (Fig 2C, 2D), and the killing of BV173 was evident at 0.01 µg/ml of the ESK-bi-specific antibody. The relative weaker killing against the CML blasts might be due to a lower level expression of HLA-A2 and ESK-binding in this sample. Control bi-specific antibody did not induce any significant cytotoxicity, indicating that the target specificity is required for the T cell activation.

The ability of ESK-bi-specific antibody to redirect cytotoxicity of T cells that had previously been repeatedly primed with a different Ag, such as EBV, was addressed. Using such T cells could potentially avoid graft-versus-host disease (GVHD) in xenograft animal models, as the antigenic specificity of T cells should be heavily biased towards only EBV Ags. The potency of T cell killing by titration of the effector: target (E:T) ratios with ESK-bi-specific antibody at 0.1 µg/ml by 5 hr-51 Cr release was tested. Potent dose-dependent killing was observed for ESK-bi-specific antibody against all the target cells tested (Fig. 2E, 2F, 2G). There was nearly 50% killing at higher E:T ratios and approximately 25% killing at an E:T ratio as low as 1.6: 1 for BV173 (Fig. 2E). The specific killing was observed for JMN target cells, at an E: T ratio of 3.13 (Fig 2F). Similarly, strong cytotoxicity against primary ovarian cancer cells was found ranging from more than 50% to 20% killing at the various E: T ratios (Fig. 2G). There was no killing in the cultures with T cells alone, or with control bi-specific antibody against WT1+/HLA-A0201+ in the mesothelioma cell line JMN, ALL BV173, and the primary ovarian cancer cells (all co-express WT1 and HLA-A0201). These results demonstrated that ESK-bi-specific antibody can specifically redirect potent cytotoxicity of previously activated T cells with a specificity other than to WT1, to lyse tumor cells that are WT1 and HLA-A0201 positive.

Next, the ESK-bi-specific antibody-mediated activation and cytotoxicity of T cells in an autologous setting was investigated more closely, mimicking the human *in vivo* situation. PBMCs from a patient with ovarian cancer were stimulated with irradiated autologous tumor cells at various E:T ratios as indicated, in the presence of 0.1 µg/ml of ESK-bi-specific antibody for 7 days. The cytotoxicity was measured by adding ⁵¹Cr-labeled autologous tumor cells in a 6 hr-culture on day 8. Dose-dependent killing was observed in the cultures with ESK-bi-specific antibody, even at 100 PBMCs at the start of the stimulation (Fig. 3A). No killing was observed in all the control groups. In parallel, T cell proliferation was measured by 3H-thymidine incorporation at the end of eight day culture (Fig. 3B). Significant cell proliferation was seen in only the cultures with ESK-bi-specific antibody. None of the PBMCs, nor the tumor cells alone responded to either ESK- or control-bi-specific antibody. In addition, co-cultures of PBMCs with autologous tumor cells alone, or with control bi-specific antibody showed no T cell proliferation, suggesting T cells are tolerant to the self-tumor Ags. Together, these data demonstrated that specific activation of T cells by ESK-bi-specific antibody required the presence of tumor cells and that ESK-bi-specific antibody could force T cells to overcome the self-tolerance. The ability of the ESK-bi-specific antibody to induce autologous T cell proliferation and killing of its target cancer cells was illustrated in a second autologous model from a patient with AML (Fig. 16A-16D).

**Therapy of human leukemia expressing WT1/HLA-A0201 in NSG mice.** The *in vivo* therapeutic efficacy of ESK-bi-specific antibody in NSG mice xenografted intravenously 6 days previously with BV173 Ph+ALL cells was tested. At the time of treatment, mice had disseminated leukemia visible in their liver, spleen, and bone marrow. T cells were i.v. injected into mice and followed by bi-specific antibodies 4 hrs later. Dramatic tumor inhibition was observed, which was especially prominent in the bone marrow of mice that received ESK-bi-specific antibody, starting three days posttreatment and persisting up to 3 weeks (Fig. 4A). On the contrary, all mice in the control groups that included the mice received tumor cells, tumor along with T cells, tumor with ESK-bi-specific antibody, or tumor cells along with T cells and control bi-specific antibody, showed increasing tumor growth and massive tumor burdens in the bone marrows and other organs (Fig.4A). The average of photon intensity from five mice showed approximately 10-fold tumor reduction in the ESK-bi-specific antibody-treated group (Fig.4B). No significant GVHD was observed up to 49 days post-tumor inoculation (Fig. 4C). The results demonstrated that ESK-bi-specific antibody could efficiently engage and direct potent T cell cytotoxicity to kill BV173 target cells.

The therapeutic efficacy *in vivo* of ESK-bi-specific antibody in NSG mice xenografted intravenously with primary ALL cells was tested. T cells were injected i.v. into mice followed by ESK-bi-specific antibody injection. All mice in the control groups showed increasing tumor growth and massive tumor burdens in the bone marrows and other organs. Dramatic bi-specific antibody-selective tumor inhibition was observed, which was especially prominent in the bone marrow of mice, persisting more than 3 weeks, 9 days after treatment was stopped (Fig. 4D). The average of photon intensity from five mice showed approximately 10-20 fold tumor reduction in the ESK- bi-specific antibody -treated group than other control groups (Fig. 4E). No significant GVHD was observed clinically up to 49 days post-tumor inoculation.

These results demonstrated that ESK- bi-specific antibody could efficiently engage and redirect potent T cell cytotoxicity to kill primary leukemia cells. As expected, EBV-specific human T cells did not show non-specific killing and therefore, could be used to address bi-specific antibody activity in xenograft models. This model suggested, and was confirmed with a pharmacokinetic study, that twice a week administration of ESK-bi-specific antibody was not sufficient, as its half-life was only a few hours.

Since ESK-bi-specific antibody seemed to be more effective in treating bone marrow leukemia as shown in BV173 model, this effect in a more aggressive AML cells SET-2 model was investigated. SET-2 cells tend to migrate to bone marrow rapidly upon engraftment. Dosing and schedule was guided by pharmacokinetic studies that showed a short, 5-hour, beta plasma half-life of the ESK-bi-specific antibody (Fig. 17A). The treatment dosage was increased to daily bi-specific antibody injection for a total of 6 consecutive days. Cells were given twice a week. The mice received treatment starting on day 4 post-tumor inoculation, when the leukemia engraftment was confirmed by BLI (Fig. 5A, first row). SET-2 cells rapidly grow in bone marrow, and a massive infiltration of leukemia was seen in the bone marrow of mice that received SET-2 cells or SET-2 along with T cells. However, no detectable leukemia was seen in the ESK-bi-specific antibody-treated group up to 14 days and only a minimum leukemia burden was observed up to 18 days later at day 32, more than a week after treatment was stopped. The gain on the BLI scale was uniformly decreased for all groups for the images from day 7 to 18, in order to show low levels of leukemia in the pretreated time point. The control-bi-specific antibody group showed slightly delayed leukemia burden in the beginning. This could be caused by activation of EBV-specific T cells by anti-CD3 arm of the control-bi-specific antibody, which had stronger binding affinity for T cells than ESK-bi-specific antibody (Fig. 1C). EBV-specific T cells had already been activated and expanded multiple times in vitro, rendering them more susceptible to a strong polyclonal stimulation. Activated T cells then release inflammatory cytokines which could create a hostile environment for tumor cells. While all the mice in ESK-bi-specific antibody-treated group were still alive after a month, there was only 20% of survival in the groups of mice treated with T cells alone or no treatment (Fig. 5B). The mice in ESK-Bi-specific antibody-treated group showed no sign of central nerve system (CNS) paralysis, caused by leukemia infiltration into vertebral bone marrow for up to 40 days, whereas nearly all animals in the control group were affected. (Fig. 5C). These data demonstrated that ESK-bi-specific antibody is a potent therapeutic agent against aggressive leukemia cells in bone marrow.

**ESK-Bi-specific antibody mediates T cell retention at tumor sites.** While it is known that bi-specific antibodies effectively engage T cells to kill targets *in vitro,* no *in vivo* studies have been reported that document the mechanism *in vivo.* Whether the therapeutic efficacy of ESK-bi-specific antibody was the result of its ability to attract T cell retention and persistence at the sites of leukemia in live animals was investigated. Ten million EBV-specific T cells transduced with Renilla/luciferase were injected into NSG mice three days after GEF/luciferase positive SET-2 AML cells were engrafted, and 4 hrs later followed by bi-specific antibody injection. T cell migration was monitored by luminescence imaging at the time of T cell injection (0 hr), 8 (i.e., 4 hrs after bi-specific antibody injection), 24, 48 and 72 hrs. T cells migrated into the lungs immediately after the injection, then distributed into other parts including liver, spleen and bone marrow at 8 hrs (Fig. 6A). T cell signal gradually declined over 72 hrs. T cell injection followed by control bi-specific antibody showed a similar distribution pattern and time course as T cells alone. However, mice treated with ESK-bi-specific antibody showed significant increase in T cell signals in lungs and BM from 4-20 hrs, which lasted up to 72 hrs. Cells declined in the lungs substantially, but remained in the liver, spleen and BM. Quantitation of bio luminescence intensity showed that 4 hrs after the ESK-bi-specific antibody injection, there was approximately three-fold more T cell accumulation into the liver, spleen and bone marrow compared to the other two control groups (Fig. 6B). Monitoring the leukemia progression at the same time revealed an inversed correlation between T cell retention and leukemia burden (Fig. 6C). These results provided strong *in vivo* evidence that ESK-bi-specific antibody could mediate prolonged retention of T cells at the leukemia sites, and effectively directed T cell cytotoxicity to the tumor cells expressing WT1 and HLA-A0201.

**Therapy of mesothelioma in NSG mice.** Having shown the potent therapeutic activity of ESK-bi-specific antibody in two leukemia xenograft models, the efficacy of ESK-bi-specific antibody in treating aggressive solid tumor was investigated using an i.p. model to simulate peritoneal cavity mesothelioma. WT1+/HLA-A0201+ JMN mesothelioma cells were mixed with EBV-specific T cells at a target-effector ratio of 1:2 and were i.p. injected into NSG mice. Bi-specific antibodies were i.v. injected one hr later and was repeated for 5 consecutive days. Bioluminescence imaging one day later showed no visible tumor in the mice treated with ESK-bi-specific antibody, while mice in three controls groups showed visible tumor burden, suggesting that ESK-bi-specific antibody had eliminated tumor cells (Fig 7A). The tumor suppression by ESK-bi-specific antibody persisted until day 23; 18 days after the treatment was stopped, only a minimum tumor burden was seen in the mice. Averaging the bioluminescence intensity of five mice per group showed a persistent and approximately more than 20-fold tumor suppression on day 23 (Fig 7B).

**Pharmacokinetics:** To determine ESK bi-specific antibody pharmacokinetics, trace ¹²⁵I-labeled construct was injected either intravenously into C57 BL6/J, or intraperitoneally into BALB/c mice, and blood radioactivity was measured over 48-52 hours. Intravenously injected bi-specific antibody quickly redistributed from blood to tissue with an alpha half-life of 30 minutes, followed by clearance with a beta half-life of 5 hours. After intraperitoneal delivery, bi-specific antibody levels increased in the blood, peaking at 3 hours post-injection. The construct then cleared with the same beta half-life. Total exposure (Area Under the Curve) from intravenous injection was greater than with intraperitoneal injection. The biodistribution pattern of the antibodies was determined using the same radiolabeled constructs. After 24 hours, <1% injected dose / gram was detected in all tissues (Fig. 17A, 17B).

ESK-Bi-specific antibody induced secondary T cell response to a HER2 epitope in the context of HLA-A0201. The mechanism of the action for bi-specific antibodies has been attributed to their direct effect of bridging the cancer cell targets and T cells to form a cytolytic synapse. Whether such a proximal contact could directly activate pre-existing T cells in the population specific for other tumor antigens expressed by the autologous tumor cells was investigated to determine if a bi-specific antibody could exert a vaccinal effect using the autologous in vitro model from a HLA-A*02:01 positive patient with ovarian cancer. The patient's PBMCs were co-cultured with her autologous tumor cells in the presence of low-dose ESK-bi-specific antibody; a week later, the epitope-specific HLA-A*02:01-restricted T cell responses were assessed by IFN-g ELISPOT assay. Control groups including the cells alone or with control bi-specific antibody did not show any specific IFN-g release. The patient's tumor cells expressed both WT1 RMF and HER2-neu on their cell surface (Fig. 18A-C). Surprisingly, a strong secondary T cell response was induced against HER2-neu-369 epitope, the autologous tumor cells alone, and SET-2 AML cells, long after ESK-bi-specific mediated interactions were terminated (Fig. 19A-F). No response in these autologous cells was measurable against 56 pooled WT1-derived epitopes, WT1 epitopes RMF, AILDF05 or LDF; p53-derived epitope 264-273; Prame-300, Prame-435; Ewings sarcoma epitope EW, Muc1-15, -16, and-18; or HL60 cells. Since SET-2 does not express HER2-neu, and the T cells did not recognize WT1 RMF, the T cell response against SET-2 showed recognition of other yet to be defined epitopes that are neither WT1 nor HER2-neu. These results clearly demonstrated that ESK-bi-specific antibody could induce secondary T cell responses to multiple tumor antigens, thereby providing an unexpected vaccinal effect.

In principle, a secondary T cell response should require professional APCs among the PBMCs that could take up and present the intracellular antigens released after tumor cell death. Alternatively tumor cells could directly activate pre-existing epitope-specific T cells during the proximate contact between T cells and tumor. To clarify the mechanism, it was investigated whether the vaccinal effect could occur with purified T cells, and NK cell-depleted and macrophage-depleted PBMCs (T plus B cells) versus whole PBMCs as effector cells. Interestingly, T cells alone as effectors still were capable of the response to HER2-neu, autologous tumor cells and SET-2 cells, in a similar magnitude as that produced by T plus B cells (Fig. 19C) or whole PBMCs (Fig. 19D). To exclude a possibility of T cell presentation itself of these self-cancer antigens, purified T cells were co-cultured with autologous tumor cell lysates, generated by repetitive freeze thaw, in the presence or absence of ESK-bi-specific antibody. No T cell response was seen against the HER2-neu epitope or tumor cells in this setting (Figure 19D). These results demonstrated that neither professional APCs nor epitope tumors were required for the bi-specific antibody-induced secondary T cell response to HER2-neu or other epitopes present in tumor cells, therefore, cross-presentation was not the mechanism.

It was also examined if tumor cell antigen presentation depended on co-stimulatory molecules. While CD14+ monocytes from a healthy donor showed strong CD86 expression, ovarian cancer cells had little expression of the CD86. The results indicated that a key co-stimulatory molecule CD86 was unlikely to be involved in the tumor cell antigen presentation (Fig. 20). No CD86 or ICOSL expression was detected. Lack of co-stimulatory molecules is one of the reasons that tumor cells are poor antigen presenting cells; however, certain cytokines may provide co-stimulatory signals to fulfill the requirements for tumor specific T cell activation. ESK-bi-specific antibody induced IFN-gamma and TNF-alpha secretion by both PBMCs and T cells (Fig. 19E-F).

To test if ESK-bi-specific antibody had any long-term effect on the T cell population, T cells were expanded *in vitro* in low-dose IL-15 and IL-2, after the first activation by ESK-bi-specific antibody. While T cells in control groups did not survive more than one week in culture, T cells activated by ESK--bi-specific antibody continued to grow for 2 months, without further activation. Phenotype analysis revealed that seven weeks after activation by ESK--bi-specific antibody and tumor, CD8+ T cells increased to 89% of the population and CD4+ T cells decreased to 6% (Fig. 21A-B). Interestingly, among the CD8+/CCR7- cells, CD45RA- /CD45RO+ cells increased, indicative of effector memory phenotype. Importantly, even at this late time point, these T cells still retained their original specificity to autologous tumor and SET-2 AML cells (Figure 21C). These results indicated that ESK-Bi-specific antibody could induce a long-lasting secondary specific CD8 T cell responses.

### Discussion

The present disclosure relates to the first bi-specific antibody construct derived from a TCR-like mAb. The ESK-bi-specific antibody was specific for the WT1 RMF epitope presented by HLA-A0201 molecule. The ESK-bi-specific antibody showed a potent anti-tumor activity against multiple cancers, including ALL, AML, mesothelioma and primary ovarian cancer cells, both *in vitro* and *in vivo.* The study presented here is distinguished from previous studies, in that: 1) the mAb is a TCR-like mAb allowing recognition of an intracellular tumor-specific target; 2) the target density on tumor cells is extremely low, providing a proof-of-concept that could open the application of bi-specific antibodies to a much larger universe of targets; 3) the mechanisms of the ESK-bi-specific antibody activity *in vivo* with simultaneous dual effector cell and target cell tracing was demonstrated (previous bi-specific antibody studies have not shown this because in most animal studies, the effector T cells and target cells were mixed before injection; 4) Using an autologous system, it was demonstrated that the ESK-bi-specific antibody could break T cell tolerance to kill primary ovarian cancer cells; 5) the ESK-bi-specific antibody induced long-lived secondary, specific T cell responses against other tumor antigens, such as HER2-neu. Further, the secondary T cell responses were not mediated by cross-presentation effected by classical APCs, but rather was a result of the direct and physically close interaction between T cells and tumor cells, fostered by the bi-specific antibody.

All the bi-specific antibody constructs that are currently in development target cell surface proteins, such as CD19, CD33, prostate specific membrane antigen (PSMA) or epidermal growth factor (EGF) receptor, that are lineage or differentiation antigens found on normal cells. In contrast, the present disclosure demonstrates for the first time that a TCR-like bi-specific antibody construct is capable of recognizing a low density epitope of a peptide/MHC complex from a tumor-specific target. This argues against a traditional view that a scFv bi-specific antibody construct might not be suitable for targeting a low density Ag because the affinity would be too low or the ability to activate cytotoxicity by T cells would be insufficient. The present disclosure demonstrates for the first time that a TCR-mimic bi-specific antibody construct is capable of potent therapeutic activity in several mouse models of both hematopoietic and solid tumors, providing the proof of concept for vastly broadening the development of bi-specific antibodies targeting other cancer-specific intracellular tumor antigens. While it is known from assays *in vitro* that bi-specific antibodies bring T cells in contact with cancer cells, such a mechanism had not previously been observed *in vivo.* The present disclosure provides the proof of concept for future development of bi-specific mAbs targeting other intracellular tumor Ags. Thus, the ESK-bi-specific antibody described herein could broaden the therapeutic application of the platform.

In one embodiment, the ESK-bi-specific antibody induced fast and potent cytotoxic activity *in vitro* against lymphoid and myeoid leukemias, mesothelioma, and primary ovarian cancer cells, co-expressing WT1 and HLA-A0201. Lysis of the target cells by resting T cells or EBV-specific T cells could be detected in five hour co-incubation, compared to the studies with various other bi-specific antibody constructs that showed T cell killing after more than 16 hour of cultures. The cytolytic activity of ESK-bi-specific antibody could be further enhanced by longer period of incubation, shown by up to 80-90% of killing achieved with the SET-2 AML cells. This shared a similar feature with other bi-specific antibody mAbs specific for the CD33 and CD19, suggesting that ESK-bi-specific antibody engages T cell to targets in a serial fashion. IFN-g secretion further demonstrated the ESK-bi-specific antibody-mediated target-specific T cell activation. An important observation with clinical relevance is that ESK-bi-specific antibody could efficiently activate un-manipulated T cells from a patient with ovarian cancer to proliferate and kill the autologous tumor cells, suggesting that the presence of ESK-bi-specific antibody could force T cells to overcome the tolerance to self-tumor Ags. It has been known that T cell infiltration is frequently observed in solid tumors. The relatively low molecular weight of the bi-specific antibody would allow ESK-bi-specific antibody better penetration into solid tumors to activate anergic or unresponsive T cells to kill the surrounding tumor cells.

The *in vitro* cytotoxicity of ESK-bi-specific antibody was evidenced *in vivo* as well, as shown by its remarkably potent therapeutic activity against Ph1+ALL, AML and mesothelioma in xenograft models. In both leukemia models, elimination of tumor cells was evident, especially in bone marrow. In addition, ESK-bi-specific antibody prevented and delayed CNS paralysis, a characteristic feature of leukemia caused by infiltration of vertebral bone marrow.

The ability of the ESK-bi-specific antibody to kill leukemia cells *in vivo* when the T cells were injected separately from the tumor cells (a schedule not typically used in preclinical bi-specific antibody experiments), raised questions about the mechanism of bi-specific antibody activity *in vivo.* The study with SET-2 cells further raised the question of whether ESK-bi-specific antibody actively recruits and retains T cell effectors at the target. These questions were addressed by simultaneously tracing effector T cells and SET-2 AML cells using dual bioluminescence imaging. There was approximately a three-fold increase in T cells in the bone marrow of the mice treated with ESK-bi-specific antibody. The T cell retention was well correlated with the tumor reduction. The present disclosure provides direct *in vivo* evidence that ESK-bi-specific antibody could efficiently engage T cells with targets and kill the tumor cells in situ.

The therapeutic activity of ESK-bi-specific antibody in a solid tumor model with a peritoneal human mesothelioma in NSG mice was investigated. The PK study showed that i.v. injection of the ESK-bi-specific antibody gave a higher level in the blood stream than i.p. injection but that the plasma half-life of the bi-specific antibody was characteristically short. Rapid and dramatic tumor inhibition was seen in the treated mice, suggesting that ESK-bi-specific antibody gained quick access to the peritoneal tumor cells. The PK studies confirmed the pharmacokinetic observations from other bi-specific antibody constructs, suggesting that continuous administration of ESK-bi-specific antibody may be necessary to achieve the best therapeutic efficacy.

Similar to other bi-specific antibody constructs, a limitation of ESK-bi-specific antibody is also the lack of a natural Fc region thus preventing interaction with the neonatal FcRn receptor, which is necessary for delaying mAb clearance and for longer blood PK. Engineering an alternative bi-specific form of mAb with dual target specificities yet maintaining its natural architecture could offer a longer half-life and could significantly improve its efficacy in clinical application. Interestingly, it was observed that the full length mAb ESK1 was more effective in eradicating disseminated leukemia BV173 cells in lung and liver in mice, while ESK-bi-specific antibody seemed to be more effective in bone marrow. This may reflect the content and types of effectors found in different organs in the body (e.g., macrophages in liver and T cells in the blood and bone marrow). This discrepancy also suggests that combinations of full length IgG and bi-specific antibody forms used together might provide additive effects, if the appropriate dose and schedule could be determined. Therapeutic TCR in mAb development is relatively recent and there are many unanswered questions regarding their possible applications. The development of bi-specific mAb approaches for TCRm mAbs will allow broadening of their scope to include not only tumor-specific antigens, but also intracellular targets and other important ultra-low density targets.

The mechanisms of bi-specific antibody action to date have been attributed to a direct interaction between T cells and the mAb-specified cellular target. An intriguing discovery was that ESK-bi-specific antibody specific for WT1 RMF epitope induced a secondary T cell response highly specific for other cancer antigens (and not the WT1 target), such as HER2-neu epitope 369, in an autologous ovarian cancer model. It was shown that such proximate contact between T cells and target cells could directly reactivate (without cross-presentation) tolerant or anergic T cells that existed in the patient, to react with other cancer antigens. Surprisingly, a robust and long-lasting HER2-neu epitope specific T cell response was detected, as well as other cancer-specific reactivities. There was an increase in CD8 T cells with effector memory phenotype, further supporting the observation of new epitope-specific T cell responses, because CD8 T cells must have been activated to proliferate in response to the HLA-A^{*}02:01 restricted epitopes. The magnitude and duration of the single ESK-bi-specific antibody activation was far superior to the traditional peptide/APC-induced epitope-specific T cell response generally observed (Dao et al., *supra).* Interestingly, ESK-bi-specific antibody did not induce T cell responses against the original target epitope WT1-RMF, even though the patient had WT1 reactive T cells in her circulation as a consequence of prior adaptive cell therapy. These findings were consistent with the hypothesis that the bi-specific antibody is bringing the T cell's TCRs close to the tumor cell to recognize new MHC/peptide epitopes directly on the tumor and that the binding of the ESK-Bi-specific antibody to its peptide/MHC epitope during this T cell stimulation phase is blocking any recognition of the RMF/A0201 epitope from the cognate TCR of the T cells.

Expansion of T cells against various tumor antigens not present in the original vaccine have been detected in patients given vaccines or T cell infusions and has been associated with clinical responses; this phenomenon is referred to as "epitope spreading", and thought to be mediated by cross-presentation by APC's processing the apoptotic tumor cells (Corbiere, V. et al. Cancer Res. 71 (4), 1253-1262 (2011)). A vaccinal effect of mAb to CD20 has been reported; the effect also appears to be mediated by antibody mediated opsonization of the target cells into APCs (Hilchey et al. Blood 113 (16), 3809-3812 (2009)).

The discovery disclosed herein of a new mechanism of action for a bi-specific mAb may have particularly important implications for future therapy, as T cell infiltration in solid tumors is correlated with clinical responses. With its low molecular weight, bi-specific antibodies could penetrate into solid tumors to activate anergic or unresponsive T cells to kill tumor cells that express not only the known antigenic target of the bi-specific antibody, but also other undefined specific tumor antigens. The bi-specific antibody-mediated cytotoxic T cell clonal expansion for a variety of new epitopes should contribute greatly to its long-term therapeutic efficacy by preventing escape of cancer cell variants or low antigen density cells, as well as by promoting long-lived immunity. In addition, the vaccinal effect could be used for ex *vivo* expansion in advance of specific adoptive T cell therapy.

## Claims

1. A recombinant antibody comprising:
(I) a first antigen-binding portion which is capable of specifically binding to WT1/HLA, comprising one of:
(A) a single chain variable fragment (scFV) comprising an amino acid sequence selected from the group consisting of SEQ ID NOS: 54, 18, 36, 72, 90, 108 and 132; or
(B) a heavy chain variable domain (VH) and a light chain variable domain (VL), wherein the VH and VL, respectively, comprise amino acid sequences selected from the group consisting of SEQ ID NOS: (i) 50 and 52; (ii) 14 and 16; (iii) 32 and 34; (iv) 68 and 70; (v) 86 and 88; (vi) 104 and 106; and (vii) 128 and 130; or
(C)
(i) a VH CDR1 comprising the amino acid sequence set forth in SEQ ID NO: 38; a VH CDR2 comprising the amino acid sequence set forth in SEQ ID NO: 39; a VH CDR3 comprising the amino acid sequence set forth in SEQ ID NO: 40; a VL CDR1 comprising the amino acid sequence set forth in SEQ ID NO: 44; a VL CDR2 comprising the amino acid sequence set forth in SEQ ID NO: 45; and a VL CDR3 comprising the amino acid sequence set forth in SEQ ID NO: 46;
(ii) a VH CDR1 comprising the amino acid sequence set forth in SEQ ID NO: 2; a VH CDR2 comprising the amino acid sequence set forth in SEQ ID NO: 3; a VH CDR3 comprising the amino acid sequence set forth in SEQ ID NO: 4; a VL CDR1 comprising the amino acid sequence set forth in SEQ ID NO: 8; a VL CDR2 comprising the amino acid sequence set forth in SEQ ID NO: 9; and a VL CDR3 comprising the amino acid sequence set forth in SEQ ID NO: 10;
(iii) a VH CDR1 comprising the amino acid sequence set forth in SEQ ID NO: 20; a VH CDR2 comprising the amino acid sequence set forth in SEQ ID NO: 21 ; a VH CDR3 comprising the amino acid sequence set forth in SEQ ID NO: 22; a VL CDR1 comprising the amino acid sequence set forth in SEQ ID NO: 26; a VL CDR2 comprising the amino acid sequence set forth in SEQ ID NO: 27; and a VL CDR3 comprising the amino acid sequence set forth in SEQ ID NO: 28;
(iv) a VH CDR1 comprising the amino acid sequence set forth in SEQ ID NO: 56; a VH CDR2 comprising the amino acid sequence set forth in SEQ ID NO: 57; a VH CDR3 comprising the amino acid sequence set forth in SEQ ID NO: 58; a VL CDR1 comprising the amino acid sequence set forth in SEQ ID NO: 62; a VL CDR2 comprising the amino acid sequence set forth in SEQ ID NO: 63; and a VL CDR3 comprising the amino acid sequence set forth in SEQ ID NO: 64;
(v) a VH CDR1 comprising the amino acid sequence set forth in SEQ ID NO: 74; a VH CDR2 comprising the amino acid sequence set forth in SEQ ID NO: 75; a VH CDR3 comprising the amino acid sequence set forth in SEQ ID NO: 76; a VL CDR1 comprising the amino acid sequence set forth in SEQ ID NO: 80; a VL CDR2 comprising the amino acid sequence set forth in SEQ ID NO: 81 ; and a VL CDR3 comprising the amino acid sequence set forth in SEQ ID NO: 82; or
(vi) a VH CDR1 comprising the amino acid sequence set forth in SEQ ID NO: 92; a VH CDR2 comprising the amino acid sequence set forth in SEQ ID NO: 93; a VH CDR3 comprising the amino acid sequence set forth in SEQ ID NO: 94; a VL CDR1 comprising the amino acid sequence set forth in SEQ ID NO: 98; a VL CDR2 comprising the amino acid sequence set forth in SEQ ID NO: 99; and a VL CDR3 comprising the amino acid sequence set forth in SEQ ID NO: 100;
and
(II) a second antigen-binding portion comprising:
(A) a single chain variable fragment (scFV) comprising the amino acid sequence set forth in SEQ ID NO: 113; or
(B) a heavy chain variable domain (VH) and a light chain variable domain (VL), wherein the VH and VL, respectively, comprise the amino acid sequences set forth in SEQ ID NOS: 112 and 111 or a heavy chain and a light chain, wherein the heavy chain and light chain, respectively, comprise the amino acid sequences set forth in SEQ ID NOS: 134 and 136.

2. The recombinant antibody of claim 1, wherein:
(I) the first antigen-binding portion comprises one of:
(A) a scFV comprising the amino acid sequence set forth in SEQ ID NO: 18; or
(B) a VH comprising the amino acid sequence set forth in SEQ ID NO: 14 and a VL comprising the amino acid sequences set forth in SEQ I D NO: 16; or
(C) a VH CDR1 comprising the amino acid sequence set forth in SEQ ID NO: 2; a VH CDR2 comprising the amino acid sequence set forth in SEQ ID NO: 3; a VH CDR3 comprising the amino acid sequence set forth in SEQ ID NO: 4; a VL CDR1 comprising the amino acid sequence set forth in SEQ ID NO: 8; a VL CDR2 comprising the amino acid sequence set forth in SEQ ID NO: 9; and a VL CDR3 comprising the amino acid sequence set forth in SEQ ID NO: 10;
(II) the first antigen-binding portion comprises one of:
(A) a scFV comprising the amino acid sequence set forth in SEQ ID NO: 36; or
(B) a VH comprising the amino acid sequence set forth in SEQ ID NO: 32 and a VL comprising the amino acid sequence set forth in SEQ ID NO: 34; or
(C) a VH CDR1 comprising the amino acid sequence set forth in SEQ ID NO: 20; a VH CDR2 comprising the amino acid sequence set forth in SEQ ID NO: 21 ; a VH CDR3 comprising the amino acid sequence set forth in SEQ ID NO: 22; a VL CDR1 comprising the amino acid sequence set forth in SEQ ID NO: 26; a VL CDR2 comprising the amino acid sequence set forth in SEQ ID NO: 27; and a VL CDR3 comprising the amino acid sequence set forth in SEQ ID NO: 28;
(III) the first antigen-binding portion comprises one of:
(A) a scFV comprising the amino acid sequence set forth in SEQ ID NO: 54; or
(B) a VH comprising the amino acid sequence set forth in SEQ ID NO: 50 and a VL comprising the amino acid sequences set forth in SEQ ID NO: 52; or
(C) a VH CDR1 comprising the amino acid sequence set forth in SEQ ID NO: 38; a VH CDR2 comprising the amino acid sequence set forth in SEQ ID NO: 39; a VH CDR3 comprising the amino acid sequence set forth in SEQ ID NO: 40; a VL CDR1 comprising the amino acid sequence set forth in SEQ ID NO: 44; a VL CDR2 comprising the amino acid sequence set forth in SEQ ID NO: 45; and a VL CDR3 comprising the amino acid sequence set forth in SEQ ID NO: 46;
(IV) the first antigen-binding portion comprises one of:
(A) a scFV comprising the amino acid sequence set forth in SEQ ID NO: 72; or
(B) a VH comprising the amino acid sequence set forth in SEQ ID NO: 68 and a VL comprising the amino acid sequences set forth in SEQ I D NO: 70; or
(C) a VH CDR1 comprising the amino acid sequence set forth in SEQ ID NO: 56; a VH CDR2 comprising the amino acid sequence set forth in SEQ ID NO: 57; a VH CDR3 comprising the amino acid sequence set forth in SEQ ID NO: 58; a VL CDR1 comprising the amino acid sequence set forth in SEQ ID NO: 62; a VL CDR2 comprising the amino acid sequence set forth in SEQ ID NO: 63; and a VL CDR3 comprising the amino acid sequence set forth in SEQ ID NO: 64;
(V) the first antigen-binding portion comprises one of:
(A) a scFV comprising the amino acid sequence set forth in SEQ ID NO: 90; or
(B) a VH comprising the amino acid sequence set forth in SEQ ID NO: 86 and a VL comprising the amino acid sequences set forth in SEQ I D NO: 88; or
(C) a VH CDR1 comprising the amino acid sequence set forth in SEQ ID NO: 74; a VH CDR2 comprising the amino acid sequence set forth in SEQ ID NO: 75; a VH CDR3 comprising the amino acid sequence set forth in SEQ ID NO: 76; a VL CDR1 comprising the amino acid sequence set forth in SEQ ID NO: 80; a VL CDR2 comprising the amino acid sequence set forth in SEQ ID NO: 81 ; and a VL CDR3 comprising the amino acid sequence set forth in SEQ ID NO: 82; or
(VI) the first antigen-binding portion comprises one of:
(A) a scFV comprising the amino acid sequence set forth in SEQ ID NO: 108; or
(B) a VH comprising the amino acid sequence set forth in SEQ ID NO: 104 and a VL comprising the amino acid sequences set forth in SEQ I D NO: 106; or
(C) a VH CDR1 comprising the amino acid sequence set forth in SEQ ID NO: 92; a VH CDR2 comprising the amino acid sequence set forth in SEQ ID NO: 93; a VH CDR3 comprising the amino acid sequence set forth in SEQ ID NO: 94; a VL CDR1 comprising the amino acid sequence set forth in SEQ ID NO: 98; a VL CDR2 comprising the amino acid sequence set forth in SEQ ID NO: 99; and a VL CDR3 comprising the amino acid sequence set forth in SEQ ID NO: 100.

3. The recombinant antibody of any one of claims 1 or 2, wherein said first antigen-binding portion and/or said second antigen-binding portion is an antibody fragment selected from the group consisting of a Fab fragment; a monovalent fragment consisting of the VL, VH, CL and CH1 domains; a F(ab)2 fragment; a bivalent fragment comprising two Fab fragments linked by a disulfide bridge at the hinge region; a Fv fragment consisting of the VL and VH domains of a single arm of an antibody; and a scFv, preferably wherein said first antigen-binding portion and/or said second antigen-binding portion is a scFv.

4. The recombinant antibody of any one of claims 1-3, wherein said first antigen-binding portion specifically binds to WT1/HLA and said second antigen-binding portion specifically binds to an immune effector cell surface antigen, optionally wherein:
(a) said immune effector cell is selected from the group consisting natural killer (NK) cells, macrophages, and T cells; or
(b) said immune effector cell is a CD3⁺ cell and the recombinant antibody specifically binds to CD3.

5. The recombinant antibody of any of claims 1-4, wherein said recombinant antibody:
(a) binds to a WT1/HLA2⁺ cell, optionally wherein the WT1/HLA2⁺ cell has a low density of WT1/HLA2 on its surface; and
(b) specifically binds to CD3, optionally wherein said recombinant antibody binds to a CD3⁺ cell.

6. The recombinant antibody of any of claims 1-5, comprising the amino acid sequence set forth in SEQ ID NO: 110.

7. A nucleic acid that encodes a recombinant antibody of any of claims 1-6.

8. A pharmaceutical composition comprising the recombinant antibody of any of claims 1-6 or the nucleic acid of claim 7.

9. The antibody of any of claims 1-6 or the composition of claim 8 for use in a method for treating a WT1-positive disease in a subject wherein the WT1-positive disease is a chronic leukemia or acute leukemia or WT1⁺ cancer, optionally wherein the WT1 -positive disease is selected from the group consisting of chronic myelocytic leukemia, multiple myeloma (MM), acute lymphoblastic leukemia (ALL), acute myeloid/myelogenous leukemia (AML), myelodysplastic syndrome (MDS), mesothelioma, ovarian cancer, gastrointestinal cancers, breast cancer, prostate cancer and glioblastoma.

10. The antibody or composition for use according to claim 9, further comprising administering to the subject an immune effector cell, optionally wherein the immune effector cell is a cytotoxic cell, a CD3⁺ cytotoxic T cell or an autologous CD3⁺ cytotoxic T cell.

11. A recombinant antibody of any of claims 1-6 for use in a method for stimulating a primary T cell response and a secondary T cell response in a subject comprising administering a composition comprising the recombinant antibody according to any one of claims 1 to 6,
wherein the primary T cell response comprises stimulating cytotoxic T cells against the first tumor antigen, and
wherein the secondary T cell response comprises stimulating effector T cells and/or memory T cells against the first tumor antigen and/or against a second tumor antigen.

12. The antibody for use according to claim 11, wherein the first tumor antigen is WT1/HLA and the second tumor antigen is a non-WT1-RMF tumor antigen, optionally wherein the non-WT1-RMF tumor antigen is selected from the group consisting of HER2-neu, mesothelin, Tert, Muc 16, Muc 1, PSMA, and Pr1.

13. The antibody for use according to any of claims 11-12:
(I) wherein the secondary T cell response:
(a) does not require antigen presenting cells or co-stimulatory molecules;
(b) comprises stimulating effector T cells and/or memory T cells against the second tumor antigen;
(c) comprises an increase in CD8 T cells;
(d) lasts for more than one week; and/or
(e) comprises T cells that were previously anergic and/or T cells previously activated with a non-WT1-RMF antigen.

14. The antibody for use according to any of claims 11-13, wherein the primary T cell response and/or the secondary T cell response comprises an increase in T cells at a tumor site, optionally wherein the tumor site is selected from the group consisting of bone marrow, lung, liver, brain, genitourinary tract, gastrointestinal tract, and spleen.

## Patentansprüche

1. Rekombinanter Antikörper, umfassend:
(I) einen ersten, zur spezifischen Bindung an WT1/HLA fähigen antigenbindenden Teil, der eines der Folgenden umfasst:
(A) ein scFv-Fragment (scFv = Single Chain Variable Fragment) mit einer Aminosäuresequenz ausgewählt aus der aus SEQ ID NOS: 54, 18, 36, 72, 90, 108 und 132 bestehenden Gruppe, oder
(B) eine variable Schwerkettendomäne (VH) und eine variable Leichtkettendomäne (VL), wobei VH beziehungsweise VL Aminosäuresequenzen ausgewählt aus der aus SEQ ID NOS: (i) 50 und 52, (ii) 14 und 16, (iii) 32 und 34, (iv) 68 und 70, (v) 86 und 88, (vi) 104 und 106 und (vii) 128 und 130 bestehenden Gruppe umfassen, oder
(C)
(i) eine die in SEQ ID NO: 38 angegebene Aminosäuresequenz VH CDR1, eine die in SEQ ID NO: 39 angegebene Aminosäuresequenz VH CDR2, eine die in SEQ ID NO: 40 angegebene Aminosäuresequenz VH CDR3, eine die in SEQ ID NO: 44 angegebene Aminosäuresequenz umfassende VL CDR1, eine die in SEQ ID NO: 45 angegebene Aminosäuresequenz umfassende VL CDR2, und eine die in SEQ ID NO: 46 angegebene Aminosäuresequenz umfassende VL CDR3,
(ii) eine die in SEQ ID NO: 2 angegebene Aminosäuresequenz VH CDR1, eine die in SEQ ID NO: 3 angegebene Aminosäuresequenz VH CDR2, eine die in SEQ ID NO: 4 angegebene Aminosäuresequenz VH CDR3, eine die in SEQ ID NO: 8 angegebene Aminosäuresequenz umfassende VL CDR1, eine die in SEQ ID NO: 9 angegebene Aminosäuresequenz umfassende VL CDR2, und eine die in SEQ ID NO: 10 angegebene Aminosäuresequenz umfassende VL CDR3,
(iii) eine die in SEQ ID NO: 20 angegebene Aminosäuresequenz VH CDR1, eine die in SEQ ID NO: 21 angegebene Aminosäuresequenz VH CDR2, eine die in SEQ ID NO: 22 angegebene Aminosäuresequenz VH CDR3, eine die in SEQ ID NO: 26 angegebene Aminosäuresequenz umfassende VL CDR1, eine die in SEQ ID NO: 27 angegebene Aminosäuresequenz umfassende VL CDR2, und eine die in SEQ ID NO: 28 angegebene Aminosäuresequenz umfassende VL CDR3,
(iv) eine die in SEQ ID NO: 56 angegebene Aminosäuresequenz VH CDR1, eine die in SEQ ID NO: 57 angegebene Aminosäuresequenz VH CDR2, eine die in SEQ ID NO: 58 angegebene Aminosäuresequenz VH CDR3, eine die in SEQ ID NO: 62 angegebene Aminosäuresequenz umfassende VL CDR1, eine die in SEQ ID NO: 63 angegebene Aminosäuresequenz umfassende VL CDR2, und eine die in SEQ ID NO: 64 angegebene Aminosäuresequenz umfassende VL CDR3,
(v) eine die in SEQ ID NO: 74 angegebene Aminosäuresequenz VH CDR1, eine die in SEQ ID NO: 75 angegebene Aminosäuresequenz VH CDR2, eine die in SEQ ID NO: 76 angegebene Aminosäuresequenz VH CDR3, eine die in SEQ ID NO: 80 angegebene Aminosäuresequenz umfassende VL CDR1, eine die in SEQ ID NO: 81 angegebene Aminosäuresequenz umfassende VL CDR2, und eine die in SEQ ID NO: 82 angegebene Aminosäuresequenz umfassende VL CDR3,
(vi) eine die in SEQ ID NO: 92 angegebene Aminosäuresequenz VH CDR1, eine die in SEQ ID NO: 93 angegebene Aminosäuresequenz VH CDR2, eine die in SEQ ID NO: 94 angegebene Aminosäuresequenz VH CDR3, eine die in SEQ ID NO: 98 angegebene Aminosäuresequenz umfassende VL CDR1, eine die in SEQ ID NO: 99 angegebene Aminosäuresequenz umfassende VL CDR2, und eine die in SEQ ID NO: 100 angegebene Aminosäuresequenz umfassende VL CDR3,
und
(II) einen zweiten antigenbindenden Teil, umfassend:
(A) ein scFv-Fragment, umfassend die in SEQ ID NO: 113 angegebene Aminosäuresequenz, oder
(B) eine variable Schwerkettendomäne (VH) und eine variable Leichtkettendomäne (VL), wobei VH beziehungsweise VL die in SEQ ID NOS: 112 und 111 angegebenen Aminosäuresequenzen umfassen, oder eine schwere Kette und eine leichte Kette, wobei die schwere Kette beziehungsweise die leichte Kette die in SEQ ID NOS: 134 und 136 angegebenen Aminosäuresequenzen umfassen.

2. Rekombinanter Antikörper nach Anspruch 1, wobei:
(I) der erste antigenbindende Teil eines der Folgenden umfasst:
(A) eine die in SEQ ID NO: 18 angegebene Aminosäuresequenz umfassende scFv oder
(B) eine die in SEQ ID NO: 14 angegebene Aminosäuresequenz VH und eine die in SEQ ID NO: 16 angegebene Aminosäuresequenz umfassende VL oder
(C) eine die in SEQ ID NO: 2 angegebene Aminosäuresequenz umfassende VH CDR1, eine die in SEQ ID NO: 3 angegebene Aminosäuresequenz VH CDR2, eine die in SEQ ID NO: 4 angegebene Aminosäuresequenz VH CDR3, eine die in SEQ ID NO: 8 angegebene Aminosäuresequenz umfassende VL CDR1, eine die in SEQ ID NO: 9 angegebene Aminosäuresequenz umfassende VL CDR2 und eine die in SEQ ID NO: 10 angegebene Aminosäuresequenz umfassende VL CDR3,
(II) der erste antigenbindende Teil eines der Folgenden umfasst:
(A) eine die in SEQ ID NO: 36 angegebene Aminosäuresequenz umfassende scFv oder
(B) eine die in SEQ ID NO: 32 angegebene Aminosäuresequenz VH und eine die in SEQ ID NO: 34 angegebene Aminosäuresequenz umfassende VL oder
(C) eine die in SEQ ID NO: 20 angegebene Aminosäuresequenz umfassende VH CDR1, eine die in SEQ ID NO: 21 angegebene Aminosäuresequenz VH CDR2, eine die in SEQ ID NO: 22 angegebene Aminosäuresequenz VH CDR3, eine die in SEQ ID NO: 26 angegebene Aminosäuresequenz umfassende VL CDR1, eine die in SEQ ID NO: 27 angegebene Aminosäuresequenz umfassende VL CDR2 und eine die in SEQ ID NO: 28 angegebene Aminosäuresequenz umfassende VL CDR3,
(III) der erste antigenbindende Teil eines der Folgenden umfasst:
(A) eine die in SEQ ID NO: 54 angegebene Aminosäuresequenz umfassende scFv oder
(B) eine die in SEQ ID NO: 50 angegebene Aminosäuresequenz VH und eine die in SEQ ID NO: 52 angegebene Aminosäuresequenz umfassende VL oder
(C) eine die in SEQ ID NO: 38 angegebene Aminosäuresequenz umfassende VH CDR1, eine die in SEQ ID NO: 39 angegebene Aminosäuresequenz VH CDR2, eine die in SEQ ID NO: 40 angegebene Aminosäuresequenz VH CDR3, eine die in SEQ ID NO: 44 angegebene Aminosäuresequenz umfassende VL CDR1, eine die in SEQ ID NO: 45 angegebene Aminosäuresequenz umfassende VL CDR2 und eine die in SEQ ID NO: 46 angegebene Aminosäuresequenz umfassende VL CDR3,
(IV) der erste antigenbindende Teil eines der Folgenden umfasst:
(A) eine die in SEQ ID NO: 72 angegebene Aminosäuresequenz umfassende scFv oder
(B) eine die in SEQ ID NO: 68 angegebene Aminosäuresequenz VH und eine die in SEQ ID NO: 70 angegebene Aminosäuresequenz umfassende VL oder
(C) eine die in SEQ ID NO: 56 angegebene Aminosäuresequenz VH CDR1, eine die in SEQ ID NO: 57 angegebene Aminosäuresequenz VH CDR2, eine die in SEQ ID NO: 58 angegebene Aminosäuresequenz VH CDR3, eine die in SEQ ID NO: 62 angegebene Aminosäuresequenz umfassende VL CDR1, eine die in SEQ ID NO: 63 angegebene Aminosäuresequenz umfassende VL CDR2 und eine die in SEQ ID NO: 64 angegebene Aminosäuresequenz umfassende VL CDR3,
(V) der erste antigenbindende Teil eines der Folgenden umfasst:
(A) eine die in SEQ ID NO: 90 angegebene Aminosäuresequenz umfassende scFv oder
(B) eine die in SEQ ID NO: 86 angegebene Aminosäuresequenz VH und eine die in SEQ ID NO: 88 angegebene Aminosäuresequenz umfassende VL oder
(C) eine die in SEQ ID NO: 74 angegebene Aminosäuresequenz VH CDR1, eine die in SEQ ID NO: 75 angegebene Aminosäuresequenz VH CDR2, eine die in SEQ ID NO: 76 angegebene Aminosäuresequenz VH CDR3, eine die in SEQ ID NO: 80 angegebene Aminosäuresequenz umfassende VL CDR1, eine die in SEQ ID NO: 81 angegebene Aminosäuresequenz umfassende VL CDR2 und eine die in SEQ ID NO: 82 angegebene Aminosäuresequenz umfassende VL CDR3,
(VI) der erste antigenbindende Teil eines der Folgenden umfasst:
(A) eine die in SEQ ID NO: 108 angegebene Aminosäuresequenz umfassende scFv oder
(B) eine die in SEQ ID NO: 104 angegebene Aminosäuresequenz VH und eine die in SEQ ID NO: 106 angegebene Aminosäuresequenz umfassende VL oder
(C) eine die in SEQ ID NO: 92 angegebene Aminosäuresequenz VH CDR1, eine die in SEQ ID NO: 93 angegebene Aminosäuresequenz VH CDR2, eine die in SEQ ID NO: 94 angegebene Aminosäuresequenz VH CDR3, eine die in SEQ ID NO: 98 angegebene Aminosäuresequenz umfassende VL CDR1, eine die in SEQ ID NO: 99 angegebene Aminosäuresequenz umfassende VL CDR2 und eine die in SEQ ID NO: 100 angegebene Aminosäuresequenz umfassende VL CDR3.

3. Rekombinanter Antikörper nach Anspruch 1 oder 2, wobei es sich bei dem ersten antigenbindenden Teil und/oder dem zweiten antigenbindenden Teil um ein Antikörperfragment ausgewählt aus der Gruppe bestehend aus einem Fab-Fragment, einem einwertigen, aus den VL-, VH-, CL- und CH1-Domänen bestehenden Fragment, einem F (ab)2-Fragment, einem zweiwertigen, zwei an der Scharnierregion durch eine Disulfidbrücke verbundene Fab-Fragmente umfassenden Fragment, einem aus den VL- und VH-Domänen eines einzelnen Arms eines Antikörpers bestehenden Fv-Fragment und einem scFv-Fragment handelt, wobei es sich bei dem ersten antigenbindenden Teil und/oder dem zweiten antigenbindenden Teil vorzugsweise um ein scFv-Fragment handelt.

4. Rekombinanter Antikörper nach einem der Ansprüche 1-3, wobei der erste antigenbindende Teil spezifisch an WT1/HLA bindet und der zweite antigenbindende Teil spezifisch an ein Oberflächenantigen einer Immuneffektorzelle bindet, wobei gegebenenfalls:
(a) die Immuneffektorzelle aus der aus Natural-Killer(NK)-Zellen, Makrophagen und T-Zellen bestehenden Gruppe ausgewählt ist oder
(b) es sich bei der Immuneffektorzelle um eine CD3⁺-Zelle handelt und der rekombinante Antikörper spezifisch and CD3 bindet.

5. Rekombinanter Antikörper nach einem der Ansprüche 1-4, wobei der rekombinante Antikörper:
(a) an eine WT1/HLA2⁺-Zelle bindet, wobei die WT1/HLA2⁺-Zelle gegebenenfalls eine niedrige WT1/HLA2-Dichte auf ihrer Oberfläche hat, und
(b) spezifisch an CD3 bindet, wobei der rekombinante Antikörper gegebenenfalls an eine CD3⁺-Zelle bindet.

6. Rekombinanter Antikörper nach einem der Ansprüche 1-5, umfassend die in SEQ ID NO: 110 angegebene Aminosäuresequenz.

7. Nukleinsäure, die für einen rekombinanten Antikörper nach einem der Ansprüche 1-6 codiert.

8. Pharmazeutische Zusammensetzung, umfassend den rekombinanten Antikörper nach einem der Ansprüche 1-6 oder die Nukleinsäure nach Anspruch 7.

9. Antikörper nach einem der Ansprüche 1-6 oder Zusammensetzung nach Anspruch 8 zur Verwendung bei einem Verfahren zur Behandlung einer WT1-positiven Krankheit in einem Subjekt, wobei es sich bei der WT1-positiven Krankheit um eine chronische Leukämie oder akute Leukämie oder WT1⁺-Krebs handelt, wobei die WT1-positive Krankheit gegebenenfalls aus der aus chronischer myeloischer Leukämie, multiplem Myelom (MM), akuter lymphatischer Leukämie (ALL), akuter myeloischer/myelogener Leukämie (AML), myelodysplastischem Syndrom (MDS), Mesotheliom, Eierstockkrebs, Magen-Darm-Krebs, Brustkrebs, Prostatakrebs und Glioblastom bestehenden Gruppe ausgewählt ist.

10. Antikörper oder Zusammensetzung zur Verwendung nach Anspruch 9, weiterhin umfassend die Verabreichung einer Immuneffektorzelle an das Subjekt, wobei es sich bei der Immuneffektorzelle gegebenenfalls um eine zytotoxische Zelle, eine CD3⁺-zytotoxische T-Zelle oder eine autologe CD3⁺-zytotoxische T-Zelle handelt.

11. Rekombinanter Antikörper nach einem der Ansprüche 1-6 zur Verwendung in einem Verfahren zur Stimulierung einer primären T-Zell-Reaktion und einer sekundären T-Zell-Reaktion in einem Subjekt, umfassend die Verabreichung einer den rekombinanten Antikörper nach einem der Ansprüche 1 bis 6 umfassenden Zusammensetzung,
wobei die primäre T-Zell-Reaktion das Stimulieren zytotoxischer T-Zellen gegen das erste Tumorantigen umfasst und
wobei die sekundäre T-Zell-Reaktion das Stimulieren von Effektor-T-Zellen und/oder Gedächtnis-T-Zellen gegen das erste Tumorantigen und/oder gegen ein zweites Tumorantigen umfasst.

12. Antikörper zur Verwendung nach Anspruch 11, wobei es sich bei dem ersten Tumorantigen um WT1/HLA handelt und es sich bei dem zweiten Tumorantigen um ein Nicht-WT1-RMF-Tumorantigen handelt, wobei das Nicht-WT1-RMF-Tumorantigen gegebenenfalls aus der aus HER2-neu, Mesothelin, Tert, Muc 16, Muc 1, PSMA und Pr1 bestehenden Gruppe ausgewählt ist.

13. Antikörper zur Verwendung nach einem der Ansprüche 11-12:
(I) wobei die sekundäre T-Zell-Reaktion:
(a) keine Antigen präsentierenden Zellen oder kostimulierenden Moleküle erfordert,
(b) das Stimulieren von Effektor-T-Zellen und/oder Gedächtnis-T-Zellen gegen das zweite Tumorantigen umfasst,
(c) eine Zunahme an CD8-T-Zellen umfasst,
(d) mehr als eine Woche anhält und/oder
(e) T-Zellen umfasst, die zuvor anergisch waren, und/oder T-Zellen umfasst, die zuvor durch ein Nicht-WT1-RMF-Antigen aktiviert wurden.

14. Antikörper zur Verwendung nach einem der Ansprüche 11-13, wobei die primäre T-Zell-Reaktion und/oder die sekundäre T-Zell-Reaktion eine Zunahme an T-Zellen an einer Tumorstelle umfasst, wobei die Tumorstelle gegebenenfalls aus der aus Knochenmark, Lunge, Leber, Hirn, Urogenitaltrakt, Magen-Darm-Trakt und Milz bestehenden Gruppe ausgewählt ist.

## Revendications

1. Anticorps recombinant comprenant :
(I) une première partie se liant à l'antigène qui est apte à se lier spécifiquement à WT1/HLA, comprenant l'un des éléments suivants :
(A) un fragment variable de chaîne unique (scFV) comprenant une séquence d'acides aminés choisie dans le groupe constitué par les SEQ ID NOS: 54, 18, 36, 72, 90, 108 et 132 ; ou
(B) un domaine variable de chaîne lourde (VH) et un domaine variable de chaîne légère (VL), où le VH et le VL comprennent respectivement des séquences d'acides aminés choisies dans le groupe constitué par les SEQ ID NOS: (i) 50 et 52 ; (ii) 14 et 16 ; (iii) 32 et 34 ; (iv) 68 et 70 ; (v) 86 et 88 ; (vi) 104 et 106 ; et (vii) 128 et 130 ; ou
(C)
(i) une CDR1 de VH comprenant la séquence d'acides aminés décrite dans la SEQ ID NO: 38 ; une CDR2 de VH comprenant la séquence d'acides aminés décrite dans la SEQ ID NO: 39 ; une CDR3 de VH comprenant la séquence d'acides aminés décrite dans la SEQ ID NO: 40 ; une CDR1 de VL comprenant la séquence d'acides aminés décrite dans la SEQ ID NO: 44 ; une CDR2 de VL comprenant la séquence d'acides aminés décrite dans la SEQ ID NO: 45 ; et une CDR3 de VL comprenant la séquence d'acides aminés décrite dans la SEQ ID NO: 46 ;
(ii) une CDR1 de VH comprenant la séquence d'acides aminés décrite dans la SEQ ID NO: 2 ; une CDR2 de VH comprenant la séquence d'acides aminés décrite dans la SEQ ID NO: 3 ; une CDR3 de VH comprenant la séquence d'acides aminés décrite dans la SEQ ID NO: 4 ; une CDR1 de VL comprenant la séquence d'acides aminés décrite dans la SEQ ID NO: 8 ; une CDR2 de VL comprenant la séquence d'acides aminés décrite dans la SEQ ID NO: 9 ; et une CDR3 de VL comprenant la séquence d'acides aminés décrite dans la SEQ ID NO: 10;
(iii) une CDR1 de VH comprenant la séquence d'acides aminés décrite dans la SEQ ID NO: 20 ; une CDR2 de VH comprenant la séquence d'acides aminés décrite dans la SEQ ID NO: 21 ; une CDR3 de VH comprenant la séquence d'acides aminés décrite dans la SEQ ID NO: 22 ; une CDR1 de VL comprenant la séquence d'acides aminés décrite dans la SEQ ID NO: 26 ; une CDR2 de VL comprenant la séquence d'acides aminés décrite dans la SEQ ID NO: 27 ; et une CDR3 de VL comprenant la séquence d'acides aminés décrite dans la SEQ ID NO: 28;
(iv) une CDR1 de VH comprenant la séquence d'acides aminés décrite dans la SEQ ID NO: 56 ; une CDR2 de VH comprenant la séquence d'acides aminés décrite dans la SEQ ID NO: 57 ; une CDR3 de VH comprenant la séquence d'acides aminés décrite dans la SEQ ID NO: 58 ; une CDR1 de VL comprenant la séquence d'acides aminés décrite dans la SEQ ID NO: 62 ; une CDR2 de VL comprenant la séquence d'acides aminés décrite dans la SEQ ID NO: 63 ; et une CDR3 de VL comprenant la séquence d'acides aminés décrite dans la SEQ ID NO: 64;
(v) une CDR1 de VH comprenant la séquence d'acides aminés décrite dans la SEQ ID NO: 74 ; une CDR2 de VH comprenant la séquence d'acides aminés décrite dans la SEQ ID NO: 75 ; une CDR3 de VH comprenant la séquence d'acides aminés décrite dans la SEQ ID NO: 76 ; une CDR1 de VL comprenant la séquence d'acides aminés décrite dans la SEQ ID NO: 80 ; une CDR2 de VL comprenant la séquence d'acides aminés décrite dans la SEQ ID NO: 81 ; et une CDR3 de VL comprenant la séquence d'acides aminés décrite dans la SEQ ID NO: 82 ; ou
(vi) une CDR1 de VH comprenant la séquence d'acides aminés décrite dans la SEQ ID NO: 92 ; une CDR2 de VH comprenant la séquence d'acides aminés décrite dans la SEQ ID NO: 93 ; une CDR3 de VH comprenant la séquence d'acides aminés décrite dans la SEQ ID NO: 94 ; une CDR1 de VL comprenant la séquence d'acides aminés décrite dans la SEQ ID NO: 98 ; une CDR2 de VL comprenant la séquence d'acides aminés décrite dans la SEQ ID NO: 99 ; et une CDR3 de VL comprenant la séquence d'acides aminés décrite dans la SEQ ID NO: 100;
et
(II) une deuxième partie se liant à l'antigène comprenant :
(A) un fragment de chaîne variable unique (scFV) comprenant la séquence d'acides aminés décrite dans la SEQ ID NO: 113 ; ou
(B) un domaine variable de chaîne lourde (VH) et un domaine variable de chaîne légère (VL), où le VH et le VL comprennent respectivement des séquences d'acides aminés décrites dans les SEQ ID NOS: 112 et 111 ou une chaîne lourde et une chaîne légère, où la chaîne lourde et la chaîne légère comprennent respectivement les séquences d'acides aminés décrites dans les SEQ ID NOS: 134 et 136.

2. Anticorps recombinant selon la revendication 1, où :
(I) la première partie se liant à l'antigène comprend l'un des éléments suivants :
(A) un scFV comprenant la séquence d'acides aminés décrite dans la SEQ ID NO:
18 ; ou
(B) un VH comprenant la séquence d'acides aminés décrite dans la SEQ ID NO: 14 ; et un VL comprenant la séquence d'acides aminés décrite dans la SEQ ID NO: 16 ; ou
(C) une CDR1 de VH comprenant la séquence d'acides aminés décrite dans la SEQ ID NO: 2 ; une CDR2 de VH comprenant la séquence d'acides aminés décrite dans la SEQ ID NO: 3 ; une CDR3 de VH comprenant la séquence d'acides aminés décrite dans la SEQ ID NO: 4 ; une CDR1 de VL comprenant la séquence d'acides aminés décrite dans la SEQ ID NO: 8 ; une CDR2 de VL comprenant la séquence d'acides aminés décrite dans la SEQ ID NO: 9 ; et une CDR3 de VL comprenant la séquence d'acides aminés décrite dans la SEQ ID NO: 10;
(II) la première partie se liant à l'antigène comprend l'un des éléments suivants :
(A) un scFV comprenant la séquence d'acides aminés décrite dans la SEQ ID NO:
36 ; ou
(B) un VH comprenant la séquence d'acides aminés décrite dans la SEQ ID NO: 32 et un VL comprenant la séquence d'acides aminés décrite dans la SEQ ID NO: 34 ; ou
(C) une CDR1 de VH comprenant la séquence d'acides aminés décrite dans la SEQ ID NO: 20 ; une CDR2 de VH comprenant la séquence d'acides aminés décrite dans la SEQ ID NO: 21 ; une CDR3 de VH comprenant la séquence d'acides aminés décrite dans la SEQ ID NO: 22 ; une CDR1 de VL comprenant la séquence d'acides aminés décrite dans la SEQ ID NO: 26 ; une CDR2 de VL comprenant la séquence d'acides aminés décrite dans la SEQ ID NO: 27 ; et une CDR3 de VL comprenant la séquence d'acides aminés décrite dans la SEQ ID NO: 28;
(III) la première partie se liant à l'antigène comprend l'un des éléments suivants :
(A) un scFV comprenant la séquence d'acides aminés décrite dans la SEQ ID NO:
54 ; ou
(B) un VH comprenant la séquence d'acides aminés décrite dans la SEQ ID NO: 50 et un VL comprenant les séquences d'acides aminés décrites dans la SEQ ID NO: 52 ; ou
(C) une CDR1 de VH comprenant la séquence d'acides aminés décrite dans la SEQ ID NO: 38 ; une CDR2 de VH comprenant la séquence d'acides aminés décrite dans la SEQ ID NO: 39 ; une CDR3 de VH comprenant la séquence d'acides aminés décrite dans la SEQ ID NO: 40 ; une CDR1 de VL comprenant la séquence d'acides aminés décrite dans la SEQ ID NO: 44 ; une CDR2 de VL comprenant la séquence d'acides aminés décrite dans la SEQ ID NO: 45 ; et une CDR3 de VL comprenant la séquence d'acides aminés décrite dans la SEQ ID NO: 46 ;
(IV) la première partie se liant à l'antigène comprend l'un des éléments suivants :
(A) un scFV comprenant la séquence d'acides aminés décrite dans la SEQ ID NO:
72 ; ou
(B) un VH comprenant la séquence d'acides aminés décrite dans la SEQ ID NO: 68 et un VL comprenant les séquences d'acides aminés décrites dans la SEQ ID NO: 70 ; ou
(C) une CDR1 de VH comprenant la séquence d'acides aminés décrite dans la SEQ ID NO: 56 ; une CDR2 de VH comprenant la séquence d'acides aminés décrite dans la SEQ ID NO: 57 ; une CDR3 de VH comprenant la séquence d'acides aminés décrite dans la SEQ ID NO: 58 ; une CDR1 de VL comprenant la séquence d'acides aminés décrite dans la SEQ ID NO: 62 ; une CDR2 de VL comprenant la séquence d'acides aminés décrite dans la SEQ ID NO: 63 ; et une CDR3 de VL comprenant la séquence d'acides aminés décrite dans la SEQ ID NO: 64 ;
(V) la première partie se liant à l'antigène comprend l'un des éléments suivants :
(A) un scFV comprenant la séquence d'acides aminés décrite dans la SEQ ID NO:
90 ; ou
(B) un VH comprenant la séquence d'acides aminés décrite dans la SEQ ID NO: 86 et un VL comprenant les séquences d'acides aminés décrites dans la SEQ ID NO: 88 ; ou
(C) une CDR1 de VH comprenant la séquence d'acides aminés décrite dans la SEQ ID NO: 74 ; une CDR2 de VH comprenant la séquence d'acides aminés décrite dans la SEQ ID NO: 75 ; une CDR3 de VH comprenant la séquence d'acides aminés décrite dans la SEQ ID NO: 76 ; une CDR1 de VL comprenant la séquence d'acides aminés décrite dans la SEQ ID NO: 80 ; une CDR2 de VL comprenant la séquence d'acides aminés décrite dans la SEQ ID NO: 81 ; et une CDR3 de VL comprenant la séquence d'acides aminés décrite dans la SEQ ID NO: 82 ; ou
(VI) la première partie se liant à l'antigène comprend l'un des éléments suivants :
(A) un scFV comprenant la séquence d'acides aminés décrite dans la SEQ ID NO: 108 ; ou
(B) un VH comprenant la séquence d'acides aminés décrite dans la SEQ ID NO: 104 et un VL comprenant les séquences d'acides aminés décrites dans la SEQ ID NO: 106 ; ou
(C) une CDR1 de VH comprenant la séquence d'acides aminés décrite dans la SEQ ID NO: 92 ; une CDR2 de VH comprenant la séquence d'acides aminés décrite dans la SEQ ID NO: 93 ; une CDR3 de VH comprenant la séquence d'acides aminés décrite dans la SEQ ID NO: 94 ; une CDR1 de VL comprenant la séquence d'acides aminés décrite dans la SEQ ID NO: 98 ; une CDR2 de VL comprenant la séquence d'acides aminés décrite dans la SEQ ID NO: 99 ; et une CDR3 de VL comprenant la séquence d'acides aminés décrite dans la SEQ ID NO: 100.

3. Anticorps recombinant selon l'une quelconque des revendications 1 ou 2, où ladite première partie se liant à l'antigène et/ou ladite deuxième partie se liant à l'antigène sont un fragment d'anticorps choisi dans le groupe constitué par un fragment Fab ; un fragment monovalent constitué par les domaines VL, VH, CL et CH1 ; un fragment F(ab)2 ; un fragment divalent comprenant deux fragments Fab liés par un pont disulfure au niveau de la région charnière ; un fragment Fv constitué par les domaines VL et VH d'un bras unique d'un anticorps ; et un scFv, préférentiellement où ladite première partie se liant à l'antigène et/ou ladite deuxième partie se liant à l'antigène sont un scFV.

4. Anticorps recombinant selon l'une quelconque des revendications 1 à 3, où ladite première partie se liant à l'antigène se lie spécifiquement à WT1/HLA et ladite deuxième partie se liant à l'antigène se lie spécifiquement à un antigène de surface de cellule effectrice immunitaire, éventuellement où :
(a) ladite cellule effectrice immunitaire est choisie dans le groupe constitué par les lymphocytes NK, les macrophages et les lymphocytes T ; ou
(b) ladite cellule effectrice immunitaire est une cellule CD3⁺ et l'anticorps recombinant se lie spécifiquement à CD3.

5. Anticorps recombinant selon l'une quelconque des revendications 1 à 4, où ledit anticorps recombinant :
(a) se lie à une cellule WT1/HLA2⁺, éventuellement où la cellule WT1/HLA2⁺ présente une faible densité de WT1/HLA2 à sa surface ; et
(b) se lie spécifiquement à CD3, éventuellement où ledit anticorps recombinant se lie à une cellule CD3⁺.

6. Anticorps recombinant selon l'une quelconque des revendications 1 à 5, comprenant la séquence d'acides aminés décrite dans la SEQ ID NO: 110.

7. Acide nucléique qui code un anticorps recombinant selon l'une quelconque des revendications 1 à 6.

8. Composition pharmaceutique comprenant l'anticorps recombinant selon l'une quelconque des revendications 1 à 6 ou l'acide nucléique selon la revendication 7.

9. Anticorps selon l'une quelconque des revendications 1 à 6 ou composition selon la revendication 8 pour utilisation dans une méthode de traitement d'une maladie WT1-positive chez un sujet, où la maladie WT1-positive est une leucémie chronique, une leucémie aiguë ou un cancer à WT1⁺, éventuellement où la maladie WT1-positive est choisie dans le groupe constitué par les suivantes : leucémie myéloïde chronique, myélome multiple (MM), leucémie aiguë lymphoblastique (LAL), leucémie myéloïde/myélogène aiguë (LMA), syndrome myélodysplasique (MDS), mésothéliome, cancer de l'ovaire, cancers gastro-intestinaux, cancer du sein, cancer de la prostate et glioblastome.

10. Anticorps ou composition pour utilisation selon la revendication 9, comprenant en outre l'administration au sujet d'une cellule effectrice immunitaire, éventuellement où la cellule effectrice immunitaire est une cellule cytotoxique, un lymphocyte T cytotoxique CD3⁺ ou un lymphocyte T cytotoxique CD3⁺ autologue.

11. Anticorps recombinant selon l'une quelconque des revendications 1 à 6 pour utilisation dans une méthode de stimulation d'une réponse primaire des lymphocytes T et d'une réponse secondaire des lymphocytes T chez un sujet comprenant l'administration d'une composition comprenant l'anticorps recombinant selon l'une quelconque des revendications 1 à 6,
où la réponse primaire des lymphocytes T comprend la stimulation de lymphocytes T cytotoxiques contre le premier antigène tumoral, et
où la réponse secondaire des lymphocytes T comprend la stimulation des lymphocytes T effecteurs et/ou des lymphocytes T à mémoire contre le premier antigène tumoral et/ou contre un deuxième antigène tumoral.

12. Anticorps pour utilisation selon la revendication 11, où le premier antigène tumoral est WT1/HLA et le deuxième antigène tumoral est un antigène tumoral autre que WT1-RMF, éventuellement où l'antigène tumoral autre que WT1-RMF est choisi dans le groupe constitué par HER2-neu, mésothéline, Tert, Muc 16, Muc 1, PSMA et Pr1.

13. Anticorps pour utilisation selon l'une quelconque des revendications 11 à 12 :
(I) où la réponse secondaire des lymphocytes T :
(a) n'exige pas de cellules présentant l'antigène ou de molécules co-simulatrices ;
(b) comprend des lymphocytes T effecteurs stimulants et/ou des lymphocytes T à mémoire contre le deuxième antigène tumoral ;
(c) comprend une augmentation des lymphocytes T CD8 ;
(d) dure plus d'une semaine ; et/ou
(e) comprend des lymphocytes T qui étaient précédemment anergiques et/ou des lymphocytes T précédemment activés par un antigène autre que WT1-RMF.

14. Anticorps pour utilisation selon l'une quelconque des revendications 11 à 13, où la réponse primaire des lymphocytes T et/ou la réponse secondaire des lymphocytes T comprend une augmentation des lymphocytes T au niveau d'un site tumoral, éventuellement où le site tumoral est choisi dans le groupe constitué par les suivants : moelle osseuse, poumon, foie, cerveau, appareil urogénital, tractus gastro-intestinal et rate.


### SEQUENCE LISTING

<110> Memorial Sloan-Kettering Cancer Center Eureka Therapeutics, Inc. SCHEINBERG, et al.
<120> ANTI-WT1/HLA BI-SPECIFIC ANTIBODY
<130> 27527/48317 PCT
<150> US 61/901,310
   <151> 2013-11-07
<150> US 62/037,875
   <151> 2014-08-15
<160> 142
<170> PatentIn version 3.5
<210> 1
   <211> 9
   <212> PRT
   <213> Artificial Sequence
<220>
   <223> Synthetic Polypeptide
<400> 1
   Arg Met Phe Pro Asn Ala Pro Tyr Leu
   1               5
<210> 2
   <211> 10
   <212> PRT
   <213> Artificial Sequence
<220>
   <223> Synthetic Polypeptide
<400> 2
   Gly Gly Thr Phe Ser Ser Tyr Ala Ile Ser
   1               5                   10
<210> 3
   <211> 17
   <212> PRT
   <213> Artificial Sequence
<220>
   <223> Synthetic Polypeptide
<400> 3
   Gly Ile Ile Pro Ile Phe Gly Thr Ala Asn Tyr Ala Gln Lys Phe Gln
   1               5                   10                  15
   Gly
<210> 4
   <211> 10
   <212> PRT
   <213> Artificial Sequence
<220>
   <223> Synthetic Polypeptide
<400> 4
   Arg Ile Pro Pro Tyr Tyr Gly Met Asp Val
   1 5 10
<210> 5
   <211> 30
   <212> DNA
   <213> Artificial Sequence
<220>
   <223> Synthetic Polynucleotide
<400> 5
   ggaggcacct tcagcagcta tgctatcagc 30
<210> 6
   <211> 51
   <212> DNA
   <213> Artificial Sequence
<220>
   <223> Synthetic Polynucleotide
<400> 6
   gggatcatcc ctatctttgg tacagcaaac tacgcacaga agttccaggg c 51
<210> 7
   <211> 30
   <212> DNA
   <213> Artificial Sequence
<220>
   <223> Synthetic Polynucleotidee
<400> 7
   cggattcccc cgtactacgg tatggacgtc 30
<210> 8
   <211> 13
   <212> PRT
   <213> Artificial Sequence
<220>
   <223> Synthetic Polypeptide
<400> 8
   Ser Gly Ser Ser Ser Asn Ile Gly Ser Asn Tyr Val Tyr
   1 5 10
<210> 9
   <211> 7
   <212> PRT
   <213> Artificial Sequence
<220>
   <223> Synthetic Polypeptide
<400> 9
   Arg Ser Asn Gln Arg Pro Ser
   1               5
<210> 10
   <211> 11
   <212> PRT
   <213> Artificial Sequence
<220>
   <223> Synthetic Polypeptide
<400> 10
   Ala Ala Trp Asp Asp Ser Leu Asn Gly Val Val
   1               5                   10
<210> 11
   <211> 39
   <212> DNA
   <213> Artificial Sequence
<220>
   <223> Synthetic Polynucleotide
<400> 11
   tctggaagca gctccaacat cggaagtaat tatgtatac 39
<210> 12
   <211> 21
   <212> DNA
   <213> Artificial Sequence
<220>
   <223> Synthetic Polynucleotide
<400> 12
   aggagtaatc agcggccctc a 21
<210> 13
   <211> 33
   <212> DNA
   <213> Artificial Sequence
<220>
   <223> Synthetic Polynucleotide
<400> 13
   gcagcatggg atgacagcct gaatggtgtg gta 33
<210> 14
   <211> 119
   <212> PRT
   <213> Artificial Sequence
<220>
   <223> Synthetic Polypeptide
<400> 14
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Arg Ile Pro Pro Tyr Tyr Gly Met Asp Val Trp Gly Gln Gly
               100                 105                 110
   Thr Thr Val Thr Val Ser Ser
           115
<210> 15
   <211> 357
   <212> DNA
   <213> Artificial Sequence
<220>
   <223> Synthetic Polynucleotide
<400> 15
   caggtgcagc tggtgcagtc tggggctgag gtgaagaagc ctgggtcctc ggtgaaggtc       60
   tcctgcaagg cttctggagg caccttcagc agctatgcta tcagctgggt gcgacaggcc      120
   cctggacaag ggcttgagtg gatgggaggg atcatcccta tctttggtac agcaaactac      180
   gcacagaagt tccagggcag agtcacgatt accgcggacg aatccacgag cacagcctac      240
   atggagctga gcagcctgag atctgaggac acggccgtgt attactgtgc gagacggatt      300
   cccccgtact acggtatgga cgtctggggc caagggacca cggtcaccgt ctcctca         357
<210> 16
   <211> 111
   <212> PRT
   <213> Artificial Sequence
<220>
   <223> Synthetic Polypeptide
<400> 16
   Gln Thr Val Val Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Tyr Val Tyr Trp Tyr Gln Gln Leu Pro Gly Thr Ala Pro Lys Leu Leu
           35                  40                  45
   Ile Tyr Arg Ser Asn Gln Arg Pro Ser Gly Val Pro Asp Arg Phe Ser
       50                  55                  60
   Gly Ser Lys Ser Gly Thr Ser Ala Ser Leu Ala Ile Ser Gly Pro Arg
   65                  70                  75                  80
   Ser Val Asp Glu Ala Asp Tyr Tyr Cys Ala Ala Trp Asp Asp Ser Leu
                   85                  90                  95
   Asn Gly Val Val Phe Gly Gly Gly Thr Lys Leu Thr Val Leu Gly
               100                 105                 110
<210> 17
   <211> 333
   <212> DNA
   <213> Artificial Sequence
<220>
   <223> Synthetic Polynucleotide
<400> 17
   cagactgtgg tgactcagcc accctcagcg tctgggaccc ccgggcagag ggtcaccatc 60
   tcttgttctg gaagcagctc caacatcgga agtaattatg tatactggta ccaacagctc 120
   ccaggaacgg cccccaaact cctcatctat aggagtaatc agcggccctc aggggtccct 180
   gaccgattct ctggctccaa gtctggcacc tcagcctccc tggccatcag tgggccccgg 240
   tccgtggatg aggctgatta ttactgtgca gcatgggatg acagcctgaa tggtgtggta 300
   ttcggcggag ggaccaagct gaccgtccta ggt 333
<210> 18
   <211> 250
   <212> PRT
   <213> Artificial Sequence
<220>
   <223> Synthetic Polypeptide
<400> 18
   Gln Thr Val Val Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Tyr Val Tyr Trp Tyr Gln Gln Leu Pro Gly Thr Ala Pro Lys Leu Leu
           35                  40                  45
   Ile Tyr Arg Ser Asn Gln Arg Pro Ser Gly Val Pro Asp Arg Phe Ser
       50                  55                  60
   Gly Ser Lys Ser Gly Thr Ser Ala Ser Leu Ala Ile Ser Gly Pro Arg
   65                  70                  75                  80
   Ser Val Asp Glu Ala Asp Tyr Tyr Cys Ala Ala Trp Asp Asp Ser Leu
                   85                  90                  95
   Asn Gly Val Val Phe Gly Gly Gly Thr Lys Leu Thr Val Leu Gly Ser
               100                 105                 110
   Arg Gly Gly Gly Gly Ser Gly Gly Gly Gly Ser Gly Gly Gly Ser Leu
           115                 120                 125
   Glu Met Ala Gln Val Gln Leu Val Gln Ser Gly Ala Glu Val Lys Lys
       130                 135                 140
   Pro Gly Ser Ser Val Lys Val Ser Cys Lys Ala Ser Gly Gly Thr Phe
   145                 150                 155                 160
   Ser Ser Tyr Ala Ile Ser Trp Val Arg Gln Ala Pro Gly Gln Gly Leu
                   165                 170                 175
   Glu Trp Met Gly Gly Ile Ile Pro Ile Phe Gly Thr Ala Asn Tyr Ala
               180                 185                 190
   Gln Lys Phe Gln Gly Arg Val Thr Ile Thr Ala Asp Glu Ser Thr Ser
           195                 200                 205
   Thr Ala Tyr Met Glu Leu Ser Ser Leu Arg Ser Glu Asp Thr Ala Val
       210                 215                 220
   Tyr Tyr Cys Ala Arg Arg Ile Pro Pro Tyr Tyr Gly Met Asp Val Trp
   225                 230                 235                 240
   Gly Gln Gly Thr Thr Val Thr Val Ser Ser
                   245                 250
<210> 19
   <211> 750
   <212> DNA
   <213> Artificial Sequence
<220>
   <223> Synthetic Polynucleotide
<400> 19
   cagactgtgg tgactcagcc accctcagcg tctgggaccc ccgggcagag ggtcaccatc      60
   tcttgttctg gaagcagctc caacatcgga agtaattatg tatactggta ccaacagctc     120
   ccaggaacgg cccccaaact cctcatctat aggagtaatc agcggccctc aggggtccct     180
   gaccgattct ctggctccaa gtctggcacc tcagcctccc tggccatcag tgggccccgg     240
   tccgtggatg aggctgatta ttactgtgca gcatgggatg acagcctgaa tggtgtggta     300
   ttcggcggag ggaccaagct gaccgtccta ggttctagag gtggtggtgg tagcggcggc     360
   ggcggctctg gtggtggatc cctcgagatg gcccaggtgc agctggtgca gtctggggct     420
   gaggtgaaga agcctgggtc ctcggtgaag gtctcctgca aggcttctgg aggcaccttc     480
   agcagctatg ctatcagctg ggtgcgacag gcccctggac aagggcttga gtggatggga     540
   gggatcatcc ctatctttgg tacagcaaac tacgcacaga agttccaggg cagagtcacg     600
   attaccgcgg acgaatccac gagcacagcc tacatggagc tgagcagcct gagatctgag     660
   gacacggccg tgtattactg tgcgagacgg attcccccgt actacggtat ggacgtctgg     720
   ggccaaggga ccacggtcac cgtctcctca                                      750
<210> 20
   <211> 12
   <212> PRT
   <213> Artificial Sequence
<220>
   <223> Synthetic Polypeptide
<400> 20
   Gly Asp Ser Val Ser Ser Asn Ser Ala Ala Trp Asn
   1               5                   10
<210> 21
   <211> 18
   <212> PRT
   <213> Artificial Sequence
<220>
   <223> Synthetic Polypeptide
<400> 21
   Arg Thr Tyr Tyr Gly Ser Lys Trp Tyr Asn Asp Tyr Ala Val Ser Val
   1               5                   10                  15
   Lys Ser
<210> 22
   <211> 9
   <212> PRT
   <213> Artificial Sequence
<220>
   <223> Synthetic Polypeptide
<400> 22
   Gly Arg Leu Gly Asp Ala Phe Asp Ile
   1               5
<210> 23
   <211> 36
   <212> DNA
   <213> Artificial Sequence
<220>
   <223> Synthetic Polynucleotide
<400> 23
   ggggacagtg tctctagcaa cagtgctgct tggaac 36
<210> 24
   <211> 54
   <212> DNA
   <213> Artificial Sequence
<220>
   <223> Synthetic Polynucleotide
<400> 24
   aggacatact acgggtccaa gtggtataat gattatgcag tatctgtgaa aagt 54
<210> 25
   <211> 27
   <212> DNA
   <213> Artificial Sequence
<220>
   <223> Synthetic Polynucleotide
<400> 25
   ggtcgcttag gggatgcttt tgatatc 27
<210> 26
   <211> 11
   <212> PRT
   <213> Artificial Sequence
<220>
   <223> Synthetic Polypeptide
<400> 26
   Arg Ala Ser Gln Ser Ile Ser Ser Tyr Leu Asn
   1               5                   10
<210> 27
   <211> 7
   <212> PRT
   <213> Artificial Sequence
<220>
   <223> Synthetic Polypeptide
<400> 27
   Ala Ala Ser Ser Leu Gln Ser
   1               5
<210> 28
   <211> 9
   <212> PRT
   <213> Artificial Sequence
<220>
   <223> Synthetic Polypeptide
<400> 28
   Gln Gln Ser Tyr Ser Thr Pro Leu Thr
   1               5
<210> 29
   <211> 33
   <212> DNA
   <213> Artificial Sequence
<220>
   <223> Synthetic Polynucleotide
<400> 29
   cgggcaagtc agagcattag cagctattta aat 33
<210> 30
   <211> 21
   <212> DNA
   <213> Artificial Sequence
<220>
   <223> Synthetic Polynucleotide
<400> 30
   gctgcatcca gtttgcaaag t 21
<210> 31
   <211> 27
   <212> DNA
   <213> Artificial Sequence
<220>
   <223> Synthetic Polynucleotide
<400> 31
   caacagagtt acagtacccc tctcact 27
<210> 32
   <211> 121
   <212> PRT
   <213> Artificial Sequence
<220>
   <223> Synthetic Polypeptide
<400> 32
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Asn Trp Ile Arg Gln Ser Pro Ser Arg Gly Leu Glu
           35                  40                  45
   Trp Leu Gly Arg Thr Tyr Tyr Gly Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Gly Arg Leu Gly Asp Ala Phe Asp Ile Trp Gly
               100                 105                 110
   Gln Gly Thr Met Val Thr Val Ser Ser
           115                 120
<210> 33
   <211> 363
   <212> DNA
   <213> Artificial Sequence
<220>
   <223> Synthetic Polynucleotide
<400> 33
   caggtacagc tgcagcagtc aggtccagga ctggtgaagc cctcgcagac cctctcactc        60
   acctgtgcca tctccgggga cagtgtctct agcaacagtg ctgcttggaa ctggatcagg       120
   cagtccccat cgagaggcct tgagtggctg ggaaggacat actacgggtc caagtggtat       180
   aatgattatg cagtatctgt gaaaagtcga ataaccatca acccagacac atccaagaac       240
   cagttctccc tgcagctgaa ctctgtgact cccgaggaca cggctgtgta ttactgtgca       300
   agaggtcgct taggggatgc ttttgatatc tggggccaag ggacaatggt caccgtctct       360
   tca                                                                     363
<210> 34
   <211> 108
   <212> PRT
   <213> Artificial Sequence
<220>
   <223> Synthetic Polypeptide
<400> 34
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Leu
                   85                  90                  95
   Thr Phe Gly Gly Gly Thr Lys Val Asp Ile Lys Arg
               100                 105
<210> 35
   <211> 324
   <212> DNA
   <213> Artificial Sequence
<220>
   <223> Synthetic Polynucleotide
<400> 35
   gacatccaga tgacccagtc tccatcctcc ctgtctgcat ctgtaggaga cagagtcacc 60
   atcacttgcc gggcaagtca gagcattagc agctatttaa attggtatca gcagaaacca 120
   gggaaagccc ctaagctcct gatctatgct gcatccagtt tgcaaagtgg ggtcccatca 180
   aggttcagtg gcagtggatc tgggacagat ttcactctca ccatcagcag tctgcaacct 240
   gaagattttg caacttacta ctgtcaacag agttacagta cccctctcac tttcggcgga 300
   gggaccaaag tggatatcaa acgt 324
<210> 36
   <211> 250
   <212> PRT
   <213> Artificial Sequence
<220>
   <223> Synthetic Polypeptide
<400> 36
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Tyr Ser Thr Pro Leu
   85 90 95
   Thr Phe Gly Gly Gly Thr Lys Val Asp Ile Lys Arg Ser Arg Gly Gly
   100 105 110
   Gly Gly Ser Gly Gly Gly Gly Ser Gly Gly Gly Gly Ser Leu Glu Met
   115 120 125
   Ala Gln Val Gln Leu Gln Gln Ser Gly Pro Gly Leu Val Lys Pro Ser
       130                 135                 140
   Gln Thr Leu Ser Leu Thr Cys Ala Ile Ser Gly Asp Ser Val Ser Ser
   145                 150                 155                 160
   Asn Ser Ala Ala Trp Asn Trp Ile Arg Gln Ser Pro Ser Arg Gly Leu
                   165                 170                 175
   Glu Trp Leu Gly Arg Thr Tyr Tyr Gly Ser Lys Trp Tyr Asn Asp Tyr
               180                 185                 190
   Ala Val Ser Val Lys Ser Arg Ile Thr Ile Asn Pro Asp Thr Ser Lys
           195                 200                 205
   Asn Gln Phe Ser Leu Gln Leu Asn Ser Val Thr Pro Glu Asp Thr Ala
       210                 215                 220
   Val Tyr Tyr Cys Ala Arg Gly Arg Leu Gly Asp Ala Phe Asp Ile Trp
   225                 230                 235                 240
   Gly Gln Gly Thr Met Val Thr Val Ser Ser
                   245                 250
<210> 37
   <211> 750
   <212> DNA
   <213> Artificial Sequence
<220>
   <223> Synthetic Polynucleotide
<400> 37
   gacatccaga tgacccagtc tccatcctcc ctgtctgcat ctgtaggaga cagagtcacc    60
   atcacttgcc gggcaagtca gagcattagc agctatttaa attggtatca gcagaaacca   120
   gggaaagccc ctaagctcct gatctatgct gcatccagtt tgcaaagtgg ggtcccatca   180
   aggttcagtg gcagtggatc tgggacagat ttcactctca ccatcagcag tctgcaacct   240
   gaagattttg caacttacta ctgtcaacag agttacagta cccctctcac tttcggcgga   300
   gggaccaaag tggatatcaa acgttctaga ggtggtggtg gtagcggcgg cggcggctct   360
   ggtggtggtg gatccctcga gatggcccag gtacagctgc agcagtcagg tccaggactg   420
   gtgaagccct cgcagaccct ctcactcacc tgtgccatct ccggggacag tgtctctagc   480
   aacagtgctg cttggaactg gatcaggcag tccccatcga gaggccttga gtggctggga   540
   aggacatact acgggtccaa gtggtataat gattatgcag tatctgtgaa aagtcgaata   600
   accatcaacc cagacacatc caagaaccag ttctccctgc agctgaactc tgtgactccc   660
   gaggacacgg ctgtgtatta ctgtgcaaga ggtcgcttag ggatgctttt tgatatctgg   720
   ggccaaggga caatggtcac cgtctcttca                                    750
<210> 38
   <211> 10
   <212> PRT
   <213> Artificial Sequence
<220>
   <223> Synthetic Polypeptide
<400> 38
   Gly Tyr Ser Phe Thr Asn Phe Trp Ile Ser
   1               5                   10
<210> 39
   <211> 17
   <212> PRT
   <213> Artificial Sequence
<220>
   <223> Synthetic Polypeptide
<400> 39
   Arg Val Asp Pro Gly Tyr Ser Tyr Ser Thr Tyr Ser Pro Ser Phe Gln
   1               5                   10                  15
   Gly
<210> 40
   <211> 12
   <212> PRT
   <213> Artificial Sequence
<220>
   <223> Synthetic Polypeptide
<400> 40
   Val Gln Tyr Ser Gly Tyr Tyr Asp Trp Phe Asp Pro
   1               5                   10
<210> 41
   <211> 30
   <212> DNA
   <213> Artificial Sequence
<220>
   <223> Synthetic Polynucleotide
<400> 41
   ggatacagct tcaccaactt ctggatcagc 30
<210> 42
   <211> 51
   <212> DNA
   <213> Artificial Sequence
<220>
   <223> Synthetic Polynucleotide
<400> 42
   agggttgatc ctggctactc ttatagcacc tacagcccgt ccttccaagg c 51
<210> 43
   <211> 36
   <212> DNA
   <213> Artificial Sequence
<220>
   <223> Synthetic Polynucleotide
<400> 43
   gtacaatata gtggctacta tgactggttc gacccc 36
<210> 44
   <211> 13
   <212> PRT
   <213> Artificial Sequence
<220>
   <223> Synthetic Polypeptide
<400> 44
   Ser Gly Ser Ser Ser Asn Ile Gly Ser Asn Thr Val Asn
   1               5                   10
<210> 45
   <211> 7
   <212> PRT
   <213> Artificial Sequence
<220>
   <223> Synthetic Polypeptide
<400> 45
   Ser Asn Asn Gln Arg Pro Ser
   1 5
<210> 46
   <211> 11
   <212> PRT
   <213> Artificial Sequence
<220>
   <223> Synthetic Polypeptide
<400> 46
   Ala Ala Trp Asp Asp Ser Leu Asn Gly Trp Val
   1 5 10
<210> 47
   <211> 39
   <212> DNA
   <213> Artificial Sequence
<220>
   <223> Synthetic Polynucleotide
<400> 47
   tctggaagca gctccaacat cggaagtaat actgtaaac 39
<210> 48
   <211> 21
   <212> DNA
   <213> Artificial Sequence
<220>
   <223> Synthetic Polynucleotide
<400> 48
   agtaataatc agcggccctc a 21
<210> 49
   <211> 33
   <212> DNA
   <213> Artificial Sequence
<220>
   <223> Synthetic Polynucleotide
<400> 49
   gcagcatggg atgacagcct gaatggttgg gtg 33
<210> 50
   <211> 121
   <212> PRT
   <213> Artificial Sequence
<220>
   <223> Synthetic Polypeptide
<400> 50
   Gln Met Gln Leu Val Gln Ser Gly Ala Glu Val Lys Glu Pro Gly Glu
   1               5                   10                  15
   Ser Leu Arg Ile Ser Cys Lys Gly Ser Gly Tyr Ser Phe Thr Asn Phe
               20                  25                  30
   Trp Ile Ser Trp Val Arg Gln Met Pro Gly Lys Gly Leu Glu Trp Met
           35                  40                  45
   Gly Arg Val Asp Pro Gly Tyr Ser Tyr Ser Thr Tyr Ser Pro Ser Phe
       50                  55                  60
   Gln Gly His Val Thr Ile Ser Ala Asp Lys Ser Thr Ser Thr Ala Tyr
   65                  70                  75                  80
   Leu Gln Trp Asn Ser Leu Lys Ala Ser Asp Thr Ala Met Tyr Tyr Cys
                   85                  90                  95
   Ala Arg Val Gln Tyr Ser Gly Tyr Tyr Asp Trp Phe Asp Pro Trp Gly
               100                 105                 110
   Gln Gly Thr Leu Val Thr Val Ser Ser
           115                 120
<210> 51
   <211> 363
   <212> DNA
   <213> Artificial Sequence
<220>
   <223> Synthetic Polynucleotide
<400> 51
   cagatgcagc tggtgcagtc cggagcagag gtgaaagagc ccggggagtc tctgaggatc 60
   tcctgtaagg gttctggata cagcttcacc aacttctgga tcagctgggt gcgccagatg 120
   cccgggaaag gcctggagtg gatggggagg gttgatcctg gctactctta tagcacctac 180
   agcccgtcct tccaaggcca cgtcaccatc tcagctgaca agtctaccag cactgcctac 240
   ctgcagtgga acagcctgaa ggcctcggac accgccatgt attactgtgc gagagtacaa 300
   tatagtggct actatgactg gttcgacccc tggggccagg gaaccctggt caccgtctcc 360
   tca 363
<210> 52
   <211> 111
   <212> PRT
   <213> Artificial Sequence
<220>
   <223> Synthetic Polypeptide
<400> 52
   Gln Ala Val Val Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Thr Val Asn Trp Tyr Gln Gln Val Pro Gly Thr Ala Pro Lys Leu Leu
           35                  40                  45
   Ile Tyr Ser Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Gln
   65                  70                  75                  80
   Ser Glu Asp Glu Ala Asp Tyr Tyr Cys Ala Ala Trp Asp Asp Ser Leu
                   85                  90                  95
   Asn Gly Trp Val Phe Gly Gly Gly Thr Lys Leu Thr Val Leu Gly
               100                 105                 110
<210> 53
   <211> 333
   <212> DNA
   <213> Artificial Sequence
<220>
   <223> Synthetic Polynucleotide
<400> 53
   caggctgtgg tgactcagcc accctcagcg tctgggaccc ccgggcagag ggtcaccatc      60
   tcttgttctg gaagcagctc caacatcgga agtaatactg taaactggta ccagcaggtc     120
   ccaggaacgg cccccaaact cctcatctat agtaataatc agcggccctc aggggtccct     180
   gaccgattct ctggctccaa gtctggcacc tcagcctccc tggccatcag tgggctccag     240
   tctgaggatg aggctgatta ttactgtgca gcatgggatg acagcctgaa tggttgggtg     300
   ttcggcggag ggaccaagct gaccgtccta ggt                                  333
<210> 54
   <211> 253
   <212> PRT
   <213> Artificial Sequence
<220>
   <223> Synthetic Polypeptide
<400> 54
   Gln Ala Val Val Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Thr Val Asn Trp Tyr Gln Gln Val Pro Gly Thr Ala Pro Lys Leu Leu
           35                  40                  45
   Ile Tyr Ser Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Gln
   65                  70                  75                  80
   Ser Glu Asp Glu Ala Asp Tyr Tyr Cys Ala Ala Trp Asp Asp Ser Leu
                   85                  90                  95
   Asn Gly Trp Val Phe Gly Gly Gly Thr Lys Leu Thr Val Leu Gly Ser
               100                 105                 110
   Arg Gly Gly Gly Gly Ser Gly Gly Gly Gly Ser Gly Gly Gly Gly Ser
           115                 120                 125
   Leu Glu Met Ala Gln Met Gln Leu Val Gln Ser Gly Ala Glu Val Lys
       130                 135                 140
   Glu Pro Gly Glu Ser Leu Arg Ile Ser Cys Lys Gly Ser Gly Tyr Ser
   145                 150                 155                 160
   Phe Thr Asn Phe Trp Ile Ser Trp Val Arg Gln Met Pro Gly Lys Gly
                   165                 170                 175
   Leu Glu Trp Met Gly Arg Val Asp Pro Gly Tyr Ser Tyr Ser Thr Tyr
               180                 185                 190
   Ser Pro Ser Phe Gln Gly His Val Thr Ile Ser Ala Asp Lys Ser Thr
           195                 200                 205
   Ser Thr Ala Tyr Leu Gln Trp Asn Ser Leu Lys Ala Ser Asp Thr Ala
       210                 215                 220
   Met Tyr Tyr Cys Ala Arg Val Gln Tyr Ser Gly Tyr Tyr Asp Trp Phe
   225                 230                 235                 240
   Asp Pro Trp Gly Gln Gly Thr Leu Val Thr Val Ser Ser
                   245                 250
<210> 55
   <211> 759
   <212> DNA
   <213> Artificial Sequence
<220>
   <223> Synthetic Polynucleotide
<400> 55
   caggctgtgg tgactcagcc accctcagcg tctgggaccc ccgggcagag ggtcaccatc      60
   tcttgttctg gaagcagctc caacatcgga agtaatactg taaactggta ccagcaggtc     120
   ccaggaacgg cccccaaact cctcatctat agtaataatc agcggccctc aggggtccct 180
   gaccgattct ctggctccaa gtctggcacc tcagcctccc tggccatcag tgggctccag 240
   tctgaggatg aggctgatta ttactgtgca gcatgggatg acagcctgaa tggttgggtg 300
   ttcggcggag ggaccaagct gaccgtccta ggttctagag gtggtggtgg tagcggcggc 360
   ggcggctctg gtggtggtgg atccctcgag atggcccaga tgcagctggt gcagtccgga 420
   gcagaggtga aagagcccgg ggagtctctg aggatctcct gtaagggttc tggatacagc 480
   ttcaccaact tctggatcag ctgggtgcgc cagatgcccg ggaaaggcct ggagtggatg 540
   gggagggttg atcctggcta ctcttatagc acctacagcc cgtccttcca aggccacgtc 600
   accatctcag ctgacaagtc taccagcact gcctacctgc agtggaacag cctgaaggcc 660
   tcggacaccg ccatgtatta ctgtgcgaga gtacaatata gtggctacta tgactggttc 720
   gacccctggg gccagggaac cctggtcacc gtctcctca 759
<210> 56
   <211> 10
   <212> PRT
   <213> Artificial Sequence
<220>
   <223> Synthetic Polypeptide
<400> 56
   Gly Tyr Asn Phe Ser Asn Lys Trp Ile Gly
   1 5 10
<210> 57
   <211> 17
   <212> PRT
   <213> Artificial Sequence
<220>
   <223> Synthetic Polypeptide
<400> 57
   Ile Ile Tyr Pro Gly Tyr Ser Asp Ile Thr Tyr Ser Pro Ser Phe Gln
   1 5 10 15
   Gly
<210> 58
   <211> 9
   <212> PRT
   <213> Artificial Sequence
<220>
   <223> Synthetic Polypeptide
<400> 58
   His Thr Ala Leu Ala Gly Phe Asp Tyr
   1 5
<210> 59
   <211> 30
   <212> DNA
   <213> Artificial Sequence
<220>
   <223> Synthetic Polynucleotide
<400> 59
   ggctacaact ttagcaacaa gtggatcggc 30
<210> 60
   <211> 51
   <212> DNA
   <213> Artificial Sequence
<220>
   <223> Synthetic Polynucleotide
<400> 60
   atcatctatc ccggttactc ggacatcacc tacagcccgt ccttccaagg c 51
<210> 61
   <211> 27
   <212> DNA
   <213> Artificial Sequence
<220>
   <223> Synthetic Polynucleotide
<400> 61
   cacacagctt tggccggctt tgactac 27
<210> 62
   <211> 11
   <212> PRT
   <213> Artificial Sequence
<220>
   <223> Synthetic Polypeptide
<400> 62
   Arg Ala Ser Gln Asn Ile Asn Lys Trp Leu Ala
   1 5 10
<210> 63
   <211> 7
   <212> PRT
   <213> Artificial Sequence
<220>
   <223> Synthetic Polypeptide
<400> 63
   Lys Ala Ser Ser Leu Glu Ser
   1               5
<210> 64
   <211> 8
   <212> PRT
   <213> Artificial Sequence
<220>
   <223> Synthetic Polypeptide
<400> 64
   Gln Gln Tyr Asn Ser Tyr Ala Thr
   1               5
<210> 65
   <211> 33
   <212> DNA
   <213> Artificial Sequence
<220>
   <223> Synthetic Polynucleotide
<400> 65
   cgggccagtc agaatatcaa taagtggctg gcc 33
<210> 66
   <211> 21
   <212> DNA
   <213> Artificial Sequence
<220>
   <223> Synthetic Polynucleotide
<400> 66
   aaggcgtcta gtttagaaag t 21
<210> 67
   <211> 24
   <212> DNA
   <213> Artificial Sequence
<220>
   <223> Synthetic Polynucleotide
<400> 67
   caacaatata atagttatgc gacg 24
<210> 68
   <211> 118
   <212> PRT
   <213> Artificial Sequence
<220>
   <223> Synthetic Polypeptide
<400> 68
   Gln Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Asn Phe Ser Asn Lys
               20                  25                  30
   Trp Ile Gly Trp Val Arg Gln Leu Pro Gly Arg Gly Leu Glu Trp Ile
           35                  40                  45
   Ala Ile Ile Tyr Pro Gly Tyr Ser Asp Ile Thr Tyr Ser Pro Ser Phe
       50                  55                  60
   Gln Gly Arg Val Thr Ile Ser Ala Asp Thr Ser Ile Asn Thr Ala Tyr
   65                  70                  75                  80
   Leu His Trp His Ser Leu Lys Ala Ser Asp Thr Ala Met Tyr Tyr Cys
                   85                  90                  95
   Val Arg His Thr Ala Leu Ala Gly Phe Asp Tyr Trp Gly Leu Gly Thr
               100                 105                 110
   Leu Val Thr Val Ser Ser
           115
<210> 69
   <211> 354
   <212> DNA
   <213> Artificial Sequence
<220>
   <223> Synthetic Polynucleotide
<400> 69
   caggtgcagc tggtgcagtc tggagcagag gtgaaaaagc ccggagagtc tctgaagatc      60
   tcctgtaagg gttctggcta caactttagc aacaagtgga tcggctgggt gcgccaattg     120
   cccgggagag gcctggagtg gatagcaatc atctatcccg gttactcgga catcacctac     180
   agcccgtcct tccaaggccg cgtcaccatc tccgccgaca cgtccattaa caccgcctac     240
   ctgcactggc acagcctgaa ggcctcggac accgccatgt attattgtgt gcgacacaca     300
   gctttggccg gctttgacta ctggggcctg ggcaccctgg tcaccgtctc ctca           354
<210> 70
   <211> 107
   <212> PRT
   <213> Artificial Sequence
<220>
   <223> Synthetic Polypeptide
<400> 70
   Asp Ile Gln Met Thr Gln Ser Pro Ser Thr Leu Ser Ala Ser Val Gly
   1               5                   10                  15
   Asp Arg Val Thr Ile Thr Cys Arg Ala Ser Gln Asn Ile Asn Lys Trp
               20                  25                  30
   Leu Ala Trp Tyr Gln Gln Arg Pro Gly Lys Ala Pro Gln Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Tyr Thr Leu Thr Ile Ser Ser Leu Gln Pro
   65                  70                  75                  80
   Asp Asp Phe Ala Thr Tyr Tyr Cys Gln Gln Tyr Asn Ser Tyr Ala Thr
                   85                  90                  95
   Phe Gly Gln Gly Thr Lys Val Glu Ile Lys Arg
               100                 105
<210> 71
   <211> 321
   <212> DNA
   <213> Artificial Sequence
<220>
   <223> Synthetic Polynucleotide
<400> 71
   gacatccaga tgacccagtc tccttccacc ctgtctgcat ctgtaggaga cagagtcaca      60
   atcacttgcc gggccagtca gaatatcaat aagtggctgg cctggtatca gcagagacca     120
   gggaaagccc ctcagctcct gatctataag gcgtctagtt tagaaagtgg ggtcccatct     180
   aggttcagcg gcagtggatc tgggacagaa tacactctca ccatcagcag cctgcagcct     240
   gatgattttg caacttatta ctgccaacaa tataatagtt atgcgacgtt cggccaaggg     300
   accaaggtgg aaatcaaacg t                                               321
<210> 72
   <211> 246
   <212> PRT
   <213> Artificial Sequence
<220>
   <223> Synthetic Polypeptide
<400> 72
   Asp Ile Gln Met Thr Gln Ser Pro Ser Thr Leu Ser Ala Ser Val Gly
   1               5                   10                  15
   Asp Arg Val Thr Ile Thr Cys Arg Ala Ser Gln Asn Ile Asn Lys Trp
               20                  25                  30
   Leu Ala Trp Tyr Gln Gln Arg Pro Gly Lys Ala Pro Gln Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Tyr Thr Leu Thr Ile Ser Ser Leu Gln Pro
   65                  70                  75                  80
   Asp Asp Phe Ala Thr Tyr Tyr Cys Gln Gln Tyr Asn Ser Tyr Ala Thr
                   85                  90                  95
   Phe Gly Gln Gly Thr Lys Val Glu Ile Lys Arg Ser Arg Gly Gly Gly
               100                 105                 110
   Gly Ser Gly Gly Gly Gly Ser Gly Gly Gly Gly Ser Leu Glu Met Ala
           115                 120                 125
   Gln Val Gln Leu Val Gln Ser Gly Ala Glu Val Lys Lys Pro Gly Glu
       130                 135                 140
   Ser Leu Lys Ile Ser Cys Lys Gly Ser Gly Tyr Asn Phe Ser Asn Lys
   145                 150                 155                 160
   Trp Ile Gly Trp Val Arg Gln Leu Pro Gly Arg Gly Leu Glu Trp Ile
                   165                 170                 175
   Ala Ile Ile Tyr Pro Gly Tyr Ser Asp Ile Thr Tyr Ser Pro Ser Phe
               180                 185                 190
   Gln Gly Arg Val Thr Ile Ser Ala Asp Thr Ser Ile Asn Thr Ala Tyr
           195                 200                 205
   Leu His Trp His Ser Leu Lys Ala Ser Asp Thr Ala Met Tyr Tyr Cys
       210                 215                 220
   Val Arg His Thr Ala Leu Ala Gly Phe Asp Tyr Trp Gly Leu Gly Thr
   225                 230                 235                 240
   Leu Val Thr Val Ser Ser
   245
<210> 73
   <211> 738
   <212> DNA
   <213> Artificial Sequence
<220>
   <223> Synthetic Polynucleotide
<400> 73
   gacatccaga tgacccagtc tccttccacc ctgtctgcat ctgtaggaga cagagtcaca      60
   atcacttgcc gggccagtca gaatatcaat aagtggctgg cctggtatca gcagagacca     120
   gggaaagccc ctcagctcct gatctataag gcgtctagtt tagaaagtgg ggtcccatct     180
   aggttcagcg gcagtggatc tgggacagaa tacactctca ccatcagcag cctgcagcct     240
   gatgattttg caacttatta ctgccaacaa tataatagtt atgcgacgtt cggccaaggg     300
   accaaggtgg aaatcaaacg ttctagaggt ggtggtggta gcggcggcgg cggctctggt     360
   ggtggtggat ccctcgagat ggcccaggtg cagctggtgc agtctggagc agaggtgaaa     420
   aagcccggag agtctctgaa gatctcctgt aagggttctg gctacaactt tagcaacaag     480
   tggatcggct gggtgcgcca attgcccggg agaggcctgg agtggatagc aatcatctat     540
   cccggttact cggacatcac ctacagcccg tccttccaag gccgcgtcac catctccgcc     600
   gacacgtcca ttaacaccgc ctacctgcac tggcacagcc tgaaggcctc ggacaccgcc     660
   atgtattatt gtgtgcgaca cacagctttg gccggctttg actactgggg cctgggcacc     720
   ctggtcaccg tctcctca                                                   738
<210> 74
   <211> 10
   <212> PRT
   <213> Artificial Sequence
<220>
   <223> Synthetic Polypeptide
<400> 74
   Gly Phe Thr Phe Asp Asp Tyr Gly Met Ser
   1               5                   10
<210> 75
   <211> 17
   <212> PRT
   <213> Artificial Sequence
<220>
   <223> Synthetic Polypeptide
<400> 75
   Gly Ile Asn Trp Asn Gly Gly Ser Thr Gly Tyr Ala Asp Ser Val Arg
   1 5 10 15
   Gly
<210> 76
   <211> 12
   <212> PRT
   <213> Artificial Sequence
<220>
   <223> Synthetic Polypeptide
<400> 76
   Glu Arg Gly Tyr Gly Tyr His Asp Pro His Asp Tyr
   1 5 10
<210> 77
   <211> 30
   <212> DNA
   <213> Artificial Sequence
<220>
   <223> Synthetic Polynucleotide
<400> 77
   gggttcacct ttgatgatta tggcatgagc 30
<210> 78
   <211> 51
   <212> DNA
   <213> Artificial Sequence
<220>
   <223> Synthetic Polynucleotide
<400> 78
   ggtattaatt ggaatggtgg tagcacaggt tatgcagact ctgtgagggg c 51
<210> 79
   <211> 36
   <212> DNA
   <213> Artificial Sequence
<220>
   <223> Synthetic Polynucleotide
<400> 79
   gagcgtggct acgggtacca tgatccccat gactac 36
<210> 80
   <211> 11
   <212> PRT
   <213> Artificial Sequence
<220>
   <223> Synthetic Polypeptide
<400> 80
   Gly Arg Asn Asn Ile Gly Ser Lys Ser Val His
   1               5                   10
<210> 81
   <211> 7
   <212> PRT
   <213> Artificial Sequence
<220>
   <223> Synthetic Polypeptide
<400> 81
   Asp Asp Ser Asp Arg Pro Ser
   1               5
<210> 82
   <211> 11
   <212> PRT
   <213> Artificial Sequence
<220>
   <223> Synthetic Polypeptide
<400> 82
   Gln Val Trp Asp Ser Ser Ser Asp His Val Val
   1               5                   10
<210> 83
   <211> 33
   <212> DNA
   <213> Artificial Sequence
<220>
   <223> Synthetic Polynucleotide
<400> 83
   gggagaaaca acattggaag taaaagtgtg cac 33
<210> 84
   <211> 21
   <212> DNA
   <213> Artificial Sequence
<220>
   <223> Synthetic Polynucleotide
<400> 84
   gatgatagcg accggccctc a 21
<210> 85
   <211> 33
   <212> DNA
   <213> Artificial Sequence
<220>
   <223> Synthetic Polynucleotide
<400> 85
   caggtgtggg atagtagtag tgatcatgtg gta 33
<210> 86
   <211> 121
   <212> PRT
   <213> Artificial Sequence
<220>
   <223> Synthetic Polypeptide
<400> 86
   Glu Val Gln Leu Val Gln Ser Gly Gly Gly Val Val Arg Pro Gly Gly
   1               5                   10                  15
   Ser Leu Arg Leu Ser Cys Ala Ala Ser Gly Phe Thr Phe Asp Asp Tyr
               20                  25                  30
   Gly Met Ser Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
       50                  55                  60
   Arg Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Glu Arg Gly Tyr Gly Tyr His Asp Pro His Asp Tyr Trp Gly
               100                 105                 110
   Gln Gly Thr Leu Val Thr Val Ser Ser
           115                 120
<210> 87
   <211> 363
   <212> DNA
   <213> Artificial Sequence
<220>
   <223> Synthetic Polynucleotide
<400> 87
   gaagtgcagc tggtgcagtc tgggggaggt gtggtacggc ctggggggtc cctgagactc 60
   tcctgtgcag cctctgggtt cacctttgat gattatggca tgagctgggt ccgccaagct 120
   ccagggaagg ggctggagtg ggtctctggt attaattgga atggtggtag cacaggttat 180
   gcagactctg tgaggggccg attcaccatc tccagagaca acgccaagaa ctccctgtat 240
   ctgcaaatga acagtctgag agccgaggac acggccttgt attactgtgc gagagagcgt 300
   ggctacgggt accatgatcc ccatgactac tggggccaag gcaccctggt gaccgtctcc 360
   tca 363
<210> 88
   <211> 109
   <212> PRT
   <213> Artificial Sequence
<220>
   <223> Synthetic Polypeptide
<400> 88
   Gln Ser Val Val Thr Gln Pro Pro Ser Val Ser Val Ala Pro Gly Lys
   1               5                   10                  15
   Thr Ala Arg Ile Thr Cys Gly Arg Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Val Val Phe Gly Gly Gly Thr Lys Leu Thr Val Leu Gly
               100                 105
<210> 89
   <211> 327
   <212> DNA
   <213> Artificial Sequence
<220>
   <223> Synthetic Polypeptide
<400> 89
   cagtctgtcg tgacgcagcc gccctcggtg tcagtggccc caggaaagac ggccaggatt      60
   acctgtggga gaaacaacat tggaagtaaa agtgtgcact ggtaccagca gaagccaggc     120
   caggcccctg tgctggtcgt ctatgatgat agcgaccggc cctcagggat ccctgagcga     180
   ttctctggct ccaactctgg gaacacggcc accctgacca tcagcagggt cgaagccggg     240
   gatgaggccg actattactg tcaggtgtgg gatagtagta gtgatcatgt ggtattcggc     300
   ggagggacca agctgaccgt cctaggt                                         327
<210> 90
   <211> 249
   <212> PRT
   <213> Artificial Sequence
<220>
   <223> Synthetic Polypeptide
<400> 90
   Gln Ser Val Val Thr Gln Pro Pro Ser Val Ser Val Ala Pro Gly Lys
   1               5                   10                  15
   Thr Ala Arg Ile Thr Cys Gly Arg Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Val Val Phe Gly Gly Gly Thr Lys Leu Thr Val Leu Gly Ser Arg Gly
               100                 105                 110
   Gly Gly Gly Ser Gly Gly Gly Gly Ser Gly Gly Ser Leu Glu Met Ala
           115                 120                 125
   Glu Val Gln Leu Val Gln Ser Gly Gly Gly Val Val Arg Pro Gly Gly
       130                 135                 140
   Ser Leu Arg Leu Ser Cys Ala Ala Ser Gly Phe Thr Phe Asp Asp Tyr
   145                 150                 155                 160
   Gly Met Ser Trp Val Arg Gln Ala Pro Gly Lys Gly Leu Glu Trp Val
                   165                 170                 175
   Ser Gly Ile Asn Trp Asn Gly Gly Ser Thr Gly Tyr Ala Asp Ser Val
               180                 185                 190
   Arg Gly Arg Phe Thr Ile Ser Arg Asp Asn Ala Lys Asn Ser Leu Tyr
           195                 200                 205
   Leu Gln Met Asn Ser Leu Arg Ala Glu Asp Thr Ala Leu Tyr Tyr Cys
       210                 215                 220
   Ala Arg Glu Arg Gly Tyr Gly Tyr His Asp Pro His Asp Tyr Trp Gly
   225                 230                 235                 240
   Gln Gly Thr Leu Val Thr Val Ser Ser
                   245
<210> 91
   <211> 747
   <212> DNA
   <213> Artificial Sequence
<220>
   <223> Synthetic Polynucleotide
<400> 91
   cagtctgtcg tgacgcagcc gccctcggtg tcagtggccc caggaaagac ggccaggatt      60
   acctgtggga gaaacaacat tggaagtaaa agtgtgcact ggtaccagca gaagccaggc     120
   caggcccctg tgctggtcgt ctatgatgat agcgaccggc cctcagggat ccctgagcga     180
   ttctctggct ccaactctgg gaacacggcc accctgacca tcagcagggt cgaagccggg     240
   gatgaggccg actattactg tcaggtgtgg gatagtagta gtgatcatgt ggtattcggc     300
   ggagggacca agctgaccgt cctaggttct agaggtggtg gtggtagcgg cggcggcggc     360
   tctggtggat ccctcgagat ggccgaagtg cagctggtgc agtctggggg aggtgtggta     420
   cggcctgggg ggtccctgag actctcctgt gcagcctctg ggttcacctt tgatgattat     480
   ggcatgagct gggtccgcca agctccaggg aaggggctgg agtgggtctc tggtattaat     540
   tggaatggtg gtagcacagg ttatgcagac tctgtgaggg gccgattcac catctccaga     600
   gacaacgcca agaactccct gtatctgcaa atgaacagtc tgagagccga ggacacggcc     660
   ttgtattact gtgcgagaga gcgtggctac gggtaccatg atccccatga ctactggggc     720
   caaggcaccc tggtgaccgt ctcctca                                         747
<210> 92
   <211> 10
   <212> PRT
   <213> Artificial Sequence
<220>
   <223> Synthetic Polypeptide
<400> 92
   Gly Phe Ser Val Ser Gly Thr Tyr Met Gly
   1               5                   10
<210> 93
   <211> 16
   <212> PRT
   <213> Artificial Sequence
<220>
   <223> Synthetic Polypeptide
<400> 93
   Leu Leu Tyr Ser Gly Gly Gly Thr Tyr His Pro Ala Ser Leu Gln Gly
   1 5 10 15
<210> 94
   <211> 10
   <212> PRT
   <213> Artificial Sequence
<220>
   <223> Synthetic Polypeptide
<400> 94
   Gly Gly Ala Gly Gly Gly His Phe Asp Ser
   1 5 10
<210> 95
   <211> 30
   <212> DNA
   <213> Artificial Sequence
<220>
   <223> Synthetic Polynucleotide
<400> 95
   gggttctccg tcagtggcac ctacatgggc 30
<210> 96
   <211> 48
   <212> DNA
   <213> Artificial Sequence
<220>
   <223> Synthetic Polynucleotide
<400> 96
   cttctttata gtggtggcgg cacataccac ccagcgtccc tgcagggc 48
<210> 97
   <211> 29
   <212> DNA
   <213> Artificial Sequence
<220>
   <223> Synthetic Polynucleotide
<400> 97
   gaggggcagg aggtggccac tttgactcc 29
<210> 98
   <211> 14
   <212> PRT
   <213> Artificial Sequence
<220>
   <223> Synthetic Polypeptide
<400> 98
   Thr Gly Ser Ser Ser Asn Ile Gly Ala Gly Tyr Asp Val His
   1 5 10
<210> 99
   <211> 7
   <212> PRT
   <213> Artificial Sequence
<220>
   <223> Synthetic Polypeptide
<400> 99
   Gly Asn Ser Asn Arg Pro Ser
   1 5
<210> 100
   <211> 11
   <212> PRT
   <213> Artificial Sequence
<220>
   <223> Synthetic Polypeptide
<400> 100
   Ala Ala Trp Asp Asp Ser Leu Asn Gly Tyr Val
   1 5 10
<210> 101
   <211> 42
   <212> DNA
   <213> Artificial Sequence
<220>
   <223> Synthetic Polynucleotide
<400> 101
   actgggagca gctccaacat cggggcaggt tatgatgtac ac 42
<210> 102
   <211> 21
   <212> DNA
   <213> Artificial Sequence
<220>
   <223> Synthetic Polynucleotide
<400> 102
   ggtaacagca atcggccctc a 21
<210> 103
   <211> 33
   <212> DNA
   <213> Artificial Sequence
<220>
   <223> Synthetic Polynucleotide
<400> 103
   gcagcatggg atgacagcct gaatggttat gtc 33
<210> 104
   <211> 118
   <212> PRT
   <213> Artificial Sequence
<220>
   <223> Synthetic Polypeptide
<400> 104
   Glu Val Gln Leu Val Glu Thr Gly Gly Gly Leu Leu Gln Pro Gly Gly
   1               5                   10                  15
   Ser Leu Arg Leu Ser Cys Ala Ala Ser Gly Phe Ser Val Ser Gly Thr
               20                  25                  30
   Tyr Met Gly Trp Val Arg Gln Ala Pro Gly Lys Gly Leu Glu Trp Val
           35                  40                  45
   Ala Leu Leu Tyr Ser Gly Gly Gly Thr Tyr His Pro Ala Ser Leu Gln
       50                  55                  60
   Gly Arg Phe Ile Val Ser Arg Asp Ser Ser Lys Asn Met Val Tyr Leu
   65                  70                  75                  80
   Gln Met Asn Ser Leu Lys Ala Glu Asp Thr Ala Val Tyr Tyr Cys Ala
                   85                  90                  95
   Lys Gly Gly Ala Gly Gly Gly His Phe Asp Ser Trp Gly Gln Gly Thr
               100                 105                 110
   Leu Val Thr Val Ser Ser
           115
<210> 105
   <211> 354
   <212> DNA
   <213> Artificial Sequence
<220>
   <223> Synthetic Polynucleotide
<400> 105
   gaggtgcagc tggtggagac cggaggaggc ttgctccagc cgggggggtc cctcagactc      60
   tcctgtgcag cctctgggtt ctccgtcagt ggcacctaca tgggctgggt ccgccaggct    120
   ccagggaagg gactggagtg ggtcgcactt ctttatagtg gtggcggcac ataccaccca    180
   gcgtccctgc agggccgatt catcgtctcc agagacagct ccaagaatat ggtctatctt    240
   caaatgaata gcctgaaagc cgaggacacg gccgtctatt actgtgcgaa aggaggggca    300
   ggaggtggcc actttgactc ctggggccaa ggcaccctgg tgaccgtctc ctca          354
<210> 106
   <211> 112
   <212> PRT
   <213> Artificial Sequence
<220>
   <223> Synthetic Polypeptide
<400> 106
   Gln Ser Val Leu Thr Gln Pro Pro Ser Val Ser Gly Ala Pro Gly Gln
   1               5                   10                  15
   Arg Val Thr Ile Ser Cys Thr Gly Ser Ser Ser Asn Ile Gly Ala Gly
               20                  25                  30
   Tyr Asp Val His Trp Tyr Gln Gln Leu Pro Gly Thr Ala Pro Lys Leu
           35                  40                  45
   Leu Ile Tyr Gly Asn Ser Asn Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Gln Ser Glu Asp Glu Ala Asp Tyr Tyr Cys Ala Ala Trp Asp Asp Ser
                   85                  90                  95
   Leu Asn Gly Tyr Val Phe Gly Thr Gly Thr Lys Leu Thr Val Leu Gly
               100                 105                 110
<210> 107
   <211> 336
   <212> DNA
   <213> Artificial Sequence
<220>
   <223> Synthetic Polynucleotide
<400> 107
   cagtctgtgt tgacgcagcc gccctcagtg tctggggccc cagggcagag ggtcaccatc      60
   tcctgcactg ggagcagctc caacatcggg gcaggttatg atgtacactg gtaccagcag     120
   cttccaggaa cagcccccaa actcctcatc tatggtaaca gcaatcggcc ctcaggggtc     180
   cctgaccgat tctctggctc caagtctggc acctcagcct ccctggccat cagtgggctc     240
   cagtctgagg atgaggctga ttattactgt gcagcatggg atgacagcct gaatggttat     300
   gtcttcggaa ctgggaccaa gctgaccgtc ctaggt                               336
<210> 108
   <211> 251
   <212> PRT
   <213> Artificial Sequence
<220>
   <223> Synthetic Polypeptide
<400> 108
   Gln Ser Val Leu Thr Gln Pro Pro Ser Val Ser Gly Ala Pro Gly Gln
   1               5                   10                  15
   Arg Val Thr Ile Ser Cys Thr Gly Ser Ser Ser Asn Ile Gly Ala Gly
               20                  25                  30
   Tyr Asp Val His Trp Tyr Gln Gln Leu Pro Gly Thr Ala Pro Lys Leu
           35                  40                  45
   Leu Ile Tyr Gly Asn Ser Asn Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Gln Ser Glu Asp Glu Ala Asp Tyr Tyr Cys Ala Ala Trp Asp Asp Ser
                   85                  90                  95
   Leu Asn Gly Tyr Val Phe Gly Thr Gly Thr Lys Leu Thr Val Leu Gly
               100                 105                 110
   Ser Arg Gly Gly Gly Gly Ser Gly Gly Gly Gly Ser Gly Gly Gly Gly
           115                 120                 125
   Ser Leu Glu Met Ala Glu Val Gln Leu Val Glu Thr Gly Gly Gly Leu
       130                 135                 140
   Leu Gln Pro Gly Gly Ser Leu Arg Leu Ser Cys Ala Ala Ser Gly Phe
   145                 150                 155                 160
   Ser Val Ser Gly Thr Tyr Met Gly Trp Val Arg Gln Ala Pro Gly Lys
                   165                 170                 175
   Gly Leu Glu Trp Val Ala Leu Leu Tyr Ser Gly Gly Gly Thr Tyr His
               180                 185                 190
   Pro Ala Ser Leu Gln Gly Arg Phe Ile Val Ser Arg Asp Ser Ser Lys
           195                 200                 205
   Asn Met Val Tyr Leu Gln Met Asn Ser Leu Lys Ala Glu Asp Thr Ala
       210                 215                 220
   Val Tyr Tyr Cys Ala Lys Gly Gly Ala Gly Gly Gly His Phe Asp Ser
   225                 230                 235                 240
   Trp Gly Gln Gly Thr Leu Val Thr Val Ser Ser
                   245                 250
<210> 109
   <211> 753
   <212> DNA
   <213> Artificial Sequence
<220>
   <223> Synthetic Polynucleotide
<400> 109
   cagtctgtgt tgacgcagcc gccctcagtg tctggggccc cagggcagag ggtcaccatc 60
   tcctgcactg ggagcagctc caacatcggg gcaggttatg atgtacactg gtaccagcag 120
   cttccaggaa cagcccccaa actcctcatc tatggtaaca gcaatcggcc ctcaggggtc 180
   cctgaccgat tctctggctc caagtctggc acctcagcct ccctggccat cagtgggctc 240
   cagtctgagg atgaggctga ttattactgt gcagcatggg atgacagcct gaatggttat 300
   gtcttcggaa ctgggaccaa gctgaccgtc ctaggttcta gaggtggtgg tggtagcggc 360
   ggcggcggct ctggtggtgg tggatccctc gagatggccg aggtgcagct ggtggagacc 420
   ggaggaggct tgctccagcc gggggggtcc ctcagactct cctgtgcagc ctctgggttc 480
   tccgtcagtg gcacctacat gggctgggtc cgccaggctc cagggaaggg actggagtgg 540
   gtcgcacttc tttatagtgg tggcggcaca taccacccag cgtccctgca gggccgattc 600
   atcgtctcca gagacagctc caagaatatg gtctatcttc aaatgaatag cctgaaagcc 660
   gaggacacgg ccgtctatta ctgtgcgaaa ggaggggcag gaggtggcca ctttgactcc 720
   tggggccaag gcaccctggt gaccgtctcc tca 753
<210> 110
   <211> 523
   <212> PRT
   <213> Artificial Sequence
<220>
   <223> Synthetic Polypeptide
<400> 110
<210> 111
   <211> 107
   <212> PRT
   <213> Artificial Sequence
<220>
   <223> Synthetic Polypeptide
<400> 111
   Asp Asp Ile Val Leu Thr Gln Ser Pro Ala Thr Leu Ser Leu Ser Pro
   1 5 10 15
   Gly Glu Arg Ala Thr Leu Ser Cys Arg Ala Ser Gln Ser Val Ser Tyr
   20 25 30
   Met Asn Trp Tyr Gln Gln Lys Pro Gly Lys Ala Pro Lys Arg Trp Ile
   35 40 45
   Tyr Asp Thr Ser Lys Val Ala Ser Gly Val Pro Ala Arg Phe Ser Gly
   50 55 60
   Ser Gly Ser Gly Thr Asp Tyr Ser Leu Thr Ile Asn Ser Leu Glu Ala
   65 70 75 80
   Glu Asp Ala Ala Thr Tyr Tyr Cys Gln Gln Trp Ser Ser Asn Pro Leu
   85 90 95
   Thr Phe Gly Gly Gly Thr Lys Val Glu Ile Lys
   100 105
<210> 112
   <211> 119
   <212> PRT
   <213> Artificial Sequence
<220>
   <223> Synthetic Polypeptide
<400> 112
   Asp Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Arg Tyr
               20                  25                  30
   Thr Met His Trp Val Arg Gln Ala Pro Gly Gln Gly Leu Glu Trp Ile
           35                  40                  45
   Gly Tyr Ile Asn Pro Ser Arg Gly Tyr Thr Asn Tyr Ala Asp Ser Val
       50                  55                  60
   Lys Gly Arg Phe Thr Ile Thr Thr Asp Lys Ser Thr Ser Thr Ala Tyr
   65                  70                  75                  80
   Met Glu Leu Ser Ser Leu Arg Ser Glu Asp Thr Ala Thr Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Val Thr Val Ser Ser
           115
<210> 113
   <211> 243
   <212> PRT
   <213> Artificial Sequence
<220>
   <223> Synthetic Polypeptide
<400> 113
   Asp Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Arg Tyr
               20                  25                  30
   Thr Met His Trp Val Arg Gln Ala Pro Gly Gln Gly Leu Glu Trp Ile
           35                  40                  45
   Gly Tyr Ile Asn Pro Ser Arg Gly Tyr Thr Asn Tyr Ala Asp Ser Val
       50                  55                  60
   Lys Gly Arg Phe Thr Ile Thr Thr Asp Lys Ser Thr Ser Thr Ala Tyr
   65                  70                  75                  80
   Met Glu Leu Ser Ser Leu Arg Ser Glu Asp Thr Ala Thr Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Val Thr Val Ser Ser Gly Glu Gly Thr Ser Thr Gly Ser Gly
           115                 120                 125
   Gly Ser Gly Gly Ser Gly Gly Ala Asp Asp Ile Val Leu Thr Gln Ser
       130                 135                 140
   Pro Ala Thr Leu Ser Leu Ser Pro Gly Glu Arg Ala Thr Leu Ser Cys
   145                 150                 155                 160
   Arg Ala Ser Gln Ser Val Ser Tyr Met Asn Trp Tyr Gln Gln Lys Pro
                   165                 170                 175
   Gly Lys Ala Pro Lys Arg Trp Ile Tyr Asp Thr Ser Lys Val Ala Ser
               180                 185                 190
   Gly Val Pro Ala Arg Phe Ser Gly Ser Gly Ser Gly Thr Asp Tyr Ser
           195                 200                 205
   Leu Thr Ile Asn Ser Leu Glu Ala Glu Asp Ala Ala Thr Tyr Tyr Cys
       210                 215                 220
   Gln Gln Trp Ser Ser Asn Pro Leu Thr Phe Gly Gly Gly Thr Lys Val
   225                 230                 235                 240
   Glu Ile Lys
<210> 114
   <211> 1503
   <212> DNA
   <213> Artificial Sequence
<220>
   <223> Synthetic Polynucleotide
<400> 114
   caggctgtcg tgactcagcc tccttctgct tctggcaccc ctggccagag agtgaccatc 60
   tcctgctccg gctcctcctc caacatcggc tccaacaccg tgaactggta tcagcaggtg 120
   cccggcaccg cccccaagct gctgatctac tctaacaacc agcggccctc cggcgtgccc      180
   gacagattct ctggctctaa gtccggcacc tccgcctccc tggctatctc tggcctgcag      240
   tctgaggacg aggccgacta ctactgcgcc gcctgggacg attctctgaa cggctgggtg      300
   ttcggcggag gcaccaagct gacagtgctg ggaagtagag gcggtggcgg atctggtggc      360
   ggaggatctg gcggaggggg ctctctggaa atggcccaga tgcagctggt gcagtctggc      420
   gccgaagtga aagagcctgg cgagtccctg cggatctcct gcaagggctc cggctacagc      480
   tttaccaact tctggatcag ctgggtgcga cagatgcccg gcaagggcct ggaatggatg      540
   ggcagagtgg accccggcta ctcctactcc acctactccc ccagcttcca gggccacgtg      600
   accatcagcg ccgacaagtc tacctccacc gcctacctgc agtggaactc cctgaaggcc      660
   tccgacaccg ccatgtacta ctgtgcccgg gtgcagtaca gcggctacta cgattggttc      720
   gacccctggg gccagggcac cctcgtgaca gtgtctagtg gcgggggagg atccgacgtg      780
   cagctggtgc agagcggagc tgaagtgaag aaacctggcg cctccgtgaa ggtgtcctgc      840
   aaagctagcg gctatacctt cacccggtac accatgcact gggtgcgcca ggcacctgga      900
   cagggactgg aatggatcgg ctacatcaac ccctcccggg gctacaccaa ctacgccgac      960
   tctgtgaagg gccggttcac catcaccacc gataagtcca ccagcaccgc ttacatggaa     1020
   ctgtcctccc tgagatccga ggacaccgct acctactatt gcgcccggta ctacgacgac     1080
   cactactgcc tggactactg gggacaggga accacagtga ccgtgtcctc tggcgagggc     1140
   acctctactg gatctggggg aagtggtggt tctggcggcg ctgacgacat cgtgctgacc     1200
   cagtctccag ccaccctgtc tctgagccca ggcgagagag ctaccctgtc ctgcagagcc     1260
   tcccagtccg tgtcctacat gaattggtat cagcagaagc ctggcaaggc ccctaagcgg     1320
   tggatctacg acacctccaa ggtggcctct ggcgtgccag cccggttttc cggatctggc     1380
   tctggcaccg actactccct gaccatcaac agcctggaag ccgaggacgc tgccacctat     1440
   tactgccagc agtggtcctc caaccccctg acctttggag gcggcaccaa ggtggaaatc     1500
   aag                                                                   1503
<210> 115
   <211> 1575
   <212> DNA
   <213> Artificial Sequence
<220>
   <223> Synthetic Polynucleotide
<400> 115
   atgggctggt cctgcatcat cctgtttctg gtggctaccg ccaccggcca ggctgtcgtg      60
   actcagcctc cttctgcttc tggcacccct ggccagagag tgaccatctc ctgctccggc     120
   tcctcctcca acatcggctc caacaccgtg aactggtatc agcaggtgcc cggcaccgcc     180
   cccaagctgc tgatctactc taacaaccag cggccctccg gcgtgcccga cagattctct     240
   ggctctaagt ccggcacctc cgcctccctg gctatctctg gcctgcagtc tgaggacgag     300
   gccgactact actgcgccgc ctgggacgat tctctgaacg gctgggtgtt cggcggaggc     360
   accaagctga cagtgctggg aagtagaggc ggtggcggat ctggtggcgg aggatctggc     420
   ggagggggct ctctggaaat ggcccagatg cagctggtgc agtctggcgc cgaagtgaaa     480
   gagcctggcg agtccctgcg gatctcctgc aagggctccg gctacagctt taccaacttc     540
   tggatcagct gggtgcgaca gatgcccggc aagggcctgg aatggatggg cagagtggac     600
   cccggctact cctactccac ctactccccc agcttccagg gccacgtgac catcagcgcc     660
   gacaagtcta cctccaccgc ctacctgcag tggaactccc tgaaggcctc cgacaccgcc     720
   atgtactact gtgcccgggt gcagtacagc ggctactacg attggttcga cccctggggc     780
   cagggcaccc tcgtgacagt gtctagtggc gggggaggat ccgacgtgca gctggtgcag     840
   agcggagctg aagtgaagaa acctggcgcc tccgtgaagg tgtcctgcaa agctagcggc     900
   tataccttca cccggtacac catgcactgg gtgcgccagg cacctggaca gggactggaa     960
   tggatcggct acatcaaccc ctcccggggc tacaccaact acgccgactc tgtgaagggc    1020
   cggttcacca tcaccaccga taagtccacc agcaccgctt acatggaact gtcctccctg    1080
   agatccgagg acaccgctac ctactattgc gcccggtact acgacgacca ctactgcctg    1140
   gactactggg gacagggaac cacagtgacc gtgtcctctg gcgagggcac ctctactgga    1200
   tctgggggaa gtggtggttc tggcggcgct gacgacatcg tgctgaccca gtctccagcc    1260
   accctgtctc tgagcccagg cgagagagct accctgtcct gcagagcctc ccagtccgtg    1320
   tcctacatga attggtatca gcagaagcct ggcaaggccc ctaagcggtg gatctacgac    1380
   acctccaagg tggcctctgg cgtgccagcc cggttttccg gatctggctc tggcaccgac    1440
   tactccctga ccatcaacag cctggaagcc gaggacgctg ccacctatta ctgccagcag    1500
   tggtcctcca accccctgac ctttggaggc ggcaccaagg tggaaatcaa gcaccaccat    1560
   catcaccact gatag                                                     1575
<210> 116
   <211> 10
   <212> PRT
   <213> Artificial Sequence
<220>
   <223> Synthetic Polypeptide
<400> 116
   Gly Tyr Ser Phe Thr Asn Phe Trp Ile Ser
   1               5                   10
<210> 117
   <211> 16
   <212> PRT
   <213> Artificial Sequence
<220>
   <223> Synthetic Polypeptide
<400> 117
   Arg Val Asp Pro Gly Tyr Ser Tyr Ser Thr Tyr Ser Pro Ser Phe Gln
   1               5                   10                  15
<210> 118
   <211> 12
   <212> PRT
   <213> Artificial Sequence
<220>
   <223> Synthetic Polypeptide
<400> 118
   Val Gln Tyr Ser Gly Tyr Tyr Asp Trp Phe Asp Pro
   1               5                   10
<210> 119
   <211> 30
   <212> DNA
   <213> Artificial Sequence
<220>
   <223> Synthetic Polynucleotide
<400> 119
   ggatacagct tcaccaactt ctggatcagc 30
<210> 120
   <211> 51
   <212> DNA
   <213> Artificial Sequence
<220>
   <223> Synthetic Polynucleotide
<400> 120
   agggttgatc ctggctactc ttatagcacc tacagcccgt ccttccaagg c 51
<210> 121
   <211> 36
   <212> DNA
   <213> Artificial Sequence
<220>
   <223> Synthetic Polynucleotide
<400> 121
   gtacaatata gtggctacta tgactggttc gacccc 36
<210> 122
   <211> 13
   <212> PRT
   <213> Artificial Sequence
<220>
   <223> Synthetic Polypeptide
<400> 122
   Ser Gly Ser Ser Ser Asn Ile Gly Ser Asn Thr Val Asn
   1 5 10
<210> 123
   <211> 7
   <212> PRT
   <213> Artificial Sequence
<220>
   <223> Synthetic Polypeptide
<400> 123
   Ser Asn Asn Gln Arg Pro Ser
   1 5
<210> 124
   <211> 11
   <212> PRT
   <213> Artificial Sequence
<220>
   <223> Synthetic Polypeptide
<400> 124
   Ala Ala Trp Asp Asp Ser Leu Asn Gly Trp Val
   1 5 10
<210> 125
   <211> 39
   <212> DNA
   <213> Artificial Sequence
<220>
   <223> Synthetic Polynucleotide
<400> 125
   tctggaagca gctccaacat cggaagtaat actgtaaac 39
<210> 126
   <211> 21
   <212> DNA
   <213> Artificial Sequence
<220>
   <223> Synthetic Polynucleotide
<400> 126
   agtaataatc agcggccctc a 21
<210> 127
   <211> 33
   <212> DNA
   <213> Artificial Sequence
<220>
   <223> Synthetic Polynucleotide
<400> 127
   gcagcatggg atgacagcct gaatggttgg gtg 33
<210> 128
   <211> 451
   <212> PRT
   <213> Artificial Sequence
<220>
   <223> Synthetic Polypeptide
<400> 128
   Gln Met Gln Leu Val Gln Ser Gly Ala Glu Val Lys Glu Pro Gly Glu
   1               5                   10                  15
   Ser Leu Arg Ile Ser Cys Lys Gly Ser Gly Tyr Ser Phe Thr Asn Phe
               20                  25                  30
   Trp Ile Ser Trp Val Arg Gln Met Pro Gly Lys Gly Leu Glu Trp Met
           35                  40                  45
   Gly Arg Val Asp Pro Gly Tyr Ser Tyr Ser Thr Tyr Ser Pro Ser Phe
       50                  55                  60
   Gln Gly His Val Thr Ile Ser Ala Asp Lys Ser Thr Ser Thr Ala Tyr
   65                  70                  75                  80
   Leu Gln Trp Asn Ser Leu Lys Ala Ser Asp Thr Ala Met Tyr Tyr Cys
                   85                  90                  95
   Ala Arg Val Gln Tyr Ser Gly Tyr Tyr Asp Trp Phe Asp Pro Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Ala Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Arg Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 129
   <211> 1356
   <212> DNA
   <213> Artificial Sequence
<220>
   <223> Synthetic Polynucleotide
<400> 129
   cagatgcagc tggtgcagtc tggcgccgaa gtgaaagagc ctggcgagtc cctgcggatc 60
   tcctgcaagg gctccggcta ctcctttacc aacttctgga tcagctgggt gcgacagatg 120
   cccggcaagg gcctggaatg gatgggcaga gtggaccccg gctacagcta ctccacctac 180
   tcccccagct tccagggcca cgtgaccatc tccgccgaca agtctacctc caccgcctac 240
   ctgcagtgga actccctgaa ggcctccgac accgccatgt actactgcgc cagagtgcag 300
   tacagcggct actacgattg gttcgacccc tggggccagg gcaccctcgt gacagtgtcc 360
   tctgcttcca ccaagggccc atcggtcttc cccctggcac cctcctccaa gagcacctct 420
   gggggcacag cggccctggg ctgcctggtc aaggactact tccccgaacc ggtgacggtg 480
   tcgtggaact caggcgccct gaccagcggc gtgcacacct tcccggccgt cctacagtcc 540
   tcaggactct actccctcag cagcgtggtg accgtgccct ccagcagctt gggcacccag 600
   acctacatct gcaacgtgaa tcacaagccc agcaacacca aggtggacaa gagagttgag 660
   cccaaatctt gtgacaaaac tcacacatgc ccaccgtgcc cagcacctga actcctgggg 720
   ggaccgtcag tcttcctctt ccccccaaaa cccaaggaca ccctcatgat ctcccggacc 780
   cctgaggtca catgcgtggt ggtggacgtg agccacgaag accctgaggt caagttcaac 840
   tggtacgtgg acggcgtgga ggtgcataat gccaagacaa agccgcggga ggagcagtac 900
   gccagcacgt accgtgtggt cagcgtcctc accgtcctgc accaggactg gctgaatggc 960
   aaggagtaca agtgcaaggt ctccaacaaa gccctcccag cccccatcga gaaaaccatc 1020
   tccaaagcca aagggcagcc ccgagaacca caggtgtaca ccctgccccc atcccgggat 1080
   gagctgacca agaaccaggt cagcctgacc tgcctggtca aaggcttcta tcccagcgac 1140
   atcgccgtgg agtgggagag caatgggcag ccggagaaca actacaagac cacgcctccc 1200
   gtgctggact ccgacggctc cttcttcctc tacagcaggc tcaccgtgga caagagcagg 1260
   tggcagcagg ggaacgtctt ctcatgctcc gtgatgcatg aggctctgca caaccactac 1320
   acgcagaaga gcctctccct gtctccgggt aaatga 1356
<210> 130
   <211> 216
   <212> PRT
   <213> Artificial Sequence
<220>
   <223> Synthetic Polypeptide
<400> 130
   Gln Ala Val Val Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Thr Val Asn Trp Tyr Gln Gln Val Pro Gly Thr Ala Pro Lys Leu Leu
           35                  40                  45
   Ile Tyr Ser Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Gln
   65                  70                  75                  80
   Ser Glu Asp Glu Ala Asp Tyr Tyr Cys Ala Ala Trp Asp Asp Ser Leu
                   85                  90                  95
   Asn Gly Trp Val Phe Gly Gly Gly Thr Lys Leu Thr Val Leu Gly Gln
               100                 105                 110
   Pro Lys Ala Asn Pro Thr Val Thr Leu Phe Pro Pro Ser Ser Glu Glu
           115                 120                 125
   Leu Gln Ala Asn Lys Ala Thr Leu Val Cys Leu Ile Ser Asp Phe Tyr
       130                 135                 140
   Pro Gly Ala Val Thr Val Ala Trp Lys Ala Asp Gly Ser Pro Val Lys
   145                 150                 155                 160
   Ala Gly Val Glu Thr Thr Lys Pro Ser Lys Gln Ser Asn Asn Lys Tyr
                   165                 170                 175
   Ala Ala Ser Ser Tyr Leu Ser Leu Thr Pro Glu Gln Trp Lys Ser His
               180                 185                 190
   Arg Ser Tyr Ser Cys Gln Val Thr His Glu Gly Ser Thr Val Glu Lys
           195                 200                 205
   Thr Val Ala Pro Thr Glu Cys Ser
       210                 215
<210> 131
   <211> 651
   <212> DNA
   <213> Artificial Sequence
<220>
   <223> Synthetic Polynucleotide
<400> 131
   caggctgtcg tgactcagcc tccttctgct tctggcaccc ctggccagag agtgaccatc        60
   tcctgctccg gctcctcctc caacatcggc tccaacaccg tgaactggta tcagcaggtg       120
   cccggcaccg cccccaagct gctgatctac tctaacaacc agcggccctc cggcgtgccc       180
   gacagattct ctggctctaa gtccggcacc tccgcctccc tggctatctc tggcctgcag       240
   tctgaggacg aggccgacta ctactgcgcc gcctgggacg attctctgaa cggctgggtg       300
   ttcggcggag gcaccaagct gacagtgctg ggccagccta aggccaaccc taccgtgacc       360
   ctgttccccc catcctccga ggaactgcag gctaacaagg ccaccctcgt gtgcctgatc       420
   tccgacttct accctggcgc cgtgaccgtg gcctggaagg ctgatggatc tcctgtgaag       480
   gccggcgtgg aaaccaccaa gccctccaag cagtccaaca acaaatacgc cgcctcctcc       540
   tacctgtccc tgacccctga gcagtggaag tcccaccggt cctacagctg ccaagtgacc       600
   cacgagggct ccaccgtgga aaagaccgtg gctcctaccg agtgctccta g                651
<210> 132
   <211> 253
   <212> PRT
   <213> Artificial Sequence
<220>
   <223> Synthetic Polypeptide
<400> 132
   Gln Ala Val Val Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Thr Val Asn Trp Tyr Gln Gln Val Pro Gly Thr Ala Pro Lys Leu Leu
           35                  40                  45
   Ile Tyr Ser Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Gln
   65                  70                  75                  80
   Ser Glu Asp Glu Ala Asp Tyr Tyr Cys Ala Ala Trp Asp Asp Ser Leu
                   85                  90                  95
   Asn Gly Trp Val Phe Gly Gly Gly Thr Lys Leu Thr Val Leu Gly Ser
               100                 105                 110
   Arg Gly Gly Gly Gly Ser Gly Gly Gly Gly Ser Gly Gly Gly Gly Ser
           115                 120                 125
   Leu Glu Met Ala Gln Met Gln Leu Val Gln Ser Gly Ala Glu Val Lys
       130                 135                 140
   Glu Pro Gly Glu Ser Leu Arg Ile Ser Cys Lys Gly Ser Gly Tyr Ser
   145                 150                 155                 160
   Phe Thr Asn Phe Trp Ile Ser Trp Val Arg Gln Met Pro Gly Lys Gly
                   165                 170                 175
   Leu Glu Trp Met Gly Arg Val Asp Pro Gly Tyr Ser Tyr Ser Thr Tyr
               180                 185                 190
   Ser Pro Ser Phe Gln Gly His Val Thr Ile Ser Ala Asp Lys Ser Thr
           195                 200                 205
   Ser Thr Ala Tyr Leu Gln Trp Asn Ser Leu Lys Ala Ser Asp Thr Ala
       210                 215                 220
   Met Tyr Tyr Cys Ala Arg Val Gln Tyr Ser Gly Tyr Tyr Asp Trp Phe
   225                 230                 235                 240
   Asp Pro Trp Gly Gln Gly Thr Leu Val Thr Val Ser Ser
                   245                 250
<210> 133
   <211> 759
   <212> DNA
   <213> Artificial Sequence
<220>
   <223> Synthetic Polynucleotide
<400> 133
   caggctgtgg tgactcagcc accctcagcg tctgggaccc ccgggcagag ggtcaccatc      60
   tcttgttctg gaagcagctc caacatcgga agtaatactg taaactggta ccagcaggtc     120
   ccaggaacgg cccccaaact cctcatctat agtaataatc agcggccctc aggggtccct     180
   gaccgattct ctggctccaa gtctggcacc tcagcctccc tggccatcag tgggctccag     240
   tctgaggatg aggctgatta ttactgtgca gcatgggatg acagcctgaa tggttgggtg     300
   ttcggcggag ggaccaagct gaccgtccta ggttctagag gtggtggtgg tagcggcggc     360
   ggcggctctg gtggtggtgg atccctcgag atggcccaga tgcagctggt gcagtccgga     420
   gcagaggtga aagagcccgg ggagtctctg aggatctcct gtaagggttc tggatacagc     480
   ttcaccaact tctggatcag ctgggtgcgc cagatgcccg ggaaaggcct ggagtggatg     540
   gggagggttg atcctggcta ctcttatagc acctacagcc cgtccttcca aggccacgtc     600
   accatctcag ctgacaagtc taccagcact gcctacctgc agtggaacag cctgaaggcc     660
   tcggacaccg ccatgtatta ctgtgcgaga gtacaatata gtggctacta tgactggttc     720
   gacccctggg gccagggaac cctggtcacc gtctcctca                            759
<210> 134
   <211> 449
   <212> PRT
   <213> Artificial Sequence
<220>
   <223> Synthetic Polypeptide
<400> 134
   Gln Val Gln Leu Val Gln Ser Gly Gly Gly Val Val Gln Pro Gly Arg
   1 5 10 15
   Ser Leu Arg Leu Ser Cys Lys Ala Ser Gly Tyr Thr Phe Thr Arg Tyr
   20 25 30
   Thr Met His Trp Val Arg Gln Ala Pro Gly Lys Gly Leu Glu Trp Ile
   35 40 45
   Gly Tyr Ile Asn Pro Ser Arg Gly Tyr Thr Asn Tyr Asn Gln Lys Phe
   50 55 60
   Lys Asp Arg Phe Thr Ile Ser Arg Asp Asn Ser Lys Asn Thr Ala Phe
   65 70 75 80
   Leu Gln Met Asp Ser Leu Arg Pro Glu Asp Thr Gly Val Tyr Phe Cys
   85 90 95
   Ala Arg Tyr Tyr Asp Asp His Tyr Cys Leu Asp Tyr Trp Gly Gln Gly
               100                 105                 110
   Thr Pro Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Arg Val Glu Pro Lys Ser Cys Asp Lys
       210                 215                 220
   Thr His Thr Cys Pro Pro Cys Pro Ala Pro Glu Leu Leu Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser His Glu Asp
               260                 265                 270
   Pro Glu Val Lys Phe Asn Trp Tyr Val Asp Gly Val Glu Val His Asn
           275                 280                 285
   Ala Lys Thr Lys Pro Arg Glu Glu Gln Tyr Ala Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Ala Leu Pro Ala Pro Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Arg Asp Glu Leu Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Leu Leu Tyr Ser Lys Leu Thr Val Asp Lys
                   405                 410                 415
   Ser Arg Trp Gln Gln Gly Asn Val Phe Ser Cys Ser Val Met His Glu
               420                 425                 430
   Ala Leu His Asn His Tyr Thr Gln Lys Ser Leu Ser Leu Ser Pro Gly
           435                 440                 445
   Lys
<210> 135
   <211> 1350
   <212> DNA
   <213> Artificial Sequence
<220>
   <223> Synthetic Polynucleotide
<400> 135
   caggtgcagc tggtgcagtc cggcggcggc gtggtgcagc ccggccggtc cctgcggctg      60
   tcctgcaagg cctccggcta caccttcacc cggtacacca tgcactgggt gcggcaggcc     120
   cccggcaagg gcctggagtg gatcggctac atcaacccct cccggggcta caccaactac     180
   aaccagaagt tcaaggaccg gttcaccatc tcccgggaca actccaagaa caccgccttc     240
   ctgcagatgg actccctgcg gcccgaggac accggcgtgt acttctgcgc ccggtactac     300
   gacgaccact actgcctgga ctactggggc cagggcaccc ccgtgaccgt gtcctccgcc     360
   tccaccaagg gcccatcggt cttccccctg gcaccctcct ccaagagcac ctctgggggc     420
   acagcggccc tgggctgcct ggtcaaggac tacttccccg aaccggtgac ggtgtcgtgg     480
   aactcaggcg ccctgaccag cggcgtgcac accttcccgg ccgtcctaca gtcctcagga     540
   ctctactccc tcagcagcgt ggtgaccgtg ccctccagca gcttgggcac ccagacctac     600
   atctgcaacg tgaatcacaa gcccagcaac accaaggtgg acaagagagt tgagcccaaa     660
   tcttgtgaca aaactcacac atgcccaccg tgcccagcac ctgaactcct ggggggaccg     720
   tcagtcttcc tcttcccccc aaaacccaag gacaccctca tgatctcccg gacccctgag     780
   gtcacatgcg tggtggtgga cgtgagccac gaagaccctg aggtcaagtt caactggtac     840
   gtggacggcg tggaggtgca taatgccaag acaaagccgc gggaggagca gtacgccagc     900
   acgtaccgtg tggtcagcgt cctcaccgtc ctgcaccagg actggctgaa tggcaaggag     960
   tacaagtgca aggtctccaa caaagccctc ccagccccca tcgagaaaac catctccaaa    1020
   gccaaagggc agccccgaga accacaggtg tacaccctgc ccccatcccg ggatgagctg    1080
   accaagaacc aggtcagcct gacctgcctg gtcaaaggct tctatcccag cgacatcgcc    1140
   gtggagtggg agagcaatgg gcagccggag aacaactaca agaccacgcc tcccgtgctg    1200
   gactccgacg gctccttcct cctctacagc aagctcaccg tggacaagag caggtggcag    1260
   caggggaacg tcttctcatg ctccgtgatg catgaggctc tgcacaacca ctacacgcag    1320
   aagagcctct ccctgtctcc gggtaaatga                                     1350
<210> 136
   <211> 213
   <212> PRT
   <213> Artificial Sequence
<220>
   <223> Synthetic Polypeptide
<400> 136
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Ser Ser Val Ser Tyr Met
               20                  25                  30
   Asn Trp Tyr Gln Gln Thr Pro Gly Lys Ala Pro Lys Arg Trp Ile Tyr
           35                  40                  45
   Asp Thr Ser Lys Leu Ala Ser Gly Val Pro Ser Arg Phe Ser Gly Ser
       50                  55                  60
   Gly Ser Gly Thr Asp Tyr Thr Phe Thr Ile Ser Ser Leu Gln Pro Glu
   65                  70                  75                  80
   Asp Ile Ala Thr Tyr Tyr Cys Gln Gln Trp Ser Ser Asn Pro Phe Thr
                   85                  90                  95
   Phe Gly Gln Gly Thr Lys Leu Gln Ile Thr Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 137
   <211> 642
   <212> DNA
   <213> Artificial Sequence
<220>
   <223> Synthetic Polynucleotide
<400> 137
   gacatccaga tgacccagtc cccctcctcc ctgtccgcct ccgtgggcga ccgggtgacc      60
   atcacctgct ccgcctcctc ctccgtgtcc tacatgaact ggtaccagca gacccccggc     120
   aaggccccca agcggtggat ctacgacacc tccaagctgg cctccggcgt gccctcccgg     180
   ttctccggct ccggctccgg caccgactac accttcacca tctcctccct gcagcccgag     240
   gacatcgcca cctactactg ccagcagtgg tcctccaacc ccttcacctt cggccagggc     300
   accaagctgc agatcacccg gaccgtggcc gccccctccg tgttcatctt ccccccctcc     360
   gacgagcagc tgaagtccgg caccgcctcc gtggtgtgcc tgctgaacaa cttctacccc     420
   cgggaggcca aggtgcagtg gaaggtggac aacgccctgc agtccggcaa ctcccaggag     480
   tccgtgaccg agcaggactc caaggactcc acctactccc tgtcctccac cctgaccctg     540
   tccaaggccg actacgagaa gcacaaggtg tacgcctgcg aggtgaccca ccagggcctg     600
   tcctcccccg tgaccaagtc cttcaaccgg ggcgagtgct ag                        642
<210> 138
   <211> 9
   <212> PRT
   <213> Artificial Sequence
<220>
   <223> Synthetic Polypeptide
<400> 138
   Lys Ile Phe Gly Ser Leu Ala Phe Leu
   1               5
<210> 139
   <211> 9
   <212> PRT
   <213> Artificial Sequence
<220>
   <223> Synthetic Polypeptide
<400> 139
   Leu Phe Glu Val Arg Val Cys Ala Cys
   1               5
<210> 140
   <211> 10
   <212> PRT
   <213> Artificial Sequence
<220>
   <223> Synthetic Polypeptide
<400> 140
   Ala Leu Tyr Val Asp Ser Leu Phe Phe Leu
   1 5 10
<210> 141
   <211> 9
   <212> PRT
   <213> Artificial Sequence
<220>
   <223> Synthetic Polypeptide
<400> 141
   Asn Leu Thr His Val Leu Tyr Pro Val
   1 5
<210> 142
   <211> 9
   <212> PRT
   <213> Artificial Sequence
<220>
   <223> Synthetic Polypeptide
<400> 142
   Gln Leu Gln Asn Pro Ser Tyr Asp Lys
   1 5